# EUROPEAN PATENT SPECIFICATION

(11) **EP 1 075 475 B1**
(45) Date of publication and mention of the grant of the patent: **18.06.2003**
(21) Application number: 99917174.7
(22) Date of filing: 26.04.1999
(51) Int. Cl.: C07D 405/14, C07D 417/14, C07D 401/14, A61K 31/44, C07D 409/14, C07D 491/04

(54) **HETEROCYCLIC CARBOXAMIDE DERIVATIVES AS INHIBITORS OF NITRIC OXIDE PRODUCTION**
HETEROCYCLISCHE CARBONSÄUREAMID DERIVATE ALS HEMMSTOFFE DER STICKSTOFFMONOXID-PRODUKTION
DERIVES DE CARBOXAMIDES HETEROCYCLIQUES EN TANT QUE INHIBITEURS DE LA PRODUCTION DU MONOXYDE D'AZOTE

(30) Priority: 04.05.1998 AU PP332498; 10.06.1998 AU PP401498
(43) Date of publication of application: 14.02.2001
(73) Proprietor: FUJISAWA PHARMACEUTICAL CO., LTD., Osaka-shi Osaka 541-8514 (JP)
(72) Inventor: SHIMA, Ichiro, Kitasouma-gun, Ibaraki 302-0119 (JP); OHKAWA, Takehiko, Yuuki-gun, Ibaraki 300-2742 (JP); OHNE, Kazuhiko, Tsukuba-shi, Ibaraki 305-0051 (JP); ZENKO, Tatsuya, Ota-ku, Tokyo 145-0063 (JP); OKU, Teruo, DECEASED (JP); YOSHIHARA, Kousei, Tsuchiura-shi, Ibaraki 300-0051 (JP); SETOI, Hiroyuki, Tsukuba-shi, Ibaraki 305-0044 (JP)
(74) Representative: Gille Hrabal Struck Neidlein Prop Roos
(86) International application number: JP9902231
(87) International publication number: WO99057114

(56) References cited:
- WO-A-96/01817
- WO-A-97/45425
- WO-A-98/27108

## Description

### TECHNICAL FIELD

This invention relates to new amide compounds and pharmaceutically acceptable salts thereof which are useful as medicament.

### BACKGROUND ART

Some peptide compounds have been known as described in, for example, EP 0 394 989 A2. Further compounds are known From WO 97 45 425.

### DISCLOSURE OF INVENTION

This invention relates to new amide compounds.

One object of this invention is to provide the new and useful amide compounds and pharmaceutically acceptable salts thereof that possess a strong inhibitory activity on the production of nitric oxide (NO).

Another object of this invention is to provide a process for the preparation of the amide compounds and salts thereof.

A further object of this invention is to provide a pharmaceutical composition comprising said amide compound or a pharmaceutically acceptable salt thereof.

Still further object of this invention is to provide a use of said amide compounds or pharmaceutically acceptable salts thereof as a medicament for prophylactic and therapeutic treatment of NO-mediated diseases such as adult respiratory distress syndrome, cardiovascular ischemia, myocarditis, heart failure, synovitis, shock (e.g., septic shock, etc.), diabetes (e.g., insulin-dependent diabetes mellitus, etc.), diabetic nephropathy, diabetic retinopathy, diabetic neuropathy, glomerulonephritis, peptic ulcer, inflammatory bowel disease (e.g., ulcerative colitis, chronic colitis, etc.), cerebral infarction, cerebral ischemia, cerebral hemorrhage, migraine, rheumatoid arthritis, gout, neuritis, postherpetic neuralgia, osteoarthritis, osteoporosis, systemic lupus erythematosus, rejection by organ transplantation, asthma, metastasis, Alzheimer's disease, arthritis, CNS disorders, dermatitis, hepatitis, liver cirrhosis, multiple sclerosis, pancreatitis, atherosclerosis, and the like in human being and animals. Further, they are useful for treatment of erectile dysfunction, male sexual dysfunction or female sexual dysfunction including orgasmic dysfunction related to clitoral disturbances.

The object amide compounds of the present invention are novel and can be represented by the following general formula (I): 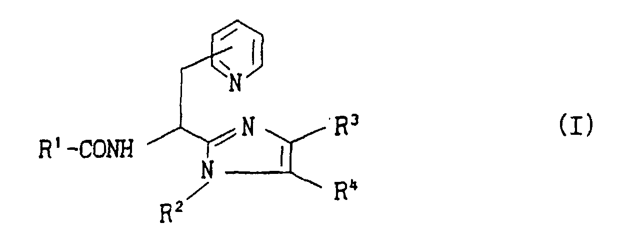 wherein
- R¹: is (a) indolyl, (b) benzothienyl having suitable substituent(s) selected from the group consisting of nitro and halogen, (c) benzothiazolyl having halogen, (d) furopyridyl which may have nitro or (e) benzofuranyl which may have suitable substituent(s) selected from the group consisting of halogen, lower alkyl, lower alkoxy, nitro, cyano, acyl and trihalo(lower)alkyl,
- R²: is lower alkyl,
- R³: is hydrogen or lower alkyl, and
- R⁴: is thienyl or a group of the formula: 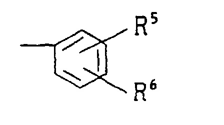
wherein
- R⁵: is hydrogen or halogen, and
- R⁶: is (a) imidazolyl which may have lower alkyl,
(b) benzimidazolyl, (c) pyridyl, (d) pyrrolyl,
(e) morpholinyl, (f) thienyl, (g) furyl, (h) phenyl,
(j) thiazolyl, (k) halogen or (1) nitro,
provided that (i) R³ is lower alkyl or R⁶ is benzimidazolyl or imidazolyl having lower alkyl when R¹ is indolyl, and (ii) R⁶ is benzimidazolyl or imidazolyl having lower alkyl when R¹ is benzofuranyl.

The present invention also provides novel amide compounds represented by the following general formula (II): 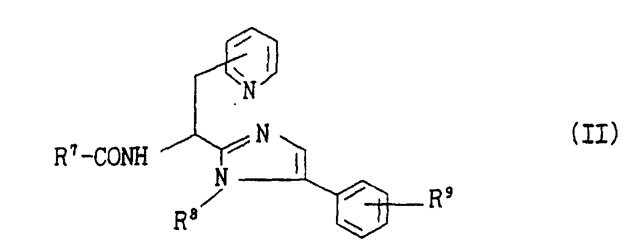 wherein
- R⁷: is indolyl, benzofuranyl, benzothienyl or benzothiazolyl,
- R⁸: is lower alkyl, and
- R⁹: is imidazolyl or nitro,
provided that (i) R⁸ is not methyl when R⁷ is indolyl, and (ii) R⁹ is nitro when R⁷ is benzofuranyl (compare WO 98 27108).

Suitable pharmaceutically acceptable salts of the object compounds (I) and (II) are conventional non-toxic salts and include, for example, a salt with a base or an acid addition salt such as a salt with an inorganic base, for example, an alkali metal salt (e.g., sodium salt, potassium salt, etc.), an alkaline earth metal salt (e.g., calcium salt, magnesium salt, etc.), an ammonium salt; a salt with an organic base, for example, an organic amine salt (e.g., triethylamine salt, pyridine salt, picoline salt, ethanolamine salt, triethanolamine salt, dicyclohexylamine salt, N,N'-dibenzylethylene-diamine salt, etc.); an inorganic acid addition salt (e.g., hydrochloride, hydrobromide, sulfate, phosphate, etc.); an organic carboxylic or sulfonic acid addition salt (e.g., formate, acetate, trifluoroacetate, maleate, tartrate, citrate, fumarate, methanesulfonate, benzenesulfonate, toluenesulfonate, etc.); and a salt with a basic or acidic amino acid (e.g., arginine, aspartic acid, glutamic acid, etc.).

In the above and subsequent descriptions of the present specification, suitable examples and illustration of the various definitions which the present invention intends to include within the scope thereof are explained in detail as follows.

The term "lower" is used to intend a group having 1 to 6, preferably 1 to 4, carbon atom(s), unless otherwise provided.

Suitable "halogen" includes, for example, fluorine, bromine, chlorine and iodine.

Suitable "lower alkyl" includes straight or branched one having 1 to 6 carbon atom(s), such as methyl, ethyl, propyl, isopropyl, butyl, isobutyl, sec-butyl, tert-butyl, pentyl, tert-pentyl and hexyl, in which more preferred one is C₁-C₄ alkyl.

Suitable "lower alkoxy" includes straight or branched one having 1 to 6 carbon atoms, such as methoxy, ethoxy, propoxy, isopropoxy, butoxy, isobutoxy, sec-butoxy, tert-butoxy, pentyloxy, tert-pentyloxy and hexyloxy, in which more preferred one is C₁-C₄ alkoxy.

Suitable "acyl" includes, aliphatic acyl group such as lower alkanoyl (e.g., formyl, acetyl, propanoyl, butanoyl, 2-methylpropanoyl, pentanoyl, 2,2-dimethylpropanoyl, hexanoyl, etc.).

Suitable "trihalo(lower)alkyl" includes, for example, trifluoromethyl, trichloromethyl and tribromomethyl, in which preferred one is trifluoromethyl.

The term "morpholinyl" includes 2-morpholinyl, 3-morpholinyl and 4-morpholinyl (i.e. morpholino).

The object compounds (I) and (II) of the present invention can be prepared by the following processes.

### Process (1)

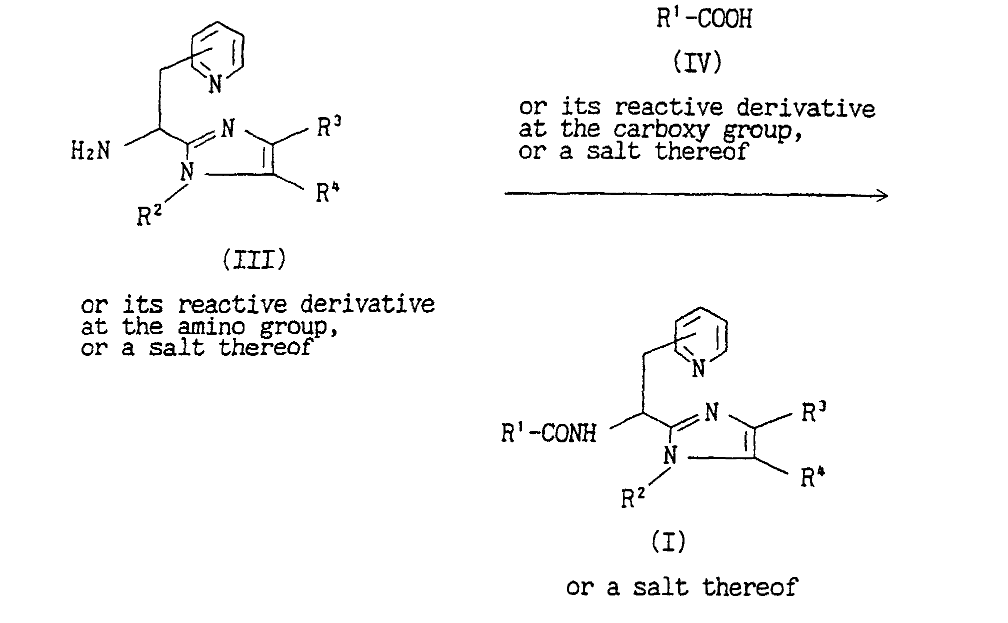

### Process (2)

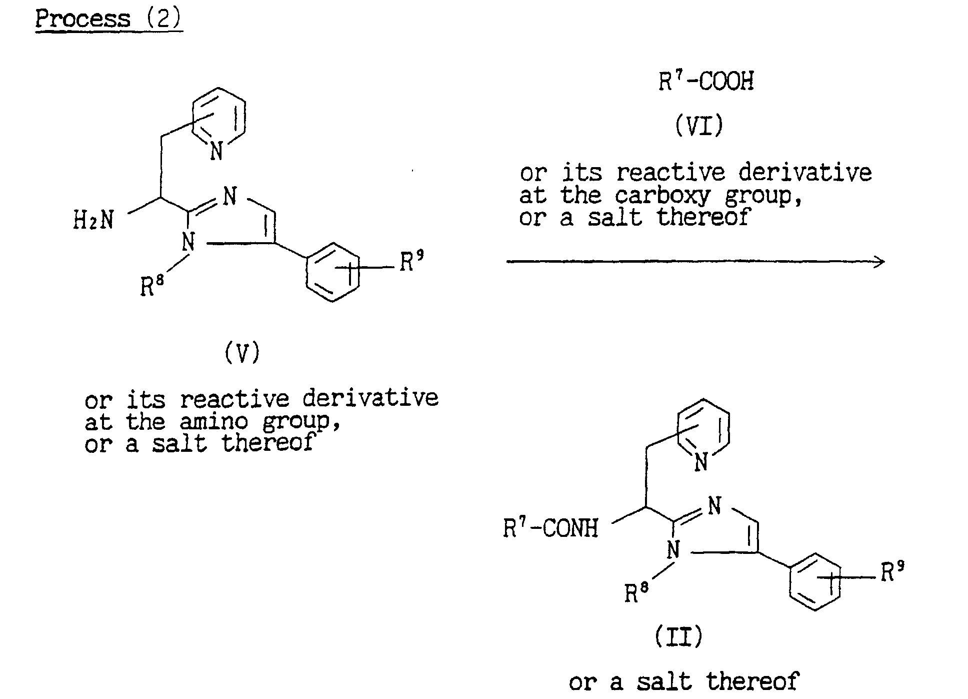

wherein R¹, R², R³, R⁴, R⁷, R⁸ and R⁹ are each as defined above.

The starting compounds can be prepared by the method of Preparation mentioned below or by a process known in the art for preparing their structually analogous compounds.

The processes for preparing the object compound are explained in detail in the following.

### Process (1)

The compound (I) or a salt thereof can be prepared by reacting the compound (III) or its reactive derivative at the amino group, or a salt thereof with the compound (IV) or its reactive derivative at the carboxy group, or a salt thereof.

Suitable reactive derivative of the compound (III) includes Schiff's base type imino or its tautomeric enamine type isomer formed by the reaction of the compound (III) with a carbonyl compound such as aldehyde, ketone or the like; a silyl derivative formed by the reaction of the compound (III) with a silyl compound such as N,O-bis(trimethylsilyl)acetamide, N-trimethylsilylacetamide or the like; a derivative formed by the reaction of the compound (III) with phosphorus trichloride or phosgene.

Suitable reactive derivative of the compound (IV) includes an acid halide, an acid anhydride and an activated ester. The suitable example may be an acid chloride; an acid azide; a mixed acid anhydride with an acid such as substituted phosphoric acid (e.g., dialkylphosphoric acid, phenylphosphoric acid, diphenylphosphoric acid, dibenzylphosphoric acid, halogenated phosphoric acid, etc.), dialkylphosphorous acid, sulfurous acid, thiosulfuric acid, alkanesulfonic acid (e.g., methanesulfonic acid, ethanesulfonic acid, etc.), sulfuric acid, alkylcarbonic acid, aliphatic carboxylic acid (e.g., pivalic acid, pentanoic acid, isopentanoic acid, 2-ethylbutyric acid, trichloroacetic acid, etc.); aromatic carboxylic acid (e.g., benzoic acid, etc.); a symmetrical acid anhydride; an activated amide with imidazole, 4-substituted imidazole, dimethylpyrazole, triazole or tetrazole; an activated ester (e.g., cyanomethyl ester, methoxymethyl ester, dimethyliminomethyl [(CH₃)₂N⁺=CH-] ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, etc.); or an ester with an N-hydroxy compound (e.g., N,N-dimethylhydroxylamine, 1-hydroxy-2-(1H)-pyridone, N-hydroxysuccinimide, N-hydroxybenzotriazole, N-hydroxyphthalimide, 1-hydroxy-6-chloro-1H-benzotriazole, etc.). These reactive derivatives can optionally be selected from them according to the kind of the compound (IV) to be used.

The reaction is usually carried out in a conventional solvent such as water, acetone, dioxane, acetonitrile, chloroform, methylene chloride, ethylene chloride, tetrahydrofuran, ethyl acetate, N,N-dimethylformamide, pyridine or any other organic solvents which do not adversely affect the reaction, or the mixture thereof.

When the compound (IV) is used in free acid form or its salt form in the reaction, the reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)carbodiimide; N,N'-diisopropylcarbodiimide; N-ethyl-N'-(3-dimethylaminopropyl)-carbodiimide; N,N-carbonyl-bis-(2-methylimidazole); pentamethyleneketene-N-cyclohexylimine; diphenylketene-N-cyclohexylimine; ethoxyacetylene; 1-alkoxy-1-chloroethylene; trialkyl phosphite; isopropyl polyphosphate; phosphorus oxychloride (phosphoryl chloride); phosphorus trichloride; thionyl chloride; oxalyl chloride; triphenylphosphine; 2-ethyl-7-hydroxybenzisoxazolium salt; 2-ethyl-5-(m-sulfophenyl)isoxazolium hydroxide intramolecular salt; 1-(p-chlorobenzenesulfonyloxy)-6-chloro-1H-benzotriazole; so-called Vilsmeier reagent prepared by the reaction of N,N-dimethylformamide with thionyl chloride, phosgene, phosphorus oxychloride, etc.; or the like.

The reaction may also be carried out in the presence of an organic or inorganic base such as an alkali metal bicarbonate, tri(lower)alkylamine, pyridine, N-(lower)alkylmorpholine, N,N-di(lower)alkylbenzylamine, or the like.

The reaction temperature is not critical, and the reaction is usually carried out under cooling to heating.

### Process (2)

The compound (II) or a salt thereof can be prepared by reacting the compound (V) or its reactive derivative at the amino group, or a salt thereof with the compound (VI) or its reactive derivative at the carboxy group, or a salt thereof.

This reaction can be carried out in a similar manner to the reaction in the aforementioned Process (1), and therefore the reagents to be used and the reaction conditions (e.g., solvent, reaction temperature, etc.) can be referred to those of the Process (1).

Suitable salts of the starting compounds and their reactive derivatives in Processes (1) and (2) can be referred to the ones as exemplified for the compounds (I) and (II).

The compounds obtained by the above process can be isolated and purified by a conventional method such as pulverization, recrystallization, column chromatography, reprecipitation, or the like.

It is to be noted that the compounds (I) and (II) and the other compounds may include one or more stereoisomer(s) such as optical isomer(s) and geometrical isomer(s) due to asymmetric carbon atom(s) and double bond(s), and all of such isomers and mixtures thereof are included within the scope of this invention.

The object compounds (I) and (II) and pharmaceutically acceptable salts thereof include solvates [e.g., enclosure compounds (e.g., hydrate, etc.)].

The object compounds (I) and (II) and pharmaceutically acceptable salts thereof possess a strong inhibitory activity on the production of nitric oxide (NO).

Accordingly, the object compounds (I) and (II) and pharmaceutically acceptable salts thereof are expected to possess a nitric oxide synthase (NOS)-inhibitory activity or a NOS-production inhibitory activity.

Accordingly, they are useful for prevention and/or treatment of NO-mediated diseases such as adult respiratory distress syndrome, cardiovascular ischemia, myocarditis, heart failure, synovitis, shock (e.g., septic shock, etc.), diabetes (e.g., insulin-dependent diabetes mellitus, etc.), diabetic nephropathy, diabetic retinopathy, diabetic neuropathy, glomerulonephritis, peptic ulcer, inflammatory bowel disease (e.g., ulcerative colitis, chronic colitis, etc.), cerebral infarction, cerebral ischemia, cerebral hemorrhage, migraine, rheumatoid arthritis, gout, neuritis, postherpetic neuralgia, osteoarthritis, osteoporosis, systemic lupus erythematosus, rejection by organ transplantation, asthma, metastasis, Alzheimer's disease, arthritis, CNS disorders, dermatitis, hepatitis, liver cirrhosis, multiple sclerosis, pancreatitis, atherosclerosis, and the like. Further, they are useful for treatment of erectile dysfunction, male sexual dysfunction or female sexual dysfunction including orgasmic dysfunction related to clitoral disturbances.

In order to illustrate the usefulness of the object compounds (I) and (II), the pharmacological test result of the representative compound of the compounds (I) and (II) is shown in the following.

### Test Compounds :

Compound (a) : (1S)-(5-chlorobenzo[b]furan-2-yl)-N-[1-[1-methyl-5-(4-morpholinophenyl)imidazol-2-yl]-2-(2-pyridyl)ethyl]formamide
Compound (b) : (1S)-N-[1-[5-(4-biphenylyl)-1-methylimidazol-2-yl]-2-(2-pyridyl)ethyl](5-bromobenzo[b]furan-2-yl)formamide
Compound (c) : (1S)-(benzo[b]thiophen-2-yl)-N-[1-[5-[4-(1-imidazolyl)-phenyl]-1-methylimidazol-2-yl]-2-(2-pyridyl)ethyl]formamide
Compound (d) : (1S)-N-[1-[5-[4-(1-imidazolyl)phenyl]-1-methylimidazol-2-yl]-2-(2-pyridyl)ethyl](5-nitrobenzo[b]thiophen-2-yl)formamide
Compound (e) : (1S)-N-[1-[1-ethyl-5-[4-(1-imidazolyl)phenyl]imidazol-2-yl]-2-(4-pyridyl)ethyl](indol-2-yl)formamide
Compound (f) : (1S)-(5-methoxybenzo[b]furan-2-yl)-N-[1-[1-methyl-5-(4-nitrophenyl)imidazol-2-yl]-2-(2-pyridyl)ethyl]formamide
Compound (g) : (1S)-(benzothiazol-2-yl)-N-[1-[5-[4-(1-imidazolyl)-phenyl]-1-methylimidazol-2-yl]-2-(2-pyridyl)ethyl]formamide

### Test 1 : Assay for inhibitory activity on the production of nitric oxide

The murine macrophage cell line RAW264.7 (American Type Culture Collection, No. TIB71) was used in this study. RAW264.7 cells were grown on F75 plastic culture flasks at 37°C, 5% in Dulbecco's modified Eagle's medium (DMEM) supplemented with L-glutamine, penicillin, streptomycin and 10% heat-inactivated fetal bovine serum. They were removed from culture flasks by rubber cell scraper and were centrifuged and resuspended in DMEM without phenol red. They were plated in 96-well microtiter plates (10⁵ cells per well) and allowed to adhere over 2 hours. The test samples were added and the cells were preincubated for 1 hour. Thereafter the cells were activated with both of lipopolysaccharide (LPS) (1 µg/ml) and interferon γ (INF γ) (3 u/ml) for 18-24 hours. An equal volume of Griess reagent (1% sulfanilamide/0.1% N-naphthylethylenediamine dihydrochloride/2. 5% H₃PO₄) was added and the cells were incubated at room temperature for 10 minutes. The absorbance was read at 570 nm using microplate reader and NO₂⁻ was measured using NaNO₂ as a standard.

### Test result :

**Table 1**

| Test compound (10⁻⁵M) | Inhibition (%) |
|---|---|
| (a) | 100 |
| (b) | 100 |
| (c) | 100 |
| (d) | 100 |
| (e) | 100 |
| (f) | 100 |
| (g) | 100 |

### Test 2 : Protective effect of the compound (a) combined with FK 506 on rat cardiac allograft

### Method :

Experiments were performed on male Lewis and ACI rats weighing 175-200 g. Rats were anesthetized with sodium pentobarbital (50 mg/kg, i.p.), and underwent allogeneic (Lewis donor to ACI recipient) heterotopic intra-abdominal cardiac transplantation. Experimental groups were divided into single-drug group and combined-drug group. Single-drug dose of FK506, which was prepared in a manner similar to that disclosed in EP-0184162, was 0.32 mg/kg. Combined-drug dose was FK506 (0.32 mg/kg) + the compound (a) (10 mg/kg). The grafted hearts were monitored by daily palpation where complete rejection was defined as the cessation of palpable contractile activity. Each drug was suspended in a solution of 0.5% methylcellulose, and administered by daily gastric intubation in a volume of 5 ml/kg of body weight for 14 days.

### Test result :

The combination of the compound (a) and an immunosuppressive agent (FK506) was examined to determine whether it could improve rat cardiac allograft survival.

Graft survival is shown in the following table 2.

**Table 2:**

| Protective effect of the compound (a) combined with FK506 on rat cardiac allograft | | |
|---|---|---|
| Test compound(s) | n | MST (day) |
| FK506 (0.32 mg/kg) | 6 | 10 |
| Compound (a) (10 mg/kg) + FK506 (0.32 mg/kg) | 9 | >30 |
| MST: Median Survival Time | | |

The combination of the compound (a) and FK506 dramatically prolonged the graft survival.

The above experimental data indicate that the activity and/or efficacy of an immunosuppressant in rejection of transplantation can be remarkably and synergistically increased by administering compound (a) in combination, which has a strong inhibitory activity on the production of nitric oxide.

For therapeutic administration, the object compounds (I) and (II) of the present invention and pharmaceutically acceptable salts thereof are used in the form of a conventional pharmaceutical preparation in admixture with a conventional pharmaceutically acceptable carrier such as an organic or inorganic solid or liquid excipient which is suitable for oral, parenteral or external administration. The pharmaceutical preparation may be compounded in a solid form such as granule, capsule, tablet, dragee, suppository or ointment, or in a liquid form such as solution, suspension or emulsion for injection, intravenous drip, ingestion, eye drop, etc. If needed, there may be included in the above preparation auxiliary substance such as stabilizing agent, wetting or emulsifying agent, buffer or any other commonly used additives.

The effective ingredient may usually be administered in a unit dose of 0.001 mg/kg to 500 mg/kg, preferably 0.01 mg/kg to 10 mg/kg, 1 to 4 times a day. However, the above dosage may be increased or decreased according to age, body weight and conditions of the patient or administering method.

The following Preparations and Example are given for the purpose of illustrating the present invention in detail.

### Preparation 1

### 1-(4-Morpholinophenyl)ethan-1-one

A mixture of 1-(4-fluorophenyl)ethan-1-one (100g), morpholine (126ml) and potassium carbonate (95g) in N,N-dimethylformamide (DMF) (1L) was heated at 150°C (bath temperature) for 48 hours. TLC showed the exhaust of the starting material. The reaction mixture was poured into water (2L), and the precipitate was collected and washed with water. Then, the residue was dissolved in ethyl acetate, dried over magnesium sulfate, and filtered. After removal of the solvent, 1-(4-morpholinophenyl)ethan-1-one (81.5g) was obtained as a yellow-brown powder.
¹H-NMR (DMSO-d₆) δ 2.46(3H,s), 3.30(4H,dd,J=4Hz,J=5Hz), 3.72(4H,dd,J=4Hz,J=5Hz), 6.98(2H,d,J=9Hz), 7.83(2H,d,J=9Hz)

### Preparation 2

### 2-Bromo-1-(4-morpholinophenyl)ethan-1-one

To a solution of 1-(4-morpholinophenyl)ethan-1-one (170g) in 48% HBr (340ml) was added Br₂ (43ml) in 48% HBr (60ml) dropwise at 65-75°C during the period of 0.5 hour. The mixture was stirred at 65°C for an additional 1 hour. The reaction mixture was cooled to 10°C and the resulting precipitate was collected by filtration. The resulting solid (HBr salt) was basified with saturated aqueous sodium hydrogencarbonate solution carefully, and extracted with chloroform (500ml). The organic layer was dried over magnesium sulfate, and filtered. After removal of the solvent, 2-bromo-1-(4-morpholinophenyl)ethan-1-one (200g) was obtained as a greenish yellow powder. Further purification was not attempted.
¹H-NMR (DMSO-d₆) δ 3.30-3.34(4H,dd,J=4Hz,J=5Hz), 3.34-3.43(4H,m), 6.99(2H,d,J=9Hz), 7.86(2H,d,J=9Hz)

### Preparation 3

### 2-[2-(4-Morpholinophenyl)-2-oxoethyl]isoindoline-1,3-dione

A mixture of 2-bromo-1-(4-morpholinophenyl)ethan-1-one (200g) and potassium phthalimide (137g) in DMF (1500ml) was stirred at 90°C for 1 hour. After cooling to 10°C, the precipitate was collected, and washed with cold-DMF (150ml) and water (250ml×2), successively. 2-[2-(4-Morpholinophenyl)-2-oxoethyl]isoindoline-1,3-dione (186g) was obtained as a pale yellow wet solid. This compound was used for next step without further dry-up or purification.
¹H-NMR (DMSO-d₆) δ 3.34(4H,dd,J=4Hz,J=5Hz), 3.72(4H,dd,J=4Hz,J=5Hz), 5.10(2H,s), 7.04(2H,d,J=9Hz), 7.86-7.97(6H,m)

### Preparation 4

### 2-Amino-1-(4-morpholinophenyl)ethan-1-one dihydrochloride

A slurry of 2-[2-(4-morpholinophenyl)-2-oxoethyl]isoindoline-1,3-dione (185g) in concentrated hydrochloric acid (1900ml) was heated under reflux for 8 hours. As a result, the slurry gradually dissolved and a clear pale yellow solution was obtained. After removal of the solvent in vacuo, the residual oily solid was triturated with methanol (400ml), and the resulting precipitate was collected by filtration, and washed with methanol (100ml) to give 2-amino-1-(4-morpholinophenyl)ethan-1-one dihydrochloride (125g) as a white powder.
¹H-NMR (DMSO-d₆) δ 3.35(4H,dd,J=4Hz,J=5Hz), 3.73(4H,dd,J=4Hz,J=5Hz), 4.45(2H,d,J=4.5Hz), 7.03(2H,d,J=9Hz), 7.87(2H,d,J=9Hz)

### Preparation 5

### (2S)-2-(tert-Butoxycarbonylamino)-N-[2-(4-morpholinophenyl)-2-oxoethyl]-3-(2-pyridyl)propionamide

To a solution of 2-amino-1-(4-morpholinophenyl)ethan-1-one dihydrochloride (3.71g), (2S)-2-(tert-butoxycarbonylamino)-3-(2-pyridyl)propanoic acid (5.73g) and diphenylphosphoryl azide (3.48g) in N,N-dimethylformamide (70ml) was added dropwise N,N-diisopropylethylamine (4.41ml) at 0°C and the mixture was stirred for 20 minutes. The mixture was heated to ambient temperature and stirred for 8 hours. The resulting mixture was diluted with ethyl acetate (200ml) and washed successively with water, saturated aqueous sodium hydrogencarbonate solution and brine. The organic layer was dried over magnesium sulfate and concentrated in vacuo. The residual solid was triturated with ethyl acetate-diisopropyl ether (1:2) to give (2S)-2-(tert-butoxycarbonylamino)-N-[2-(4-morpholinophenyl)-2-oxoethyl]-3-(2-pyridyl)propionamide (2.06g) as off-white crystals.
ESI-MS: 469(M+H)
¹H-NMR (300MHz,CDCl₃) δ 1.46(9H,s), 3.20-3.38(6H,m), 3.82-3.88(4H,m), 4.60(2H,d,J=5Hz), 4.64-4.74(1H,br), 6.37-6.45(1H,br), 6.86(2H,d,J=9Hz), 7.14(1H,dd,J=5,8Hz), 7.21(1H,d,J=8Hz), 7.59(1H,t,J=8Hz), 7.82-7.90(3H,m), 8.56(1H,d,J=5Hz)

### Preparation 6

### (1S)-(tert-Butoxy)-N-[1-[1-methyl-5-(4-morpholinophenyl)imidazol-2-yl]-2-(2-pyridyl)ethyl]formamide

To a solution of (2S)-2-(tert-butoxycarbonylamino)-N-[2-(4-morpholinophenyl)-2-oxoethyl]-3-(2-pyridyl)propionamide (2.0g) in acetic acid (4.0ml) and xylene (60ml) was added methylamine (40% in water, 4.0ml) and the mixture was refluxed for 3 hours in a roundbottomed flask equipped with a Dean-Stark apparatus. The mixture was cooled to ambient temperature and a mixture of acetic acid (4.0ml) and methylamine (40% in water, 4.0ml) was added to the solution. The solution was refluxed for 2 hours and cooled to ambient temperature. The solution was extracted with 1N hydrochloric acid (100ml) and the aqueous layer was washed with ethyl acetate (50ml). The aqueous layer was basified with saturated aqueous sodium hydrogencarbonate solution and extracted with ethyl acetate (100ml). The organic layer was washed successively with aqueous sodium hydrogencarbonate solution and brine, dried over magnesium sulfate and concentrated in vacuo. The residue was purified by silica gel column chromatography (2% methanol in chloroform) to give (1S)-(tert-butoxy)-N-[1-[1-methyl-5-(4-morpholinophenyl)imidazol-2-yl]-2-(2-pyridyl)ethyl]-formamide (1.45g) as yellow crystals.
ESI-MS: 464(M+H)
¹H-NMR (300MHz,CDCl₃) δ 1.37(9H,s), 3.17-3.23(4H,m), 3.40(3H,s), 3.41-3.47(2H,m), 3.83-3.92(4H,m), 5.33-5.47(2H,m), 6.93(1H,s), 6.94(2H,d,J=9Hz), 7.08-7.16(2H,m), 7.21(2H,d,J=9Hz), 7.56(1H,t,J=8Hz), 8.55(1H,d,J=5Hz)

### Preparation 7

### (1S)-1-[1-Methyl-5-(4-morpholinophenyl)imidazol-2-yl]-2-(2-pyridyl)ethylamine

To a solution of (1S)-(tert-butoxy)-N-[1-[1-methyl-5-(4-morpholinophenyl)imidazol-2-yl]-2-(2-pyridyl)ethyl]formamide (1.43g) in dichloromethane (25ml) was added trifluoroacetic acid (5.0ml) at 0°C and the mixture was stirred at ambient temperature for 2.5 hours. The resulting mixture was concentrated in vacuo and the residue was dissolved in water (20ml). The aqueous layer was basified with saturated aqueous sodium hydrogencarbonate solution and extracted with chloroform (80ml). The organic layer was dried over magnesium sulfate and concentrated in vacuo. The residue was triturated with ethyl acetate-diisopropyl ether (1:2) to give (1S)-1-[1-methyl-5-(4-morpholinophenyl)imidazol-2-yl]-2-(2-pyridyl)ethylamine (1.02g) as yellow crystals.
ESI-MS: 364(M+H)
¹H-NMR (300MHz,CDCl₃) δ 3.17-3.23(4H,m), 3.27-3.47(2H,m), 3.49(3H,s), 3.84-3.91(4H,m), 4.58(1H,dd,J=5,8Hz), 6.95(2H,d,J=9Hz), 6.97(1H,s), 7.11-7.18(2H,m), 7.23(2H,d,J=9Hz), 7.59(1H,t,J=8Hz), 8.58(1H,d,J=5Hz)

### Preparation 8

### (2S)-2-(tert-Butoxycarbonylamino)-N-[2-(4-biphenylyl)-2-oxoethyl]-3-(2-pyridyl)propionamide

The title compound was obtained in substantially the same manner as in Preparation 5.
mp 129-132°C
MS: 460(M+1)
¹H-NMR (DMSO-d₆) δ : 1.29(9H,s), 2.90-3.11(1H,m), 3.17-3.23(1H,m), 4.47-4.55(1H,m), 4.56-4.78(2H,m), 7.09(1H,d,J=8Hz), 7.20(1H,t,J=8Hz), 7.30(1H,d,J=8Hz), 7.40-7.58(3H,m), 7.70(1H,t,J=8Hz), 7.78(2H,d,J=8Hz), 7.85(2H,d,J=8Hz), 8.09(2H,d,J=8Hz), 8.21(1H,t,J=6Hz), 8.50(1H,d,J=4Hz)

### Preparation 9

### (1S)-(tert-Butoxy)-N-[1-[5-(4-biphenylyl)-1-methylimidazol-2-yl]-2-(2-pyridyl)ethyl]formamide

The title compound was obtained from the compound of Preparation 8 in substantially the same manner as in Preparation 6.
solid
MS: 455(M+1)
¹H-NMR (DMSO-d₆) δ : 1.30(9H,s), 3.20-3.30(1H,m), 3.33-3.47(1H,m), 3.59(3H,s), 5.30(1H,q,J=8Hz), 7.00(1H,s), 7.15-7.30(3H,m), 7.33-7.58(4H,m), 7.61-7.80(4H,m), 7.82(1H,d,J=8Hz), 8.09(1H,d,J=8Hz), 8.50(1H,d,J=4Hz)

### Preparation 10

### (1S)-1-[5-(4-Biphenylyl)-1-methylimidazol-2-yl]-2-(2-pyridyl)-ethylamine

The title compound was obtained from the compound of Preparation 9 in substantially the same manner as in Preparation 7.
oil
MS: 355(M+1)
¹H-NMR (DMSO-d₆) δ : 3.10-3.20(1H,m), 3.28-3.38(1H,m), 3.60(3H,s), 4.48(1H,t,J=8Hz), 6.99(1H,s), 7.18-7.30(2H,m), 7.36-7.59(4H,m), 7.60-7.80(6H,m), 8.51(1H,d,J=2Hz)

### Preparation 11

### 2-Bromo-1-[4-(1-imidazolyl)phenyl]ethan-1-one hydrobromide

To a solution of 1-[4-(1-imidazolyl)phenyl]ethan-1-one (50.25g) in acetic acid (400ml) was added 30% hydrogen bromide/acetic acid (d 1.35, 80ml). Bromine (40.9g) was added dropwise to the mixture for 20 minutes while the temperature of the reaction mixture was maintained between 20-25°C. After the addition was complete, the mixture was heated at 50°C for 1 hour and allowed to cool to room temperature. The mixture was diluted with diisopropyl ether (400ml) and the product was filtered and washed with diisopropyl ether. Recrystallization from methanol (750 ml) gave 2-bromo-1-[4-(1-imidazolyl)phenyl]ethan-1-one hydrobromide as a white powder (68.83g).
MS(ESI)m/z: 265,267(free, M+H)⁺
¹H-NMR (DMSO-d₆,300MHz)δ : 5.01(2H,s), 7.94(1H,s), 8.02(2H,d,J=8Hz), 8.24(2H,d,J=8Hz), 8.41(1H,s), 9.89(1H,s)

### Preparation 12

### 2-Azido-1-[4-(1-imidazolyl)phenyl]ethan-1-one

To a suspension of 2-bromo-1-[4-(1-imidazolyl)phenyl]ethan-1-one hydrobromide (48.7g) in N,N-dimethylformamide (500ml) was added sodium azide (9.15g) at 5°C. The mixture was stirred at the same temperature for 30 minutes, then at room temperature for 1 hour. The mixture was poured into diluted sodium hydrogencarbonate solution (1.6L) and extracted three times with ethyl acetate. The extract was washed twice with brine and dried over magnesium sulfate. Evaporation of the solvent gave 2-azido-1-[4-(1-imidazolyl)-phenyl]ethan-1-one as a white solid (18.9g).
MS(ESI)m/z: 228(M+H)⁺
¹H-NMR (DMSO-d₆,300MHz) δ : 4.92(2H,s), 7.16(1H,s), 7.88(2H,d,J=8Hz), 7.92(1H,s), 8.07(2H,d,J=8Hz), 8.46(1H,s)

### Preparation 13

### 2-Amino-1-[4-(1-imidazolyl)phenyl]ethan-1-one dihydrochloride

A solution of 2-azido-1-[4-(1-imidazolyl)phenyl]ethan-1-one (18.9g) in a mixture of 2N hydrochloric acid (90ml) and methanol (90ml) was hydrogenated (3 atm) over 10% palladium on carbon (1.9g) at room temperature for 3 hours. After the catalyst was filtered off, the filtrate was concentrated to give a white powder. The white powder was collected by filtration, washed with methanol and dried in vacuo to give 2-amino-1-[4-(1-imidazolyl)phenyl]ethan-1-one dihydrochloride (16.0g).
MS(ESI)m/z: 202(free,M+H)⁺
¹H-NMR (DMSO-d₆,300MHz) δ : 4.67(2H,q,J=5Hz), 7.89(1H,s), 8.08(2H,d,J=8Hz), 8.27(2H,d,J=8Hz), 8.41(1H,s), 8.52(3H,br s), 9.78(1H,s)

### Preparation 14

### (2S)-2-(tert-Butoxycarbonylamino)-N-[2-[4-(1-imidazolyl)phenyl]-2-oxoethyl]-3-(2-pyridyl)propionamide

The title compound was obtained from the compound of Preparation 13 in substantially the same manner as in Preparation 5.
oil
MS: 450(M+1)
¹H-NMR (CDCl₃) δ 1.42(9H,s), 3.20-3.30(1H,m), 3.31-3.42(1H,m), 4.62-4.73(1H,m), 4.70(2H,d,J=6Hz), 6.42(1H,br s), 7.15(1H,t,J=6Hz), 7.21(1H,d,J=6Hz), 7.23(1H,s), 7.33(1H,s), 7.50(2H,d,J=8Hz), 7.60(1H,t,J=8Hz), 7.97(1H,s), 8.00(1H,br s), 8.08(2H,d,J=8Hz), 8.57(1H,d,J=8Hz)

### Preparation 15

### (1S)-(tert-Butoxy)-N-[1-[5-[4-(1-imidazolyl)phenyl]-1-methylimidazol-2-yl]-2-(2-pyridyl)ethyl]formamide

The title compound was obtained from the compound of Preparation 14 in substantially the same manner as in Preparation 6.
amorphous solid
MS: 445(M+1)
¹H-NMR (CDCl₃) δ 1.38(9H,s), 3.39-3.52(2H,m), 3.49(3H,s), 5.38-5.52(1H,m), 5.149(1H,br s), 7.01(1H,s), 7.12(2H,d,J=8Hz), 7.22(2H,d,J=8Hz), 7.30(1H,s), 7.38-7.50(3H,m), 7.57(1H,t,J=8Hz), 7.90(1H,s), 8.53(1H,d,J=2Hz)

### Preparation 16

### (1S)-1-[5-[4-(1-Imidazolyl)phenyl]-1-methylimidazol-2-yl]-2-(2-pyridyl)ethylamine tetrahydrochloride

The title compound was obtained from the compound of Preparation 15 in substantially the same manner as in Preparation 26.
mp 253-256°C
¹H-NMR (DMSO-d₆) δ : 3.80-4.03(2H,m), 3.88(3H,s), 5.54(1H,t,J=6Hz), 7.65(1H,t,J=5Hz), 7.69-7.85(4H,m), 7.98-8.08(3H,m), 8.16(1H,t,J=8Hz), 8.40(1H,s), 8.69(1H,d,J=5Hz)

### Preparation 17

### (2S)-2-(tert-Butoxycarbonylamino)-N-[2-(4-nitrophenyl)-2-oxoethyl]-3-(2-pyridyl)propionamide

The title compound was obtained in substantially the same manner as in Preparation 5.
MS(ESI)m/z: 429(M+H)⁺
¹H-NMR (CDCl₃,300MHz) δ 1.46(9H,s), 3.20-3.43(2H,m), 4.62-4.78(3H,m), 6.43(1H,br d,J=8Hz), 7.12-7.27(2H,m), 7.56-7.67(1H,m), 8.04(1H,br s), 8.10(2H,d,J=8Hz), 8.32(2H,d,J=8Hz), 8.54(1H,d,J=5Hz)

### Preparation 18

### (1S)-(tert-Butoxy)-N-[1-[1-methyl-5-(4-nitrophenyl)imidazol-2-yl]-2-(2-pyridyl)ethyl]formamide

The title compound was obtained from the compound of Preparation 17 in substantially the same manner as in Preparation 6.
MS(ESI)m/z: 424(M+H)⁺
¹H-NMR (CDCl₃, 300MHz) δ 1.38(9H,s), 3.38-3.50(2H,m), 3.53(3H,s), 5.37-5.51(1H,m), 5.54(1H,br d,J=8Hz), 7.05-7.20(3H,m), 7.46(2H,d,J=8Hz), 7.55(1H,t,J=8Hz), 8.27(2H,d,J=8Hz), 8.52(1H,d,J=5Hz)

### Preparation 19

### (1S)-1-[1-Methyl-5-(4-nitrophenyl)imidazol-2-yl]-2-(2-pyridyl)-ethylamine

The title compound was obtained from the compound of Preparation 18 in substantially the same manner as in Preparation 26.
MS(ESI)m/z: 324 (M+H)⁺
¹H-NMR (CDCl₃,300MHz) δ 3.27-3.50(2H,m), 3.61(3H,s), 4.620H,dd,J=8 and 6Hz), 7.11-7.22(3H,m), 7.50(2H,d,J=8Hz), 7.61(1H,t,J=7Hz), 8.29(2H,d,J=8Hz), 8.58(1H,d,J=5Hz)

### Preparation 20

### (1S)-N-[1-[5-(4-Aminophenyl)-1-methylimidazol-2-yl]-2-(2-pyridyl)ethyl](5-chlorobenzo[b]furan-2-yl)formamide

To a solution of (1S)-(5-chlorobenzo[b]furan-2-yl)-N-[1-[1-methyl-5-(4-nitrophenyl)imidazol-2-yl]-2-(2-pyridyl)ethyl]formamide (425mg) in ethanol (10ml) were added iron powder (473mg) and acetic acid (508mg) and the mixture was refluxed for 4 hours. The reaction mixture was filtered through a bed of Celite and the filtrate was concentrated in vacuo. The residue was diluted with a mixture of ethyl acetate and saturated aqueous sodium hydrogencarbonate solution and the mixture was filtered through a bed of Celite again. The organic layer was separated and washed with water and brine. The organic layer was dried over sodium sulfate, concentrated, and purified by silica gel column chromatography (3% methanol in chloroform) to give (1S)-N-[1-[5-(4-aminophenyl)-1-methylimidazol-2-yl]-2-(2-pyridyl)ethyl](5-chlorobenzo[b]furan-2-yl)formamide (230mg).
MS(ES+): 472(M+H)
¹H-NMR (300MHz, CDCl₃) δ 3.46(3H,s), 3.54-3.62(2H,m), 5.92(1H,m), 6.63-6.73(2H,m), 6.94(1H,s), 7.02-7.15(5H,m), 7.30-7.63(6H,m), 7.68-7.77(1H,m), 8.53(1H,m)

### Preparation 21

### 2-[2-(4-Fluorophenyl)-2-oxoethyl]isoindoline-1,3-dione

A mixture of 2-bromo-1-(4-fluorophenyl)ethan-1-one (40g) and potassium phthalimide (35.8g) in 1,4-dioxane (300ml) was refluxed for 6 hours. After cooling, the precipitated salt was filtered off and the filtrate was concentrated in vacuo. The crystalline residue was washed with diisopropyl ether to give 2-[2-(4-fluorophenyl)-2-oxoethyl]isoindoline-1,3-dione (49g) as light yellow crystals.
mp 140-144°C
MS(m/z): 282(M⁺-H),146(bp)
¹H-NMR (CDCl₃) δ 5.10(2H,s), 7.21(2H,t,J=7.5Hz), 7.77-7.80(2H,m), 7.89-7.93(2H,m), 8.03-8.09(2H,m)

### Preparation 22

### 2-[2-[4-(2-Methylimidazol-1-yl)phenyl]-2-oxoethyl]isoindoline-1,3-dione

To a suspension of sodium hydride (424mg) in DMF (20ml) was added 2-methylimidazole (1.28g) portionwise at 0°C under stirring. After stirring at 0°C for 0.5 hour, 2-[2-(4-fluorophenyl)-2-oxoethyl]isoindoline-1,3-dione (2.0g) was added and the mixture was stirred at 60°C for 4 hours. After cooling, the solvent was distilled away. The residue was diluted with ethyl acetate. The precipitated crystals were collected by suction filtration. The collected crystals were washed with a small amount of water and diethyl ether and dried to give 2-[2-[4-(2-methylimidazol-1-yl)-phenyl]-2-oxoethyl]isoindoline-1,3-dione (894mg) as orange crystals.
mp 310-315°C (decomposition)
MS(m/z): 346(M⁺+H,bp)
¹H-NMR (CDCl₃) δ 2.45(3H,s), 5.18(2H,s), 7.09(2H,d,J=4Hz), 7.48(2H,d,J=8Hz), 7.78-7.81(2H,m), 7.77-7.96(2H,m), 8.16(2H,d,J=8Hz)

### Preparation 23

### 2-Amino-1-[4-(2-methylimidazol-1-yl)phenyl]ethan-1-one dihydrochloride

2-[2-[4-(2-Methylimidazol-1-yl)phenyl]-2-oxoethyl]isoindoline-1,3-dione (884mg) was added to 37% hydrochloric acid (9ml) and refluxed for 8 hours. After cooling, water was distilled away and the residue was washed with a small amount of methanol (2ml) to give 2-amino-1-[4-(2-methylimidazol-1-yl)phenyl]ethan-1-one dihydrochloride (345mg) as colorless crystals.
mp 320°C (decomposition)
MS(m/z): 216(M⁺+H),148(bp)
¹H-NMR (DMSO-d₆) δ 2.60(3H,s), 4.68(2H,s), 7.81(1H,d,J=4Hz), 7.89(2H,d,J=8Hz), 7.97(1H,d,J=4Hz), 8.28(2H,d,J=8Hz), 8.59(2H,br.s)

### Preparation 24

### (2S)-2-(tert-Butoxycarbonylamino)-N-[2-[4-(2-methylimidazol-1-yl)-phenyl]-2-oxoethyl]-3-(2-pyridyl)propionamide

To a solution of (2S)-2-(tert-butoxycarbonylamino)-3-(2-pyridyl)-propanoic acid (648mg) in DMF (5ml) were added diphenylphosphoryl azide (0.55ml) and 2-amino-1-[4-(2-methylimidazol-1-yl)phenyl]ethan-1-one dihydrochloride (701mg) at 5°C under stirring. Diisopropylethylamine (1.4ml) was added dropwise at 5°C. The reaction mixture was stirred at room temperature for 3 hours. The reaction mixture was diluted with ethyl acetate and washed with saturated aqueous sodium hydrogencarbonate solution. The aqueous layer was extracted with ethyl acetate for another 2 times. The combined organic layers were washed with 1N hydrochloric acid. The aqueous layer was basified with saturated aqueous sodium hydrogencarbonate solution to pH 3, treated with charcoal, filtered and washed with water. The filtrate was basified with saturated aqueous sodium hydrogencarbonate solution and 1N aqueous sodium hydroxide solution to pH 8 and extracted with chloroform. The organic layer was dried over magnesium sulfate and concentrated in vacuo. The residue was purified by flash column chromatography on silica gel eluting with a mixture of methanol and chloroform (1:20) to give (2S)-2-(tert-butoxycarbonylamino)-N-[2-[4-(2-methylimidazol-1-yl)phenyl]-2-oxoethyl]-3-(2-pyridyl)propionamide (340mg) as a brown oil.
MS(m/z): 464(M⁺+H,bp)
¹H-NMR (CDCl₃) δ 1.61(9H,s), 2.42(3H,s), 3.24-3.42(3H,m), 4.72(2H,d,J=5Hz), 7.06(2H,d,J=7.5Hz), 7.15-7.24(2H,m), 7.43(2H,d,J=8Hz), 7.63(1H,t,J=8Hz), 8.03(2H,s) 8.09(2H,d,J=8Hz), 8.58(1H,d,J=5Hz)

### Preparation 25

### (1S)-(tert-Butoxy)-N-[1-[1-methyl-5-[4-(2-methylimidazol-1-yl)-phenyl]imidazol-2-yl]-2-(2-pyridyl)ethyl]formamide

To a solution of (2S)-2-(tert-butoxycarbonylamino)-N-[2-[4-(2-methylimidazol-1-yl)phenyl]-2-oxoethyl]-3-(2-pyridyl)-propionamide (340mg) in xylene (3.2ml) were added acetic acid (0.32ml) and methylamine (40% in water) (0.32ml) under stirring and refluxed azeotropically for 3 hours. After cooling, the solvent was distilled away. The residue was diluted with saturated aqueous sodium hydrogencarbonate solution and extracted with chloroform. The organic layer was dried over magnesium sulfate and concentrated in vacuo. The residue was purified by flash column chromatography on silica gel eluting with a mixture of methanol and chloroform (1:20) to give (1S)-(tert-butoxy)-N-[1-[1-methyl-5-[4-(2-methylimidazol-1-yl)phenyl]-imidazol-2-yl]-2-(2-pyridyl)ethyl]formamide (250mg) as a brown oil.
MS(m/z): 459(M⁺+H,bp)
¹H-NMR (CDCl₃) δ 1.38(9H,s), 2.40(3H,s), 3.45(1H,d,J=6Hz), 3.53(3H,s), 5.44(2H,br.s), 7.05(2H,d,J=7.5Hz), 7.08(1H,s), 7.12(2H,d,J=7.5Hz), 7.38(4H,AB,J=8Hz,J=8Hz), 7.54-7.60(1H,m), 8.55(2H,d,J=6Hz)

### Preparation 26

### (1S)-1-[1-Methyl-5-[4-(2-methylimidazol-1-yl)phenyl]imidazol-2-yl]-2-(2-pyridyl)ethylamine

To a solution of (1S)-(tert-butoxy)-N-[1-[1-methyl-5-[4-(2-methylimidazol-1-yl)phenyl]imidazol-2-yl]-2-(2-pyridyl)-ethyl]formamide (242mg) in methanol (1ml) was added 4N hydrogen chloride in ethyl acetate (1.2ml) at room temperature under stirring. The resulting mixture was stirred at room temperature for 5 hours. After evaporation of the solvent, the residue was basified with 1N aqueous sodium hydroxide solution and extracted with chloroform. The organic layer was dried over magnesium sulfate and concentrated in vacuo. The residue was purified by flash column chromatography on silica gel eluting with a mixture of methanol and chloroform (1:5∼ 1:1) to give (1S)-1-[1-methyl-5-[4-(2-methylimidazol-1-yl)phenyl]-imidazol-2-yl]-2-(2-pyridyl)ethylamine (143mg) as a yellow oil.
MS(m/z): 359(M⁺+H,bp)
¹H-NMR (CDCl₃) δ 2.42(3H,s), 3.31-3.48(2H,m), 3.44(3H,s), 4.68(1H,dd,J=8Hz,J=7Hz),7.08(2H,d,J=7Hz), 7.10(1H,s), 7.16-7.20(2H,m),7.40(4H,AB,J=8Hz,J=8Hz), 7.62(1H,t,J=7Hz), 8.60(1H,d,J=7Hz)

### Preparation 27

### (2S)-2-(tert-Butoxycarbonylamino)-N-[2-(4-bromophenyl)-2-oxoethyl]-3-(2-pyridyl)propionamide

The title compound was obtained in substantially the same manner as in Preparation 5.
MS(ESI)m/z: 462,464(M+H)⁺
¹H-NMR (CDCl₃,300MHz) δ 1.44(9H,s), 3.18-3.43(2H,m), 4.58-4.75(1H,m), 4.64(2H,d,J=5Hz), 6.42(1H,br d,J=8Hz), 7.10-7.23(2H,m), 7.53-7.65(1H,m), 7.61(2H,d,J=8Hz), 7.79(2H,d,J=8Hz), 7.92(1H,br s), 8.53(1H,d,J=5Hz)

### Preparation 28

### (1S)-(tert-Butoxy)-N-[1-[5-(4-bromophenyl)-1-methylimidazol-2-yl]-2-(2-pyridyl)ethyl]formamide

The title compound was obtained from the compound of Preparation 27 in substantially the same manner as in Preparation 6.
MS(ESI)m/z: 457,459(M+H)⁺
¹H-NMR (CDCl₃,300MHz) δ 1.37(9H,s), 3.33-3.52(2H,m), 3.42(3H,s), 5.31-5.52(2H,m), 6.99(1H,s), 7.05-7.15(2H,m), 7.18(2H,d,J=8Hz), 7.48-7.61(1H,m), 7.53(2H,d,J=8Hz), 8.53(1H,d,J=5Hz)

### Preparation 29

### (1S)-(tert-Butoxy)-N-[1-[5-[4-(2-furyl)phenyl]-1-methylimidazol-2-yl]-2-(2-pyridyl)ethyl]formamide

A mixture of 2-(tributylstannyl)furan(264mg), (1S)-(tert-butoxy)-N-[1-[5-(4-bromophenyl)-1-methylimidazol-2-yl]-2-(2-pyridyl)ethyl]-formamide (260mg), tetrakis(triphenylphosphine) palladium (66mg) and anhydrous lithium chloride (72mg) in toluene (10ml) was heated at 100°C for 2 hours. The cooled mixture was filtered through a bed of Celite and concentrated in vacuo. The residue was purified by silica gel column chromatography (3% methanol in chloroform) to give (1S)-(tert-butoxy)-N-[1-[5-[4-(2-furyl)phenyl]-1-methylimidazol-2-yl]-2-(2-pyridyl)ethyl]formamide (218mg) as a white powder.
mp 131-135°C
MS(ES+): 445(M+H)
¹H-NMR (300MHz, CDCl₃) δ 1.37(9H,s), 3.41-3.51(5H,m), 5.35-5.48(2H,m), 6.48(1H,m), 6.68(1H,m), 7.02(1H,s), 7.07-7.16(2H,m), 7.32(2H,d,J=8Hz), 7.48(1H,s), 7.55(1H,dd,J=8Hz,J=8Hz), 7.70(2H,d,J=8Hz), 8.54(1H,m)

### Preparation 30

### (1S)-1-[5-[4-(2-Furyl)phenyl]-1-methylimidazol-2-yl]-2-(2-pyridyl)ethylamine

The title compound was obtained from the compound of Preparation 29 in substantially the same manner as in Preparation 26.
MS(ES+): 345(M+H)
¹H-NMR (300MHz, CDCl₃) δ 1.73(2H,d,J=7Hz), 3.33(1H,m), 3.45(1H,m), 3.54(3H,s), 4.59(1H,m), 6.48(1H,m), 6.68(1H,m), 7.06(1H,s), 7.10-7.21(2H,m), 7.35(2H,d,J=8Hz), 7.49(1H,m), 7.60(1H,m), 7.72(2H,d,J=8Hz), 8.58(1H,d,J=6Hz)

### Preparation 31

### (1S)-(tert-Butoxy)-N-[1-[1-methyl-5-[4-(2-thienyl)phenyl]-imidazol-2-yl]-2-(2-pyridyl)ethyl]formamide

The title compound was obtained from the compound of Preparation 28 in substantially the same manner as in Preparation 29.
MS(ES+): 461(M+H)
¹H-NMR (300MHz, CDCl₃) δ 1.37(9H,s), 3.38-3.51(m,5H), 5.32-5.51(m,2H), 7.02(12H,d), 7.06-7.20(4H,m), 7.27-7.37(2H,m), 7.49-7.60(3H,m), 7.65(1H,d,J=8Hz), 8.54(1H,m)

### Preparation 32

### (1S)-1-[1-Methyl-5-[4-(2-thienyl)phenyl]imidazol-2-yl]-2-(2-pyridyl)ethylamine

The title compound was obtained from the compound of Preparation 31 in substantially the same manner as in Preparation 26.
MS(ES+): 361(M+H)
¹H-NMR (300MHz, CDCl₃) δ 1.78(2H,br), 3.26-3.38(1H,m), 3.38-3.50(1H,m), 3.52 and 3.56(s,Total 3H), 4.54-4.65(1H,m), 7.00-7.22(5H,m), 7.28-7.38(2H,m), 7.51-7.70(4H,m), 8.58(1H,m)

### Preparation 33

### (1S)-(tert-Butoxy)-N-[1-[1-methyl-5-[4-(2-thiazolyl)phenyl]-imidazol-2-yl]-2-(2-pyridyl)ethyl]formamide

The title compound was obtained from the compound of Preparation 28 and 2-(trimethylstannyl)thiazole in substantially the same manner as in Preparation 29.
MS(ES+): 462(M+H)
¹H-NMR (300MHz,CDCl₃) δ 1.37(9H,s), 3.41-3.67(5H,m), 5.42-5.54(1H,m), 5.61-5.72(1H,m), 7.09(1H,s), 7.10-7.18(2H,m), 7.34-7.42(3H,m), 7.56(1H,dd,J=8Hz,J=8Hz), 7.89(1H,d,J=4Hz), 8.02(2H,d,J=8Hz), 8.52(1H,m)

### Preparation 34

### (1S)-1-[1-Methyl-5-[4-(2-thiazolyl)phenyl]imidazol-2-yl]-2-(2-pyridyl)ethylamine

The title compound was obtained from the compound of Preparation 33 in substantially the same manner as in Preparation 26.
MS(ES+): 362(M+H)
¹H-NMR (300MHz,CDCl₃) δ 1.74(2H,br), 3.34(1H,dd,J=15Hz,J=8Hz), 3.46(1H,dd,J=15Hz,J=6H2), 3.55(3H,s), 4.60(1H,br), 7.04-7.21(3H,m), 7.35(1H,d,J=5Hz), 7.40(2H,d,J=8Hz), 7.60(1H,m), 7.89(1H,d,J=5Hz), 8.03(2H,d,J=8Hz), 8.58(1H,d,J=6Hz)

### Preparation 35

### 1-(3-Fluoro-4-morpholinophenyl)ethan-1-one

A mixture of 1-(3,4-difluorophenyl)ethan-1-one (3g), morpholine (3.4ml) and potassium carbonate (2.5g) in DMF (30ml) was heated at 150°C (bath temperature) for 48 hours. TLC showed the exhaust of the starting material. The reaction mixture was poured into water (60ml), and the precipitate was collected and washed with water. Then, the residue was dissolved in ethyl acetate, dried over magnesium sulfate, and filtered. After removal of the solvent, 1-(3-fluoro-4-morpholinophenyl)ethan-1-one (3.04g) was obtained as a yellow-brown powder.
¹H-NMR (DMSO-d₆) δ 3.14(4H,dd,J=4Hz,J=5Hz), 3.33(3H,s), 3.73(4H,dd,J=4Hz,J=5Hz), 7.10(2H,dd,J=9Hz), 7.66(2H,dd,J=15Hz,J=1.5Hz), 7.73(2H,dd,J=9Hz,J=1.5Hz)

### Preparation 36

### 2-Bromo-1-(3-fluoro-4-morpholinophenyl)ethan-1-one

To a solution of 1-(3-fluoro-4-morpholinophenyl)ethan-1-one (3.0g) in 48% HBr (5ml) was added Br₂ (0.7ml) in 48% HBr (2ml) dropwise at 65-70°C during the period of 0.5 hour. The mixture was stirred at 65°C for an additional 1 hour. The reaction mixture was cooled to 10°C and basified with saturated aqueous sodium hydrogencarbonate solution carefully, and extracted with ethyl acetate (50ml). The organic layer was dried over magnesium sulfate, and filtered. After removal of the solvent, 2-bromo-1-(3-fluoro-4-morpholinophenyl)ethan-1-one (3.7g) was obtained as a brown solid.

### Preparation 37

### 2-[2-(3-Fluoro-4-morpholinophenyl)-2-oxoethyl]isoindoline-1,3-dione

A mixture of 2-bromo-1-(3-fluoro-4-morpholinophenyl)ethan-1-one (3.7g) and potassium phthalimide (2.38g) in DMF (30ml) was stirred at 90°C for 1 hour. After cooling to 10°C, the precipitate was collected and washed with cold-DMF (10ml) and water (25ml × 2), successively. 2-[2-(3-Fluoro-4-morpholinophenyl)-2-oxoethyl]-isoindoline-1,3-dione (2.95g) was obtained as a pale yellow wet solid. This compound was used for next step without further dry-up or purification.

### Preparation 38

### 2-Amino-1-(3-fluoro-4-morpholinophenyl)ethan-1-one dihydrochloride

A slurry of 2-[2-(3-fluoro-4-morpholinophenyl)-2-oxoethyl]-isoindoline-1,3-dione (2.9g) in concentrated hydrochloric acid (30ml) was heated under reflux for 8 hours. As a result, the slurry gradually dissolved and a clear pale yellow solution was obtained. After removal of the solvent in vacuo, the residual oily solid was triturated with methanol (10ml), collected by filtration, and washed with methanol to give 2-amino-1-(3-fluoro-4-morpholinophenyl)ethan-1-one dihydrochloride (1.64g) as a white powder.
¹H-NNR (DMSO-d₆) δ 3.20(4H,dd,J=4Hz,J=5Hz), 3.73(4H,dd,J=4Hz,J=5Hz), 4.50(2H,s), 7.15(2H,dd,J=9Hz), 7.71-7.83(3H,m)

### Preparation 39

### (2S)-2-(tert-Butoxycarbonylamino)-N-[2-(3-fluoro-4-morpholinophenyl)-2-oxoethyl]-3-(2-pyridyl)propionamide

The title compound was obtained from the compound of Preparation 38 in substantially the same manner as in Preparation 5.
MS: 487(ES+)

### Preparation 40

### (1S)-(tert-Butoxy)-N-[1-[5-(3-fluoro-4-morpholinophenyl)-1-methylimidazol-2-yl]-2-(2-pyridyl)ethyl]formamide

The title compound was obtained from the compound of Preparation 39 in substantially the same manner as in Preparation 6.

### Preparation 41

### (1S)-1-[5-(3-Fluoro-4-morpholinophenyl)-1-methylimidazol-2-yl]-2-(2-pyridyl)ethylamine

The title compound was obtained from the compound of Preparation 40 in substantially the same manner as in Preparation 26.

### Preparation 42

### 2-Bromo-1-(2-thienyl)ethan-1-one

To a stirred solution of 2-acetylthiophene (3g) in dichloromethane (50ml) and methanol (20ml) was added tetrabutylammonium tribromide (12.6g) at room temperature. The reaction mixture was stirred at room temperature for 12 hours. After evaporation of the solvent, the residue was taken up in ether, washed with 5% aqueous sodium thiosulfate and water, dried over magnesium sulfate and concentrated in vacuo to give 2-bromo-1-(2-thienyl)ethan-1-one (4.53g) as a yellow oil.
¹H-NMR (CDCl₃) δ 4.36(2H,s), 7.18(1H,dd,J=5Hz,J=4Hz), 7.73(1H,d,J=5Hz), 7.81(1H,d,J=4Hz)

### Preparation 43

### 2-Azido-1-(2-thienyl)ethan-1-one

To a solution of 2-bromo-1-(2-thienyl)ethan-1-one (9.5g) in DMF (50ml) was added sodium azide (3.01g) at 0°C under stirring. The resulting mixture was stirred at room temperature for 4 hours. The reaction mixture was poured into ice-water and extracted with ethyl acetate. The organic layer was washed with brine, dried over magnesium sulfate and concentrated in vacuo. The residue was purified by flash column chromatography on silica gel eluting with a mixture of ethyl acetate and hexane (1:10) to give 2-azido-1-(2-thienyl)-ethan-1-one (1.88g) as a brown oil.
¹H-NMR (CDCl₃) δ 4.46(2H,s), 7.16-7.19(1H,m), 7.71-7.75(2H,m)

### Preparation 44

### 2-Amino-1-(2-thienyl)ethan-1-one hydrochloride

A mixture of 2-azido-1-(2-thienyl)ethan-1-one (2.57g) and hydrochloric acid (2.8ml) in a mixture of methanol (35ml), tetrahydrofuran (35ml) and water (35ml) was hydrogenated over 10% palladium carbon (420mg) under 2 atm of hydrogen for 5 hours at room temperature. The catalyst was filtered off through Celite pad. After evaporation of the filtrate, the resulting residue was washed with diisopropyl ether to give 2-amino-1-(2-thienyl)ethan-1-one hydrochloride (2.51g) as green crystals.
MS(m/z): 142(M⁺+H,bp)
¹H-NMR (DMSO-d₆) δ 4.52(2H,s), 7.32-7.35(1H,m), 8.11(1H,d,J=5Hz), 8.17(1H,d,J=5Hz), 8.37(2H,br.s)

### Preparation 45

### (2S)-2-(tert-Butoxycarbonylamino)-N-[2-oxo-2-(2-thienyl)ethyl]-3-(2-pyridyl)propionamide

The title compound was obtained from the compound of Preparation 44 in substantially the same manner as in Preparation 5.
MS(m/z): 390(M⁺+H,bp)
¹H-NMR (CDCl₃) δ 1.46(9H,s), 3.21-3.40(2H,m), 4.62(2H,d,J=7Hz), 4.65-4.70(1H,m), 6.40(1H,d,J=7Hz), 7.13-7.22(3H,m), 7.59(1H,ddd,J=7Hz,J=7Hz,J=2Hz), 7.68(1H,dd,J=7Hz,J=2Hz), 7.75(1H,dd,J=7Hz,J=2Hz), 7.91(1H,br.s), 8.54(1H,d,J=7Hz)

### Preparation 46

### (1S)-(tert-Butoxy)-N-[1-[1-methyl-5-(2-thienyl)imidazol-2-yl]-2-(2-pyridyl)ethyl]formamide

The title compound was obtained from the compound of Preparation 45 in substantially the same manner as in Preparation 6.
MS(m/z): 385(M⁺+H,bp)
¹H-NMR (CDCl₃) δ 1.37(9H,s), 3.43(2H,d,J=7Hz), 3.47(3H,s), 5.36-5.48(2H,m), 6.99(1H,d,J=3Hz), 7.14-7.17(4H,m), 7.35(1H,d,J=7Hz), 7.56(1H,t,J=7Hz), 8.53(1H,d,J=3Hz)

### Preparation 47

### (1S)-1-[1-Methyl-5-(2-thienyl)imidazol-2-yl]-2-(2-pyridyl)-ethylamine

The title compound was obtained from the compound of Preparation 46 in substantially the same manner as in Preparation 26.
MS(m/z): 285(M⁺+H,bp)
¹H-NMR (CDCl₃) δ 3.36(2H,dddd,J=15Hz,J=14Hz,J=8Hz,J=7Hz), 3.56(3H,s), 4.58(1H,dd,J=8Hz,J=7Hz), 7.03(1H,d,J=3Hz), 7.08-7.17(4H,m), 7.34(1H,dd,J=7Hz,J=3Hz), 7.59(1H,ddd,J=8Hz,J=8Hz,J=3Hz), 8.57(1H,d,J=7Hz)

### Preparation 48

### 2-Bromo-1-(2,4-dichlorophenyl)ethan-1-one

To a solution of 2,4-dichloroacetophenone (5g) and hydrochloric acid (0.1ml) in acetic acid (50ml) was added dropwise a solution of bromine (1.36ml) in acetic acid (9ml) at 15°C under stirring. The reaction mixture was stirred at room temperature for 6 hours. After evaporation of the solvent, the residue was taken up in ethyl acetate and washed with saturated aqueous sodium hydrogencarbonate solution, 5% aqueous sodium thiosulfate and brine, dried over magnesium sulfate and concentrated in vacuo to give 2-bromo-1-(2,4-dichlorophenyl)ethan-1-one (7.09g) as a light yellow oil.
MS(m/z): 269,271(M⁺+H),235(bp)
¹H-NMR (CDCl₃) δ 4.50(2H,s), 7.37(1H,dd,J=8Hz,J=2Hz), 7.48(1H,d,J=2Hz), 7.55(1H,d,J=8Hz)

### Preparation 49

### 2-Azido-1-(2,4-dichlorophenyl)ethan-1-one

The title compound was obtained from the compound of Preparation 48 in substantially the same manner as in Preparation 43.
¹H-NMR (CDCl₃) δ 4.51(2H,s),7.37(1H,dd,J=8Hz,J=2Hz), 7.48(1H,d,J=2Hz),7.60(1H,d,J=8Hz)

### Preparation 50

2-Amino-1-(2,4-dichlorophenyl)ethan-1-one hydrochloride

The title compound was obtained from the compound of Preparation 49 in substantially the same manner as in Preparation 44.
MS(m/z);204(M⁺+H),85(bp)
¹H-NMR (DMSO-d₆) δ 4.51(2H,d,J=7Hz), 7.640H,dd,J=8Hz,J=3Hz), 7.84(1H,d,J=3Hz), 8.00(1H,d,J=8Hz), 8.45(2H,br.s)

### Preparation 51

### (2S)-2-(tert-Butoxycarbonylamino)-N-[2-(2,4-dichlorophenyl)-2-oxoethyl]-3-(2-pyridyl)propionamide

The title compound was obtained from the compound of Preparation 50 in substantially the same manner as in Preparation 5.
MS(m/z): 452,454(M⁺,bp)
¹H-NMR (CDCl₃) δ 1.46(9H,s), 3.16-3.35(2H,m), 4.26(2H,d,J=7Hz), 4.60-4.69(1H,m), 6.19(1H,br.s), 7.72-7.22(2H,m), 7.30(1H,dd,J=8Hz,J=2Hz), 7.43(1H,d,J=2Hz), 7.56(1H,d,J=8Hz), 7.62(1H,ddd,J=8Hz,J=8Hz,J=2Hz), 8.00(1H,br.s), 8.52(1H,d,J=7Hz)

### Preparation 52

### (1S)-(tert-Butoxy)-N-[1-[5-(2,4-dichlorophenyl)-1-methylimidazol-2-yl]-2-(2-pyridyl)ethyl]formamide

The title compound was obtained from the compound of Preparation 51 in substantially the same manner as in Preparation 6.
MS(m/z): 447,449(M⁺,bp)
¹H-NMR (CDCl₃) δ 1.39(9H,s), 3.21(3H,s), 3.43(2H,t,J=7Hz), 5.38(1H,q,J=7Hz), 7.05-7.14(4H,m), 7.20(1H,d,J=8Hz), 7.27(1H,ddd,J=8Hz,J=8Hz,J=2Hz), 7.45-7.55(2H,m), 8.51(1H,d,J=6Hz)

### Preparation 53

### (1S)-1-[5-(2,4-Dichlorophenyl)-1-methylimidazol-2-yl]-2-(2-pyridyl)ethylamine

The title compound was obtained from the compound of Preparation 52 in substantially the same manner as in Preparation 26.
MS(m/z): 347,349(M⁺,bp)
¹H-NMR (CDCl₃) δ 3.29(3H,s), 3.33-3.48(2H,m), 4.58(1H,t,J=7Hz), 6.98(1H,s), 7.09(1H,d,J=7.5Hz), 7.13(1H,t,J=7.5Hz), 7.21(1H,d,J=7.5Hz), 7.30(1H,ddd,J=7.5Hz,J=7.5Hz,J=2Hz), 7.49(1H,d,J=2Hz), 7.57(1H,ddd,J=7Hz,J=7Hz,J=2Hz), 8.55(1H,d,J=7Hz)

### Preparation 54

### 1-[4-(Iminomethoxymethyl)phenyl]ethan-1-one hydrochloride

To a solution of 4-cyanoacetophenone (8.0g) in methanol (80ml) was added a solution of sodium methoxide (298mg) in methanol (5ml) and the mixture was stirred at 60°C for 1 hour. The reaction mixture was cooled and 5N ethanolic hydrogen chloride (5ml) was added thereto. The precipitated sodium chloride was filtered off and the mother liquid was evaporated. The crystalline residue was triturated and washed with diethyl ether (100ml) to give 1-[4-(iminomethoxymethyl)-phenyl]ethan-1-one hydrochloride (2.7g) as white crystals.
mp 105-145°C
¹H-NMR (DMSO-d₆) δ 2.62(3H,s), 2.64(3H,s), 8.02(2H,d,J=8Hz), 8.13(2H,d,J=8Hz)

### Preparation 55

### 1-[4-[(2,2-Diethoxyethylamino)iminomethyl]phenyl]ethan-1-one hydrochloride

A mixture of 1-[4-(iminomethoxymethyl)phenyl]ethan-1-one hydrochloride (3.3g), aminoacetaldehyde diethyl acetal (2.03g) and ethanol (33ml) was refluxed for 4 hours. The solvent was distilled away and the crystalline residue was triturated and washed with diethyl ether (50ml) to give 1-[4-[(2,2-diethoxyethylamino)-iminomethyl]phenyl]ethan-1-one hydrochloride (2.3g) as white crystals.
mp 143-146°C
¹H-NMR (DMSO-d₆) δ 1.15(6H,t,J=6Hz), 2.65(3H,s), 3.50-3.64(4H,m), 3.64-3.78(2H,m) ,4.82(1H,t,J=6Hz), 7.88(2H,d,J=8Hz), 8.13(2H,d,J=8Hz)

### Preparation 56

### 1-[4-(2-Imidazolyl)phenyl]ethan-1-one

A solution of 1-[4-[(2,2-diethoxyethylamino)iminomethyl]phenyl]-ethan-1-one hydrochloride (2.3g) in 6N hydrochloric acid (23ml) was stirred for 15 hours at ambient temperature and then 3 hours at 100 °C. The reaction mixture was evaporated. The crystalline residue was washed with ethanol (20ml). The obtained salt was partitioned between chloroform (100ml) and saturated aqueous sodium hydrogencarbonate solution (100ml). The organic layer was separated, dried over sodium sulfate (20g) and evaporated. The crystalline residue was triturated and washed with ethyl acetate (5ml) to give 1-[4-(2-imidazolyl)phenyl]ethan-1-one (300mg) as white crystals.
mp 213-216°C
ESI-MS: 185(M-1)
¹H-NMR (DMSO-d₆) δ 2.60(3H,s), 7.10-7.40(2H,br), 8.00-8.12(4H,m)

### Preparation 57

### 1-[4-(1-Methylimidazol-2-yl)phenyl]ethan-1-one

To a suspension of sodium hydride (709mg) in tetrahydrofuran (100ml) was added 1-[4-(2-imidazolyl)phenyl]ethan-1-one (5.0g) at 5°C and the mixture was stirred for 1 hour at ambient temperature. The reaction mixture was cooled to -78°C and methyl iodide (4g) was added dropwise. The mixture was warmed to ambient temperature and stirred for 15 hours. The reaction mixture was evaporated and the residue was partitioned between ethyl acetate (100ml) and water (50ml). The organic layer was dried over sodium sulfate (20g) and evaporated. The crystalline residue was triturated and washed with diisopropyl ether (20ml) to give 1-[4-(1-methylimidazol-2-yl)phenyl]ethan-1-one (3.98g) as white crystals.
mp 88-93°C
ESI-MS: 201(M+1)
¹H-NMR (CDCl₃) δ 2.67(3H,s), 3.82(3H,s), 7.03(1H,s), 7.18(1H,s), 7.78(2H,d,J=8Hz), 8.05(2H,d,J=8Hz)

### Preparation 58

### 2-Bromo-1-[4-(1-methylimidazol-2-yl)phenyl]ethan-1-one hydrobromide

To a solution of 1-[4-(1-methylimidazol-2-yl)phenyl]ethan-1-one (1.0g) in acetic acid (10ml) was added 30% hydrogen bromide in acetic acid (2ml) at 5°C and the mixture was stirred for 10 minutes. To the mixture was added bromine (0.24ml) and the mixture was stirred for 1 hour at ambient temperature. The reaction mixture was diluted with diisopropyl ether (20ml) and the precipitate was collected by filtration and washed with diisopropyl ether (10ml). The obtained solid was recrystallized from methanol (10ml) to give 2-bromo-1-[4-(1-methylimidazol-2-yl)phenyl]ethan-1-one hydrobromide (1.2g) as pale brown crystals.
mp 202-206°C
ESI-MS: 278(M+1)
¹H-NMR (DMSO-d₆) δ 3.91(3H,s), 5.05(2H,s), 7.88(1H,s), 7.91(1H,s), 8.00(2H,d,J=8Hz), 8.25(2H,d,J=8Hz)

### Preparation 59

### 2-Azido-1-[4-(1-methylimidazol-2-yl)phenyl]ethan-1-one

To a solution of 2-bromo-1-[4-(1-methylimidazol-2-yl)phenyl]-ethan-1-one hydrobromide (1.13g) in DMF (11ml) was added sodium azide (224mg) at 5°C and the mixture was stirred for 1 hour at ambient temperature. To the reaction mixture was added water (20ml) and the precipitate was collected by filtration and washed with water. The solid was air-dried to give 2-azido-1-[4-(1-methylimidazol-2-yl)-phenyl]ethan-1-one (700mg) as a pale brown solid.
mp >250°C
ESI-MS: 242(M+1)
¹H-NMR (CDCl₃) δ 3.83(3H,s), 4.60(2H,s), 7.04(1H,s), 7.19(1H,s), 7.82(2H,d,J=8Hz), 8.01(2H,d,J=8Hz)

### Preparation 60

### 2-Amino-1-[4-(1-methylimidazol-2-yl)phenyl]ethan-1-one dihydrochloride

A mixture of 2-azido-1-[4-(1-methylimidazol-2-yl)phenyl]ethan-1-one (920mg), palladium on carbon (90mg), methanol (10ml), water (5ml) and concentrated hydrochloric acid (1ml) was stirred under hydrogen (3 atm) at ambient temperature for 4 hours. The reaction mixture was filtered and the filtrate was evaporated. The crystalline residue was triturated and washed with methanol (5ml) to give 2-amino-1-[4-(1-methylimidazol-2-yl)phenyl]ethan-1-one dihydrochloride (500mg) as white crystals.
mp >250°C
ESI-MS: 216(M+1)
¹H-NMR (DMSO-d₆) δ 3.92(3H,s), 4.70(2H,d,J=6Hz), 7.83(1H,s), 7.90(1H,s), 8.05(2H,d,J=8Hz), 8.26(2H,d,J=8Hz), 8.55(2H,br.s)

### Preparation 61

### (2S)-2-(tert-Butoxycarbonylamino)-N-[2-[4-(1-methylimidazol-2-yl)phenyl]-2-oxoethyl]-3-(2-pyridyl)propionamide

The title compound was obtained from the compound of Preparation 60 in substantially the same manner as in Preparation 5.
mp 120-124°C
ESI-MS: 464(M+1)
¹H-NMR (CDCl₃) δ 1.47(9H,s), 3.20-3.45(2H,m), 3.82(3H,s), 4.63-4.75(3H,m), 6.45(1H,br.s), 7.02(1H,s), 7.12-7.19(2H,m), 7.23(1H,d,J=8Hz), 7.59(2H,d,J=8Hz), 7.80(2H,d,J=8Hz), 7.95(1H,br.s), 8.03(2H,d,J=8Hz), 8.57(1H,d,J=4Hz)

### Preparation 62

### (1S)-(tert-Butoxy)-N-[1-[5-[4-(1-methylimidazol-2-yl)phenyl]-1-methylimidazol-2-yl]-2-(2-pyridyl)ethyl]formamide

The title compound was obtained from the compound of Preparation 61 in substantially the same manner as in Preparation 6.
mp 160-163°C
ESI-MS: 459(M+1)
¹H-NMR (CDCl₃) δ 1.38(9H,s), 3.40-3.53(5H,m), 3.80(3H,s), 5.35-5.52(2H,m), 7.00(1H,s), 7.05(1H,s), 7.08-7.18(3H,m), 7.39(2H,d,J=8Hz), 7.55(1H,t,J=8Hz), 7.70(2H,d,J=8Hz), 8.55(1H,d,J=4Hz)

### Preparation 63

### (1S)-1-[5-[4-(1-Methylimidazol-2-yl)phenyl]-1-methylimidazol-2-yl]-2-(2-pyridyl)ethylamine tetrahydrochloride

The title compound was obtained from the compound of Preparation 62 in substantially the same manner as in Preparation 26.
mp >250°C
¹H-NMR (DMSO-d₆) δ 3.85(3H,s), 3.93(3H,s), 5.48(1H,br.s), 7.60(1H,t,J=5Hz), 7.65(1H,s), 7.68(1H,d.J=8Hz), 7.78(2H,d,J=8Hz), 7.86(1H,s), 7.91(1H,s), 8.00(2H,d,J=8Hz), 8.13(1H,t,J=8Hz), 8.68(1H,d,J=5Hz)

### Preparation 64

### 2-Bromo-1-(4-fluorophenyl)propan-1-one

To an ice-cooled solution of 1-(4-fluorophenyl)propan-1-one (10g) in diethyl ether (200ml) was added bromine (10.4g) dropwise and the mixture was stirred for 30 minutes. The reaction mixture was washed with water (100ml), aqueous sodium hydrogencarbonate solution (100ml), aqueous sodium thiosulfate solution (100ml) and brine (50ml), dried over sodium sulfate and evaporated to give 2-bromo-1-(4-fluorophenyl)propan-1-one (18g).
oil
ESI-MS: 232(M+1)
¹H-NMR (CDCl₃) δ 1.91(3H,d,J=6Hz), 5.25(1H,q,J=6Hz), 7.10-7.20(2H,m), 8.04-8.12(2H,m)

### Preparation 65

### 2-[2-(4-Fluorophenyl)-1-methyl-2-oxoethyl]isoindoline-1,3-dione

The title compound was obtained from the compound of Preparation 64 in substantially the same manner as in Preparation 21.
ESI-MS: 298(M+1)
¹H-NMR (CDCl₃) δ 1.72(3H,d,J=6Hz), 5.63(1H,q,J=6Hz), 7.01-7.13(2H,m), 7.68-7.90(6H,m)

### Preparation 66

### 2-[2-[4-(1-Imidazolyl)phenyl]-1-methyl-2-oxoethyl]isoindoline-1,3-dione

The title compound was obtained from the compound of Preparation 65 in substantially the same manner as in Preparation 22.
ESI-MS: 346(M+1)
¹H-NMR (CDCl₃) δ 1.74(3H,d,J=6Hz), 5.66(1H,q,J=6Hz), 7.21(1H,s), 7.30(1H,s), 7.43(2H,d,J=8Hz), 7.69-7.75(2H,m), 7.80-7.86(2H,m), 7.88(1H,s), 7.95(2H,d,J=8Hz)

### Preparation 67

### 2-Amino-1-[4-(1-imidazolyl)phenyl]propan-1-one dihydrochloride

The title compound was obtained from the compound of Preparation 66 in substantially the same manner as in Preparation 23.
ESI-MS: 216(M+1)
¹H-NMR (DMSO-d₆) δ 1.45(3H,d,J=6Hz), 5.15-5.30(1H,m), 7.91(1H,s), 8.09(2H,d,J=8Hz), 8.33(2H,d,J=8Hz), 8.42(1H,s), 8.61(2H,s), 9.80(1H,s)

### Preparation 68

### (2S)-2-(tert-Butoxycarbonylamino)-N-[2-[4-(1-imidazolyl)phenyl]-1-methyl-2-oxoethyl]-3-(2-pyridyl)propionamide

The title compound was obtained from the compound of Preparation 67 in substantially the same manner as in Preparation 5.
amorphous solid
ESI-MS: 464(M+1)
¹H-NMR (CDCl₃) δ 1.25(3x2/5H,d,J=6Hz), 1.37(3x3/5H,d,J=6Hz), 1.45(9H,s), 3.15-3.45(2H,m), 5.47(1H,q,J=6Hz), 7.07-7.24(2H,m), 7.36(1H,s), 7.45-7.83(4H,m), 7.95(1H,s), 8.08(2H,d,J=8Hz), 8.48(1x2/5H,d,J=4Hz), 8.54(1x3/5H,d,J=4Hz)

### Preparation 69

### (1S)-(tert-Butoxy)-N-[1-[5-[4-(1-imidazolyl)phenyl]-1,4-dimethylimidazol-2-yl]-2-(2-pyridyl)ethyl]formamide

The title compound was obtained from the compound of Preparation 68 in substantially the same manner as in Preparation 6.
amorphous solid
ESI-MS: 459(M+1)
¹H-NMR (CDCl₃) δ 1.36(9H,s), 2.20(3H,s), 3.35(3H,s), 3.43(2H,d,J=6Hz), 3.47(2H,d,J=8Hz), 5.30-5.55(2H,m), 7.08-7.18(2H,m), 7.24(1H,s), 7.30-7.37(3H,m), 7.55(2H,d,J=8Hz), 7.80(1H,d,J=8Hz), 7.90(1H,s), 8.57(1H,d,J=4Hz)

### Preparation 70

### (1S)-1-[5-[4-(1-Imidazolyl)phenyl]-1,4-dimethylimidazol-2-yl]-2-(2-pyridyl)ethylamine tetrahydrochloride

The title compound was obtained from the compound of Preparation 69 in substantially the same manner as in Preparation 26.
ESI-MS: 359(M+1)
¹H-NMR (DMSO-d₆) δ 2.26(3H,s), 3.81(3H,s), 3.90-4.10(2H,m), 5.60(1H,t,J=6Hz), 7.63(1H,t,J=8Hz), 7.70-7.80(2H,m), 8.00(2H,s), 8.07(2H,d,J=8Hz), 8.16(1H,t,J=8Hz), 8.44(1H,s), 8.69(1H,d,J=4Hz)

### Preparation 71

### (1S)-(tert-Butoxy)-N-[1-[1-ethyl-5-[4-(1-imidazolyl)phenyl]-imidazol-2-yl]-2-(2-pyridyl)ethyl]formamide

The title compound was obtained from the compound of Preparation 14 in substantially the same manner as in Preparation 6.
oil
MS: 459(M+1)
¹H-NMR (CDCl₃) δ 1.18(3H,t,J=8Hz), 1.40(9H,s), 3.42-3.52(1H,m), 3.53-3.70(1H,m), 3.95-4.12(2H,m), 5.50(1H,q,J=8Hz), 5.70(1H,br s), 7.08(1H,s), 7.10-7.20(2H,m), 7.21-7.30(2H,m), 7.31(1H,s), 7.40-7.51(3H,m), 7.58(1H,t,J=8Hz), 7.90(1H,s), 8.52(1H,d,J=2Hz)

### Preparation 72

### (1S)-1-[1-Ethyl-5-[4-(1-imidazolyl)phenyl]imidazol-2-yl]-2-(2-pyridyl)ethylamine

The title compound was obtained from the compound of Preparation 71 in substantially the same manner as in Preparation 7.
oil
MS: 359(M+1)
¹H-NMR (CDCl₃) δ 1.20(3H,t,J=8Hz), 3.35-3.60(2H,m), 3.90-4.17(2H,m), 4.62-4.72(1H,m), 7.03(1H,s), 7.18(2H,d,J=8Hz), 7.23(2H,d,J=8Hz), 7.31(1H,s), 7.40-7.50(2H,m), 7.61(1H,t,J=8Hz), 7.89-7.92(2H,m), 8.59(1H,d,J=2Hz)

### Preparation 73

### (1S)-(tert-Butoxy)-N-[1-[5-[4-(1-imidazolyl)phenyl]-1-propylimidazol-2-yl]-2-(2-pyridyl)ethyl]formamide

The title compound was obtained from the compound of Preparation 14 in substantially the same manner as in Preparation 6.
MS(ESI)m/z: 473(M+H)⁺
¹H-NMR (CDCl₃,300MHz) δ : 0.76(3H,t,J=7Hz), 1.38(9H,s), 1.40-1.60(2H,m), 3.48-3.80(2H,m), 3.88-4.08(2H,m), 5.40-5.60(2H,m), 7.02-7.65(10H,m), 7.92(1H,s), 8.52(1H,d,J=5Hz)

### Preparation 74

### (1S)-1-[5-[4-(1-Imidazolyl)phenyl]-1-propylimidazol-2-yl]-2-(2-pyridyl)ethylamine

The title compound was obtained from the compound of Preparation 73 in substantially the same manner as in Preparation 26.
MS(ESI)m/z: 373(M+H)⁺
¹H-NMR (CDCl₃,300MHz) δ : 0.78(3H,t,J=7Hz), 1.36-1.72(2H,m), 3.42-3.74(2H,m), 3.85-4.24(2H,m), 4.81-5.02(1H,m), 7.08(1H,s), 7.15-7.72(9H,m), 7.93(1H,s), 8.55(1H,d,J=5Hz)

### Preparation 75

### 2-[2-[4-(1-Benzimidazolyl)phenyl]-2-oxoethyl]isoindoline-1,3-dione

The title compound was obtained from the compound of Preparation 21 in substantially the same manner as in Preparation 22.
mp 290-295°C (decomposition)
MS(m/z): 382(M⁺+H),119(bp)
¹H-NMR (CDCl₃) δ 5.20(2H,s), 7.38-7.43(2H,m), 7.61-7.66(1H,m), 7.73(2H,d,J=8Hz), 7.77-7.82(2H,m), 7.89-7.95(3H,m), 8.24(3H,t,J=7Hz)

### Preparation 76

### 2-Amino-1-[4-(1-benzimidazolyl)phenyl]ethan-1-one dihydrochloride

The title compound was obtained from the compound of Preparation 75 in substantially the same manner as in Preparation 23.
mp 295-300°C (decomposition)
MS(m/z): 252(M⁺+H),85(bp)
¹H-NMR (DMSO-d₆) δ 4.69(2H,d,J=7Hz), 7.52-7.58(2H,m), 7.80-7.85(1H,m), 7.91-7.96(1H,m), 8.04(2H,d,J=8Hz), 8.32(2H,d,J=8Hz), 8.58(2H,s), 9.49(1H,s)

### Preparation 77

### (2S)-N-[2-[4-(1-Benzimidazolyl)phenyl]-2-oxoethyl]-2-(tert-butoxycarbonylamino)-3-(2-pyridyl)propionamide

The title compound was obtained from the compound of Preparation 76 in substantially the same manner as in Preparation 5.
MS(m/z): 500(M⁺+H,bp)
¹H-NMR (CDCl₃) δ 1.49(9H,s), 3.24-3.50(3H,m), 4.77(2H,d,J=7Hz), 7.15-7.24(2H,m), 7.37-7.40(3H,m), 7.59-7.63(2H,m), 7.66-7.70(3H,m), 7.89-7.93(1H,m), 8.02(1H,s), 8.14-8.19(2H,m),8.58(1H,d,J=7Hz)

### Preparation 78

### (1S)-N-[1-[5-[4-(1-Benzimidazolyl)phenyl]-1-methylimidazol-2-yl]-2-(2-pyridyl)ethyl](tert-butoxy)formamide

The title compound was obtained from the compound of Preparation 77 in substantially the same manner as in Preparation 6.
MS(m/z): 495(M⁺+H,bp)
¹H-NMR (CDCl₃) δ 1.40(9H,s), 3.49(2H,d,J=7Hz), 3.57(3H,s), 5.45-5.50(2H,m), 7.10(1H,s), 7.15(2H,d,J=7.5Hz), 7.36-7.40(2H,m), 7.51-7.60(6H,m), 7.89-7.93(1H,m), 8.17(1H,s), 8.58(1H,d,J=7Hz)

### Preparation 79

### (1S)-1-[5-[4-(1-Benzimidazolyl)phenyl]-1-methylimidazol-2-yl]-2-(2-pyridyl)ethylamine

The title compound was obtained from the compound of Preparation 78 in substantially the same manner as in Preparation 26.
MS(m/z): 395(M⁺+H,bp)
¹H-NMR (CDCl₃) δ 3.32-3.52(2H,m), 3.62(3H,s), 4.64(1H,dd,J=8Hz,J=5Hz), 7.14-7.20(2H,m), 7.27(1H,s), 7.33-7.40(2H,m), 7.50-7.69(6H,m), 7.89-7.95(1H,m), 8.18(1H,s), 8.61(1H,d,J=7Hz)

### Preparation 80

### 2-[2-[4-(2-Ethylimidazol-1-yl)phenyl]-2-oxoethyl]isoindoline-1,3-dione

The title compound was obtained from the compound of Preparation 21 in substantially the same manner as in Preparation 22.
MS(ES+): 360(M+H)
¹H-NMR (300MHz,CDCl₃) δ 1.31(3H,t,J=7Hz), 2.74(2H,q,J=7Hz), 5.17(2H,s), 7.06(1H,s), 7.11(1H,s), 7.49(2H,d,J=8Hz), 7.74-7.83(2H,m), 7.89-7.98(2H,m), 8.17(2H,d,J=8Hz)

### Preparation 81

### 2-Amino-1-[4-(2-ethylimidazol-1-yl)phenyl]ethan-1-one dihydrochloride

The title compound was obtained from the compound of Preparation 80 in substantially the same manner as in Preparation 23.
MS(ES+): 230(M+H)
¹H-NMR (300MHz,DMSO-d₆) δ 1.22(3H,t,J=7Hz), 2.93(2H,q,J=7Hz), 4.62-4.74(2H,m), 7.85(1H,s), 7.90(2H,d,J=8Hz), 7.98(1H,s), 8.29(2H,d,J=8Hz), 8.66(2H,br)

### Preparation 82

### (2S)-2-(tert-Butoxycarbonylamino)-N-[2-[4-(2-ethylimidazol-1-yl)-phenyl]-2-oxoethyl]-3-(2-pyridyl)propionamide

The title compound was obtained from the compound of Preparation 81 in substantially the same manner as in Preparation 5.
MS(ES+): 478(M+H)
¹H-NMR (300MHz,CDCl₃) δ 1.28(3H,t,J=7Hz), 1.47(9H,s), 2.71(2H,q,J=7Hz), 3.27(1H,m), 3.39(1H,m), 4.65-4.77(3H,m), 6.46(1H,m), 7.02(1H,s), 7.10(1H,s), 7.16(1H,m), 7.22(1H,d,J=8Hz), 7.42(2H,d,J=8Hz), 7.61(1H,m), 8.01(1H,br), 8.07(2H,d,J=8Hz), 8.56(1H,d,J=5Hz)

### Preparation 83

### (1S)-(tert-Butoxy)-N-[1-[5-[4-(2-ethylimidazol-1-yl)phenyl]-1-methylimidazol-2-yl]-2-(2-pyridyl)ethyl]formamide

The title compound was obtained from the compound of Preparation 82 in substantially the same manner as in Preparation 6.
MS(ES+): 473(M+H)
¹H-NMR (300MHz,CDCl₃) δ 1.28(3H,t,J=7Hz), 1.38(9H,s), 2.70(2H,q,J=7Hz), 3.46(2H,d,J=8Hz), 3.52(3H,s), 5.35-5.65(2H,m), 7.01(1H,s), 7.03-7.18(4H,m), 7.34(2H,d,J=8Hz), 7.42(2H,d,J=8Hz), 7.57(1H,m), 8.56(1H,m)

### Preparation 84

### (1S)-1-[5-[4-(2-Ethylimidazol-1-yl)phenyl]-1-methylimidazol-2-yl]-2-(2-pyridyl)ethylamine

The title compound was obtained from the compound of Preparation 83 in substantially the same manner as in Preparation 26.
MS(ES+): 373(M+H)
¹H-NMR (300MHz,CDCl₃) δ 1.29(3H,t,J=7Hz), 1.94(2H,br), 2.71(2H,q,J=7Hz), 3.34(1H,m), 3.47(1H,m). 3.60(3H,s), 4.62(1H,m), 7.14-7.21(4H,m), 7.34(2H,d,J=8Hz), 7.43(2H,d,J=8Hz), 7.60(1H,dd,J=8Hz,J=8Hz), 8.59(1H,m)

### Preparation 85

### 1-[4-(3-Pyridyl)phenyl]ethan-1-one

A mixture of diethyl(3-pyridyl)borane (1.66g), 4-bromo-acetophenone (3.37g), powdered potassium hydroxide (1.9g), tetrabutylammonium bromide (1.82g) and tetrakis(triphenylphosphine)-palladium (1.3g) in tetrahydrofuran (45ml) was refluxed under nitrogen for 1.5 hours. After removal of the solvent, the catalyst was removed and washed with ethyl acetate. The filtrate was washed with saturated aqueous sodium hydrogencarbonate solution and brine, and dried over sodium sulfate. After removal of the solvent, the residue was purified by silica gel column chromatography with ethyl acetate-hexane (1:1) as an eluent to give 1-[4-(3-pyridyl)phenyl]ethan-1-one (2.0g) as crystals.
MS(ES+): 198(M+H)
¹H-NMR (300MHz,CDCl₃) δ 2.68(3H,s), 7.41(1H,m), 7.69(2H,d,J=8Hz), 7.93(1H,m), 8.08(2H,d,J=8Hz), 8.65(1H,m), 8.89(1H,m)

### Preparation 86

### 2-Bromo-1-[4-(3-pyridyl)phenyl]ethan-1-one hydrobromide

The title compound was obtained from the compound of Preparation 85 in substantially the same manner as in Preparation 58.
MS(ES+): 357(M+H)
¹H-NMR (300MHz,DMSO-d₆) δ 5.02(2H,s), 8.02-8.13(3H,m), 8.17(2H,d,J=8Hz), 8.85(1H,m), 8.91(1H,m), 9.30(1H,s)

### Preparation 87

### 2-Azido-1-[4-(3-pyridyl)phenyl]ethan-1-one

The title compound was obtained from the compound of Preparation 86 in substantially the same manner as in Preparation 59.
MS(ES+): 239(M+H)
¹H-NMR (300MHz,CDCl₃) δ 4.61(2H,s), 7.42(1H,m), (7.73(2H,d,J=8Hz), 7.93(1H,m). 8.04(2H,d,J=8Hz), 8.66(1H,m), 8.89(1H,m)

### Preparation 88

### 2-Amino-1-[4-(3-pyridyl)phenyl]ethan-1-one dihydrochloride

The title compound was obtained from the compound of Preparation 87 in substantially the same manner as in Preparation 60.
MS(ES+): 213(M(free)+H)
¹H-NMR (300MHz,DMSO-d₆) δ 4.60-4.76(2H,m), 7.99(1H,m), 8.11(2H,d,J=8Hz), 8.19(2H,d,J=8Hz), 8.52(2H,br), 8.75(1H,d,J=8Hz), 8.88(1H,d,J=5Hz), 9.28(1H,m)

### Preparation 89

### (2S)-2- (tert-Butoxycarbonylamino)-N-[2-[4-(3-pyridyl)phenyl]-2-oxoethyl]-3-(2-pyridyl)propionamide

The title compound was obtained from the compound of Preparation 88 in substantially the same manner as in Preparation 5.
MS(ES+): 461(M+H)
¹H-NMR (300MHz,CDCl₃) δ 1.48(9H,s), 3.18-3.48(2H,m), 4.60-4.81(3H,m), 6.45(1H,m), 7.10-7.31(2H,m), 7.40(1H,m), 7.59(1H,m), 7.68(2H,d,J=8Hz), 7.86-8.14(4H,m), 8.57(1H,d,J=5Hz), 8.65(1H,m), 8.88(1H,m)

### Preparation 90

### (1S)-(tert-Butoxy)-N-[1-[1-methyl-5-[4-(3-pyridyl)phenyl]-imidazol-2-yl]-2-(2-pyridyl)ethyl]formamide

The title compound was obtained from the compound of Preparation 89 in substantially the same manner as in Preparation 6.
MS(ES+): 456(M+H)
¹H-NMR (300MHz,CDCl₃) δ 1.38(9H,s), 3.40-3.56(5H,m), 5.35-5.56(2H,m), 7.06(1H,s), 7.07-7.19(2H,m), 7.34-7.48(3H,m), 7.56(1H,dd,J=8Hz,J=8Hz), 7.63(2H,d,J=8Hz), 7.91(1H,m), 8.54(1H,m), 8.62(1H,m), 8.87(1H,m)

### Preparation 91

### (1S)-1-[1-Methyl-5-[4-(3-pyridyl)phenyl]imidazol-2-yl]-2-(2-pyridyl)ethylamine

The title compound was obtained from the compound of Preparation 90 in substantially the same manner as in Preparation 26.
MS(ES+): 356(M+H)
¹H-NMR (300MHz,CDCl₃) δ 1.85(2H,br.s), 3.34(1H,m), 3.46(1H,m), 3.58(3H,s), 4.62(1H,m), 7.10(1H,s), 7.12-7.22(2H,m), 7.34-7.53(3H,m),7.55-7.74(3H,m), 7.90(1H,m), 8.52-8.69(2H,m), 8.88(1H,s)

### Preparation 92

### (1S)-(tert-Butoxy)-N-[1-[1-methyl-5-[4-(2-pyridyl)phenyl]-imidazol-2-yl]-2-(2-pyridyl)ethyl]formamide

(1S)-(tert-Butoxy)-N-[1-[5-(4-bromophenyl)-1-methylimidazol-2-yl]-2-(2-pyridyl)ethyl]formamide (200mg), 2-pyridyl trifluoromethanesulfonate (99mg), tetrakis(triphenylphosphine)palladium (25mg) and anhydrous lithium chloride (55mg) were mixed under nitrogen. Hexamethylditin (143mg) and anhydrous 1,4-dioxane (10ml) were added successively, and the mixture was refluxed for 23 hours. The cooled mixture was poured into a mixture of saturated aqueous potassium fluoride (25ml) and ethyl acetate, and the mixture was vigorously stirred for 2 hours. The two phase mixture was filtered through a bed of Celite and the separated organic layer was extracted with 1N hydrochloric acid (10ml×2). The aqueous layer was basified (pH 9) with sodium hydrogencarbonate and extracted with chloroform. The organic layer was dried over sodium sulfate and concentrated in vacuo to give (1S)-(tert-butoxy)-N-[1-[1-methyl-5-[4-(2-pyridyl)phenyl]-imidazol-2-yl]-2-(2-pyridyl)ethyl]formamide (220mg) as a yellow amorphous solid.
MS(ES+): 456(M+H)
¹H-NMR (300MHz,CDCl₃) δ 1.36(9H,s), 3.39-3.54(5H,m), 5.42(1H,m), 5.52(1H,m), 7.02-7.86(10H,m), 8.05(1H,d,J=8Hz), 8.55(1H,m), 8.71(1H,m)

### Preparation 93

### (1S)-1-[1-Methyl-5-[4-(2-pyridyl)phenyl]imidazol-2-yl]-2-(2-pyridyl)ethylamine

The title compound was obtained from the compound of Preparation 92 in substantially the same manner as in Preparation 26.
MS(ES+): 356(M+H)
¹H-NMR (300MHz,CDCl₃) δ 1.76(2H,br), 3.24-3.69(5H,m), 4.60(1H,br), 6.96-8.15(11H,m), 8.58(1H,m), 8.70(1H,m)

### Preparation 94

### (1S)-(tert-Butoxy)-N-[1-[1-methyl-5-[4-(4-pyridyl)phenyl]-imidazol-2-yl]-2-(2-pyridyl)ethyl]formamide

A mixture of trimethyl(4-pyridyl)stannane (222mg), (1S)-(tert-butoxy)-N-[1-[5-(4-bromophenyl)-1-methylimidazol-2-yl]-2-(2-pyridyl)-ethyl]formamide (350mg), bis(triphenylphosphine)palladium chloride (53.7mg) and anhydrous lithium chloride (55mg) in 1,4-dioxane (10ml) was refluxed for 24 hours. The cooled mixture was poured into a mixture of saturated aqueous potassium fluoride solution (25ml) and ethyl acetate, and the mixture was vigorously stirred for 2 hours. The two phase mixture was filtered through a bed of Celite and the separated organic layer was extracted with 1N hydrochloric acid (10ml ×2). The aqueous layer was basified (pH 9) with sodium hydrogencarbonate and extracted with chloroform. The organic layer was dried over sodium sulfate and concentrated in vacuo. The residue was purified by silica gel column chromatography (3% methanol in chloroform) to give (1S)-(tert-butoxy)-N-[1-[1-methyl-5-[4-(4-pyridyl)-phenyl]imidazol-2-yl]-2-(2-pyridyl)ethyl]formamide (135mg).
MS(ES+): 456(M+H)
¹H-NMR (300MHz,CDCl₃) δ 1.37(9H,s), 3.37-3.56(5H,m), 5.34-5.58(2H,m), 7.07(1H,s), 7.09-7.23(2H,m), 7.43(2H,d,J=8Hz), 7.48-7.63(3H,m), 7.70(2H,d,J=8Hz), 8.55(1H,m), 8.68(2H,d,J=8Hz)

### Preparation 95

### (1S)-1-[1-Methyl-5-[4-(4-pyridyl)phenyl]imidazol-2-yl]-2-(2-pyridyl)ethylamine

The title compound was obtained from the compound of Preparation 94 in substantially the same manner as in Preparation 26.
MS(ES+): 356(M+H)
¹H-NMR (300MHz,CDCl₃) δ 1.93(2H,s), 3.35(1H,m), 3.46(1H,m), 3.59(3H,s), 4.63(1H,m), 7.06-7.23(3H,m), 7.39-7.76(7H,m), 8.59(1H,m), 8.68(2H,d,J=7Hz)

### Preparation 96

### (1S)-(tert-Butoxy)-N-[1-[1-methyl-5-[4-(5-thiazolyl)phenyl]-imidazol-2-yl]-2-(2-pyridyl)ethyl]formamide

The title compound was obtained from the compound of Preparation 28 and 5-(trimethylstannyl)thiazole in substantially the same manner as in Preparation 29.
MS(ES+): 462(M+H)
¹H-NMR (300MHz,CDCl₃) δ 1.39(9H,s), 3.40-3.66(5H,m), 7.07(1H,s), 7.10-7.18(2H,m), 7.35(2H,d,J=8Hz), 7.57(1H,m), 7.65(2H,d,J=8Hz), 8.13(1H,s), 8.52(1H,m), 8.80(1H,s)

### Preparation 97

### (1S)-1-[1-Methyl-5-[4-(5-thiazolyl)phenyl]imidazol-2-yl]-2-(2-pyridyl)ethylamine

The title compound was obtained from the compound of Preparation 96 in substantially the same manner as in Preparation 26.
MS(ES+): 362(M+H)
¹H-NMR (300MHz,CDCl₃) δ 1.75(2H,br), 3.33(1H,m), 3.46(1H,m), 3.58(3H,s), 4.51(1H,br), 7.08(1H,s), 7.12-7.21(2H,m), 7.38(2H,d,J=8Hz), 7.56-7.68(3H,m), 8.12(1H,s), 8.59(1H,m), 8.78(1H,s)

### Preparation 98

### (2S)-2-(tert-Butoxycarbonylamino)-N-[2-[4-(1-imidazolyl)phenyl]-2-oxoethyl]-3-(4-pyridyl)propionamide

The title compound was obtained from the compound of Preparation 13 in substantially the same manner as in Preparation 5.
amorphous solid
ESI-MS: 450(M+1)
¹H-NMR (CDCl₃) δ : 1.42(9H,s), 3.00-3.37(2H,m), 4.58(1H,br s), 4.65-4.85(2H,m), 5.08(1H,d,J=6Hz), 7.07(1H,br s), 7.t8(2H,d,J=8Hz), 7.38(1H,s), 7.55(2H,d,J=8Hz), 7.98(1H,s), 8.10(2H,d,J=8Hz), 8.55(2H,d,J=8Hz)

### Preparation 99

### (1S)-(tert-Butoxy)-N-[1-[5-[4-(1-imidazolyl)phenyl]-1-propylimidazol-2-yl]-2-(4-pyridyl)ethyl]formamide

The title compound was obtained from the compound of Preparation 98 in substantially the same manner as in Preparation 6.
MS(m/z): 473(M⁺+H,bp)
¹H-NMR (CDCl₃) δ 0.94(3H,t,J=7Hz), 1.40(9H,s), 3.19-3.25(2H,m), 3.31-3.50(2H,m), 5.12(1H,d,J=8Hz), 5.28(1H,d,J=8Hz), 7.07(1H,s), 7.10-7.18(2H,m), 7.32(1H,s), 7.37-7.50(4H,m), 7.91(2H,s), 8.45-8.60(3H,m)

### Preparation 100

### (1S)-1-[5-[4-(1-Imidazolyl)phenyl]-1-propylimidazol-2-yl]-2-(4-pyridyl)ethylamine

The title compound was obtained from the compound of Preparation 99 in substantially the same manner as in Preparation 26.
MS(m/z): 346(M⁺+H,bp)
¹H-NMR (CDCl₃) δ 0.69(3H,t,J=7Hz), 1.30-1.50(2H,m), 3.23-3.42(2H,m), 3.55-3.65(1H,m), 3.75-3.84(1H,m), 4.28(1H,t,J=7Hz), 7.10(3H,t,J=3Hz), 7.25(1H,s), 7.33(1H,s), 7.45(4H,dd,J=7Hz,7Hz), 7.93(1H,s), 8.53(2H,d,J=3Hz)

### Preparation 101

### (1S)-(tert-Butoxy)-N-[1-[1-ethyl-5-[4-(1-imidazolyl)phenyl]-imidazol-2-yl]-2-(4-pyridyl)ethyl]formamide

The title compound was obtained from the compound of Preparation 98 in substantially the same manner as in Preparation 6.
MS(m/z): 459(M⁺+H),42(bp)
¹H-NMR (CDCl₃) δ 1.00(3H,t,J=7Hz), 1.41(9H,s), 3.22-3.39(3H,m), 5.12(1H,d,J=8Hz), 5.30(1H,d,J=8Hz), 7.08(1H,s), 7.10-7.18(3H,m), 7.34(1H,s), 7.40-7.49(4H,m), 7.89-7.92(2H,m), 8.49-8.55(3H,m)

### Preparation 102

### (1S)-1-[1-Ethyl-5-[4-(1-imidazolyl)phenyl]imidazol-2-yl]-2-(4-pyridyl)ethylamine

The title compound was obtained from the compound of Preparation 101 in substantially the same manner as in Preparation 26.
MS(m/z): 359(M⁺+H,bp)
¹H-NMR (CDCl₃) δ 1.09(3H,t,J=7Hz), 3.15-3.22(1H,m), 3.29-3.40(1H,m), 3.70-3.80(1H,m), 3.89-3.97(1H,m), 4.18(1H,d,J=7Hz), 7.09(1H,s), 7.10(2H,d,J=7.5Hz), 7.25(1H,s), 7.33(1H,d,J=3Hz), 7.42-7.49(4H,m), 7.91(1H,d,J=3Hz), 8.52(2H,d,J=7.5Hz)

### Preparation 103

### (1S)-1-[5-(4-Bromophenyl)-1-methylimidazol-2-yl]-2-(2-pyridyl)-ethylamine

The title compound was obtained from the compound of Preparation 28 in substantially the same manner as in Preparation 26.
MS(ESI)m/z: 357,359(M+H)⁺
¹H-NMR (CDCl₃,300MHz) δ 3.23-3.47(2H,m), 3.49(3H,s), 4.59(1H,t,J=7Hz), 7.01(1H,s), 7.05-7.22(4H,m), 7.54(2H,d,J=8Hz), 7.55-7.64(1H,m), 8.57(1H,d,J=5Hz)

### Example 1

### (1S)-(5-Chlorobenzo[b]furan-2-yl)-N-[1-[1-methyl-5-(4-morpholinophenyl)imidazol-2-yl]-2-(2-pyridyl)ethyl]formamide

To a solution of (1S)-1-[1-methyl-5-(4-morpholinophenyl)imidazol-2-yl]-2-(2-pyridyl)ethylamine (120mg), 5-chlorobenzo[b]furan-2-carboxylic acid (68.1mg) and 1-hydroxybenzotriazole (49.1mg) in N,N-dimethylformamide (2.0ml) was added 1-(3-dimethylaminopropyl)-3-ethylcarbodiimide hydrochloride (69.6mg). The mixture was stirred at ambient temperature for 2 hours and allowed to stand overnight. The resulting mixture was diluted with water (20ml) and extracted with ethyl acetate (25ml). The organic layer was extracted with 1N hydrochloric acid (15ml) and the aqueous layer was basified with saturated aqueous sodium hydrogencarbonate solution, then extracted with ethyl acetate (20ml). The organic layer was washed with brine, dried over magnesium sulfate and concentrated in vacuo. The residual solid was treated with hot acetonitrile (1.5ml) and the mixture was cooled to ambient temperature. The solid was collected by filtration and washed with acetonitrile to give (1S)-(5-chlorobenzo[b]furan-2-yl)-N-[1-[1-methyl-5-(4-morpholinophenyl)imidazol-2-yl]-2-(2-pyridyl)-ethyl]formamide (89mg) as off-white crystals.
mp 162-164°C
ESI-MS: 542(M+)
¹H-NMR (300MHz, DMSO-d₆) δ 3.11-3.17(4H,m), 3.42-3.60(2H,m), 3.53(3H,s), 3.70-3.77(4H,m), 5.83(1H,q,J=8Hz), 6.88(1H,s), 6.99(2H,d,J=9Hz), 7.18(1H,dd,J=5,8Hz), 7.26(2H,d,J=9Hz), 7.30(1H,d,J=8Hz), 7.47(1H,d,J=8Hz), 7.59(1H,s), 7.63(1H,d,J=8Hz), 7.68(1H,d,J=8Hz), 7.87(1H,s), 8.48(1H,d,J=5Hz), 9.32(1H,d,J=8Hz)
[α]_{D}=171.90 (CHCl₃, c=1.030%)

### Example 2

### (1S)-(5-Bromobenzo[b]furan-2-yl)-N-[1-[1-methyl-5-(4-morpholinophenyl)imidazol-2-yl]-2-(2-pyridyl)ethyl]formamide

The title compound was obtained from the compound of Preparation 7 in substantially the same manner as in Example 1.
mp 173-174°C
ESI-MS: 586.2(M+)
¹H-NMR (300MHz,DMSO-d₆) δ 3.11-3.18(4H,m), 3.42-3.61(2H,m), 3.54(3H,s), 3.70-3.78(4H,m), 5.83(1H,q,J=8Hz), 6.88(1H,s), 6.99(2H,d,J=9Hz), 7.18(1H,dd,J=8Hz,J=5Hz), 7.26(2H,d,J=9Hz), 7.30(1H,d,J=8Hz), 7.57-7.68(4H,m), 8.02(1H,s), 8.48(1H,d,J=5Hz), 9.32(1H,d,J=8Hz)

### Example 3

### (1S)-(5-Methoxybenzo[b]furan-2-yl)-N-[1-[1-methyl-5-(4-morpholinophenyl)imidazol-2-yl]-2-(2-pyridyl)ethyl]formamide

The title compound was obtained from the compound of Preparation 7 in substantially the same manner as in Example 1.
mp 122-124°C
ESI-MS: 538(M+H)
¹H-NMR (300MHz,DMSO-d₆) δ 3.11-3.18(4H,m), 3.42-3.62(2H,m), 3.53(3H,s), 3.71-3.77(4H,m), 3.80(3H,s), 5.82(1H,q,J=8Hz), 6.87(1H,s), 7.00(2H,d,J=9Hz), 7.03(1H,d,J=8Hz), 7.18(1H,dd,J=8Hz), 7.26(2H,d,J=9Hz), 7.27-7.33(2H,m), 7.52(1H,s), 7.53(1H,d,J=8Hz), 7.65(1H,t,J=8Hz), 8.49(1H,d,J=5Hz), 9.17(1H,d,J=8Hz)

### Example 4

### (1S)-(5-Fluorobenzo[b]furan-2-yl)-N-[1-[1-methyl-5-(4-morpholinophenyl)imidazol-2-yl]-2-(2-pyridyl)ethyl]formamide

The title compound was obtained from the compound of Preparation 7 in substantially the same manner as in Example 1.
mp 130-132°C
ESI-MS: 526(M+H)
¹H-NMR (300MHz,DMSO-d₆) δ 3.10-3.20(4H,m), 3.42-3.62(2H,m), 3.53(3H,s), 3.70-3.80(4H,m), 5.83(1H,q,J=8Hz), 6.88(1H,s), 7.00(2H,d,J=9Hz), 7.18(1H,dd,J=8Hz,J=5Hz), 7.23-7.38(4H,m), 7.57-7.73(4H,m), 8.49(1H,d,J=5Hz), 9.29(1H,d,J=8Hz)

### Example 5

### (1S)-(5-Methylbenzo[b]furan-2-yl)-N-[1-[1-methyl-5-(4-morpholinophenyl)inidazol-2-yl]-2-(2-pyridyl)ethyl]formamide

The title compound was obtained from the compound of Preparation 7 in substantially the same manner as in Example 1.
mp 125-127°C
ESI-MS: 522(M+H)
¹H-NMR (300MHz,DMSO-d₆) δ 2.40(3H,s), 3.11-3.18(4H,m), 3.48(1H,dd,J=15Hz,J=8Hz), 3.52-3.60(1H,m), 3.54(3H,s), 3.70-3.78(4H,m), 5.83(1H,q,J=8Hz), 6.87(1H,s), 7.00(2H,d,J=9Hz), 7.18(1H,dd,J=8Hz,J=5Hz), 7.23-7.32(4H,m), 7.49-7.56(3H,m), 7.56(1H,t,J=8Hz), 8.48(1H,d,J=5Hz), 9.17(1H,d,J=8Hz)

### Example 6

### (1S)-(5-Chlorobenzothiazol-2-yl)-N-[1-[1-methyl-5-(4-morpholinophenyl)imidazol-2-yl]-2-(2-pyridyl)ethyl]formamide

The title compound was obtained from the compound of Preparation 7 in substantially the same manner as in Example 1.
mp 96-99°C
MS(ES+): 559(M+)
¹H-NMR (300MHz,CDCl₃) δ 3.18-3.21(4H,m), 3.50(3H,s), 3.56(2H,d,J=7Hz), 3.85-3.86(4H,m), 5.93(1H,m), 6.93(2H,d,J=8Hz), 6.98(1H,s), 7.09-7.15(2H,m), 7.20(2H,d,J=8Hz), 7.45(1H,d,J=8Hz), 7.53(1H,dd,J=8Hz,J=8Hz), 7.85(1H,d,J=8Hz), 8.06(1H,s), 8.30(1H,d,J=8Hz), 8.54(1H,d,J=5Hz)

### Example 7

### (1S)-(5-Chlorobenzo[b]thiophen-2-yl)-N-[1-[1-methyl-5-(4-morpholinophenyl)imidazol-2-yl]-2-(2-pyridyl)ethyl]formamide

The title compound was obtained from the compound of Preparation 7 in substantially the same manner as in Example 1.
ESI-MS: 558.2(M+)
¹H-NMR (300MHz,DMSO-d₆) δ 3.09-3.18(4H,m), 3.42-3.63(2H,m), 3.55(3H,s), 3.69-3.78(4H,m), 5.80(1H,q,J=8Hz), 6.88(1H,s), 6.99(2H,d,J=9Hz), 7.18(1H,dd,J=8Hz,J=5Hz), 7.25(2H,d,J=9Hz), 7.31(1H,d,J=8Hz), 7.47(1H,dd,J=8Hz,J=2Hz), 7.64(1H,t,J=8Hz), 8.04(1H,d,J=8Hz), 8.05(1H,s), 8.18(1H,s), 8.48(1H,d,J=5Hz), 9.45(1H,d,J=8Hz)

### Example 8

### (1S)-N-[1-[5-(4-Biphenylyl)-1-methylimidazol-2-yl]-2-(2-pyridyl)-ethyl] (5-bromobenzo[b]furan-2-yl)formamide

The title compound was obtained from the compound of Preparation 10 in substantially the same manner as in Example 1.
mp 180.5-181.5°C
MS(m/z): 577,579 (M⁺),146(bp)
¹H-NMR (CDCl₃) δ 3.58(3H,s), 3.62(2H,d,J=7Hz), 5.98(1H,q,J=7Hz), 7.09(1H,s), 7.15(2H,d,J=7.5Hz), 7.34-7.38(4H,m), 7.40-7.50(3H,m), 7.55(1H,d,J=7.5Hz), 7.57-7.66(4H,m), 7.75(1H,d,J=7.5Hz), 7.80(1H,s), 8.56(1H,d,J=7Hz)

### Example 9

### (1S)-N-[1-[5-(4-Biphenylyl)-1-methylimidazol-2-yl]-2-(2-pyridyl)-ethyl](5-methoxybenzo[b]furan-2-yl)formamide

The title compound was obtained from the compound of Preparation 10 in substantially the same manner as in Example 1.
mp 150-152°C
MS(m/z): 529(M⁺,bp)
¹H-NMR (CDCl₃) δ 3.58(3H,s), 3.64(1H,d,J=7Hz), 3.86(3H,s), 5.98(1H,q,J=7Hz), 7.00-7.16(5H,m), 7.36-7.40(5H,m), 7.43-7.49(2H,m), 7.53-7.59(1H,m), 7.63(6H,t,J=7Hz), 8.57(1H,d,J=7Hz)

### Example 10

### (1S)-N-[1-[5-(4-Biphenylyl)-1-methylimidazol-2-yl]-2-(2-pyridyl)-ethyl](5-chlorobenzo[b]furan-2-yl)formamide

The title compound was obtained from the compound of Preparation 10 in substantially the same manner as in Example 1.
mp 171-174°C
MS(m/z): 533(M⁺,bp)
¹H-NMR (CDCl₃) δ 3.59(3H,s), 3.64(2H,d,J=7Hz), 5.98(1H,q,J=7Hz), 7.09(1H,s), 7.15(2H,d,J=7Hz), 7.36-7.40(5H,m), 7.43-7.50(3H,m), 7.55-7.67(6H,m), 7.74(1H,d,J=7Hz), 8.57(1H,d,J=7Hz)

### Example 11

### (1S)-(6-Chlorobenzo[b]furan-2-yl)-N-[1-[5-[4-(1-imidazolyl)-phenyl]-1-methylimidazol-2-yl]-2-(2-pyridyl)ethyl]formamide

The title compound was obtained from the compound of Preparation 16 in substantially the same manner as in Example 1.
mp 165-169°C
MS(m/z): 523(M⁺+H,bp)
¹H-NMR (CDCl₃) δ 3.60(3H,s), 3.64(2H,d,J=7Hz), 5.98(1H,q,J=7Hz), 7.09(1H,s), 7.13-7.18(2H,m), 7.24(1H,s), 7.30-7.32(2H,m), 7.40-7.48(5H,m), 7.53-7.60(3H,m), 7.75(1H,d,J=7Hz), 7.90(1H,s), 8.57(1H,d,J=7Hz)

### Example 12

### (1S)-(5-Chlorobenzo[b]furan-2-yl)-N-[1-[1-methyl-5-(4-nitrophenyl)imidazol-2-yl]-2-(2-pyridyl)ethyl]formamide

The title compound was obtained from the compound of Preparation 19 in substantially the same manner as in Example 1.
mp 199-201°C
MS: 500(ES-)
¹H-NMR (DMSO-d₆) δ 3.50(1H,dd,J=15Hz,J=7.5Hz), 3.60(1H,dd,J=15Hz,J=7.5Hz), 3.70(3H,s), 5.88(1H,dd,J=15Hz,J=7.5Hz), 7.16-7.34(2H,m), 7.46-7.73(4H,m), 7.75(2H,d,J=9Hz), 7.89(1H,m), 8.28(2H,d,J=9Hz), 8.50(1H,d,J=6Hz), 9.41(1H,d,J=9Hz)

### Example 13

### (1S)-(5-Chlorobenzo[b]furan-2-yl)-N-[1-[1-methyl-5-[4-(1-pyrrolyl)phenyl]imidazol-2-yl]-2-(2-pyridyl)ethyl]formamide

A solution of (1S)-N-[1-[5-(4-aminophenyl)-1-methylimidazol-2-yl]-2-(2-pyridyl)ethyl](5-chlorobenzo[b]furan-2-yl)formamide (220mg) and 2,5-dimethoxytetrahydrofuran (67.8mg) in glacial acetic acid (1ml) was heated at 95°C for 2 hours. The acetic acid was removed by vacuum distillation, and the residue was diluted with ethyl acetate and washed with saturated aqueous sodium hydrogencarbonate solution and brine. The organic layer was dried over sodium sulfate, concentrated, and purified by silica gel column chromatography (2% methanol in chloroform) to give (1S)-(5-chlorobenzo[b]furan-2-yl)-N-[1-[1-methyl-5-[4-(1-pyrrolyl)phenyl]imidazol-2-yl]-2-(2-pyridyl)-ethyl]formamide (111mg) as a brown amorphous solid.
MS(ES+): 522(M+H)
¹H-NMR (300MHz,CDCl₃) δ 3.54-3.68(5H,m), 5.99(1H,m), 6.36(2H,m), 7.04(1H,s), 7.08-7.18(4H,m), 7.30-7.40(4H,m), 7.40-7.48(3H,m), 7.56(1H,m), 7.61(1H,s), 7.91(1H,d,J=8Hz), 8.56(1H,m)

### Example 14

### (1S)-(Indol-2-yl)-N-[1-[1-methyl-5-[4-(2-methylimidazol-1-yl)-phenyl]imidazol-2-yl]-2-(2-pyridyl)ethyl]formamide

The title compound was obtained from the compound of Preparation 26 in substantially the same manner as in Example 1.
mp 207-211°C
MS(m/z): 500(M⁺-H,bp)
¹H-NMR (DMSO-d₆) δ 2.32(3H,s), 3.46-3.64(2H,m), 3.70(3H,s), 5.92(1H,q,J=7Hz), 6.92(1H,s), 7.01(1H,t,J=7.5Hz), 7.10(1H,s), 7.14-7.21(2H,m), 7.27(1H,s), 7.33-7.40(3H,m), 7.50-7.69(7H,m), 8.49(1H,d,J=7Hz), 9.07(1H,d,J=7.5Hz)

### Example 15

### (1S)-(Benzo[b]furan-2-yl)-N-[1-[1-methyl-5-[4-(2-methylimidazol-1-yl)phenyl]imidazol-2-yl]-2-(2-pyridyl)ethyl]formamide

The title compound was obtained from the compound of Preparation 26 in substantially the same manner as in Example 1.
mp 167-171 °C
MS(m/z): 503(M⁺+H,bp)
¹H-NMR (DMSO-d₆) δ 2.41(3H,s), 3.60-3.65(5H,m), 5.99(1H,q,J=7Hz), 7.04(2H,d,J=7Hz), 7.10(1H,s), 7.12-7.19(2H,m), 7.29-7.45(7H,m), 7.51-7.60(2H,m), 7.66(1H,d,J=7.5Hz), 7.71(1H,d,J=7.5Hz), 8.56(1H,d,J=7Hz)

### Example 16

### (1S)-(5-Chlorobenzo[b]furan-2-yl)-N-[1-[5-[4-(2-furyl)phenyl]-1-methylimidazol-2-yl]-2-(2-pyridyl)ethyl]formamide

The title compound was obtained from the compound of Preparation 30 in substantially the same manner as in Example 1.
mp 144-146°C
MS(ES+): 523(M+H)
¹H-NMR (300MHz,CDCl₃) δ 3.56(3H,s), 3.61(2H,d,J=8Hz), 5.96(1H,dd,J=8Hz,J=8Hz), 6.49(1H,m), 6.69(1H,m), 7.06(1H,s), 7.09-7.17(2H,m), 7.27-7.40(4H,m), 7.41-7.51(2H,m), 7.55(1H,m), 7.62(1H,m), 7.66-7.76(3H,m), 8.55(1H,m)

### Example 17

### (1S)-(5-Chlorobenzo[b]furan-2-yl)-N-[1-[1-methyl-5-[4-(2-thienyl)-phenyl]imidazol-2-yl]-2-(2-pyridyl)ethyl]formamide

The title compound was obtained from the compound of Preparation 32 in substantially the same manner as in Example 1.
mp 110-114°C
MS(ES+): 539(M+)
¹H-NMR (300MHz,CDCl₃) δ 3.50-3.66(5H,m), 5.88-6.02(1H,m), 7.00-7.19(5H,m), 7.27-7.47(5H,m), 7.49-7.59(2H,m), 7.59-7.68(2H,m), 7.71-7.80(1H,n), 8.51-8.58(1H,m)

### Example 18

### (1S)-(5-Chlorobenzo[b]furan-2-yl)-N-[1-[1-methyl-5-[4-(2-thiazolyl)phenyl]imidazol-2-yl]-2-(2-pyridyl)ethyl]formamide

The title compound was obtained from the compound of Preparation 34 in substantially the same manner as in Example 1.
mp 144-146°C
MS(ES+): 540(M+)
¹H-NMR (300MHz,CDCl₃) δ 3.54-3.69(5H,m), 5.97(1H,dd,J=15Hz,J=7Hz), 7.06-7.18(3H,m), 7.31-7.48(6H,m), 7.50-7.66(2H,m), 7.74(1H,d,J=8Hz), 7.89(1H,d,J=4Hz), 8.00(2H,d,J=8Hz), 8.55(1H,m)

### Example 19

### (1S)-(5-Chlorobenzo[b]furan-2-yl)-N-[1-[5-(3-fluoro-4-morpholinophenyl)-1-methylimidazol-2-yl]-2-(2-pyridyl)ethyl]formamide

The title compound was obtained from the compound of Preparation 41 in substantially the same manner as in Example 1.
mp 134-135°C
MS: 560(ES+)
¹H-NMR (DMSO-d₆) δ 3.00-3.06(4H,m), 3.37-3.55(2H,m), 3.58(3H,s), 3.70-3.77(4H,m), 5.78-5.86(1H,m), 6.96-7.88(10H,m), 8.48(1H,d,J=4.5Hz), 9.33(1H,d,J=9Hz)

### Example 20

### (1S)-(5-Chlorobenzo[b]furan-2-yl)-N-[1-[1-methyl-5-(2-thienyl)-imidazol-2-yl]-2-(2-pyridyl)ethyl]formamide

The title compound was obtained from the compound of Preparation 47 in substantially the same manner as in Example 1.
mp 70-74°C
MS(m/z): 463(M⁺+H,bp)
¹H-NMR (CDCl₃) δ 3.56(3H,s), 3.58-3.60(2H,m), 5.95(1H,q,J=7.5Hz), 7.00(1H,d,J=2Hz), 7.05-7.14(4H,m), 7.33-7.39(3H,m), 7.42-7.45(1H,m), 7.55(1H,ddd,J=7Hz,J=7Hz,J=2Hz), 7.62(1H,d,J=2Hz), 7.76(1H,d,J=8Hz), 8.54(1H,d,J=6Hz)

### Example 21

### (1S)-(5-Chlorobenzo[b]furan-2-yl)-N-[1-[5-(2,4-dichlorophenyl)-1-methylimidazol-2-yl]-2-(2-pyridyl)ethyl]formamide

The title compound was obtained from the compound of Preparation 53 in substantially the same manner as in Example 1.
mp 181-183°C
MS(m/z): 525,527,529(M⁺),100(bp)
¹H-NMR (CDCl₃) δ 3.32(3H,s), 3.59(2H,ABX,J=15Hz,J=15Hz,J=7Hz), 5.91(1H,q,J=7Hz), 6.98(1H,s), 7.08-7.13(2H,m), 7.18(1H,d,J=7.5Hz), 7.27-7.30(1H,m), 7.35-7.39(2H,m), 7.44-7.47(2H,m), 7.53(1H,ddd,J=7.5Hz,J=7.5Hz,J=2Hz), 7.63(1H,d,J=2Hz), 7.70(1H,d,J=7.5Hz), 8.52(1H,d,J=5Hz)

### Example 22

### (1S)-(Indol-2-yl)-N-[1-[5-[4-(1-methylimidazol-2-yl)phenyl]-1-methylimidazol-2-yl]-2-(2-pyridyl)ethyl]formamide

The title compound was obtained from the compound of Preparation 63 in substantially the same manner as in Example 1.
mp 218-222°C
ESI-MS(M+1): 502
¹H-NMR (DMSO-d₆) δ 3.45-3.65(2H,m), 3.70(3H,s), 3.77(3H,s), 5.91(1H,q,J=6Hz), 7.00(1H,s), 7.03(1H,d,J=8Hz), 7.10(1H,s), 7.13-7.20(2H,m), 7.24-7.29(2H,m), 7.30-7.40(2H,m), 7.53(2H,d,J=8Hz), 7.57-7.67(2H,m), 7.78(2H,d,J=8Hz), 8.50(1H,d,J=4Hz), 9.07(1H,d,J=8Hz)

### Example 23

### (1S)-N-[1-[5-[4-(1-Imidazolyl)phenyl]-1,4-dimethylimidazol-2-yl]-2-(2-pyridyl)ethyl](indol-2-yl)formamide

The title compound was obtained from the compound of Preparation 70 in substantially the same manner as in Example 1.
mp 156-157°C
ESI-MS(M+1): 502
¹H-NMR (CDCl₃) δ 2.20(3H,s), 3.48(3H,s), 3.64(2H,t,J=6Hz), 6.00(1H,q,J=6Hz), 7.03(1H,s), 7.08-7.18(3H,m), 7.23(1H,s), 7.28-7.42(5H,m), 7.48(2H,d,J=8Hz), 7.52-7.60(2H,m), 7.66(1H,d,J=8Hz), 7.81(1H,d,J=8Hz), 7.92(1H,s), 8.55(1H,d,J=4Hz), 9.52(1H,s)

### Example 24

### (1S)-(5-Formylbenzo[b]furan-2-yl)-N-[1-[5-[4-(1-imidazolyl)-phenyl]-1-methylimidazol-2-yl]-2-(2-pyridyl)ethyl]formamide

The title compound was obtained from the compound of Preparation 16 in substantially the same manner as in Example 1.
mp 194-197°C
ESI-MS(M+1): 517
¹H-NMR (CDCl₃) δ 3.53-3.70(5H,m), 5.98(1H,q,J=6Hz), 7.08(1H,s), 7.12-7.20(2H,m), 7.25(1H,s), 7.31(1H,s), 7.36-7.50(4H,m), 7.52-7.63(2H,m), 7.67(1H,d,J=8Hz), 7.83(1H,d,J=8Hz), 7.90(1H,s), 8.00(1H,dd,J=8Hz,J=2Hz), 8.22(1H,s), 8.56(1H,d,J=4Hz)

### Example 25

### (1S)-(5-Cyanobenzo[b]furan-2-yl)-N-[1-[5-[4-(1-imidazolyl)-phenyl]-1-methylimidazol-2-yl]-2-(2-pyridyl)ethyl]formamide

The title compound was obtained from the compound of Preparation 16 in substantially the same manner as in Example 1.
mp 174-176°C
ESI-MS(M+1): 514
¹H-NMR (CDCl₃) δ 3.55-3.70(5H,m), 5.98(1H,q,J=6Hz), 7.09(1H,s), 7.11-7.20(2H,m), 7.24(1H,s), 7.33(1H,s), 7.37-7.50(4H,m), 7.58(1H,dd,J=8Hz,J=2Hz), 7.63(1H,d,J=8Hz), 7.72(1H,dd,J=8Hz,J=2Hz), 7.85-7.95(2H,m), 8.04(1H,s), 8.57(1H,d,J=4Hz)

### Example 26

### (1S)-N-[1-[5-[4-(1-Imidazolyl)phenyl]-1-methylimidazol-2-yl]-2-(2-pyridyl)ethyl)[5-(trifluoromethyl)benzo[b]furan-2-yl]formamide

The title compound was obtained from the compound of Preparation 16 in substantially the same manner as in Example 1.
mp 150-151 °C
MS(m/z): 557(M⁺+H,bp)
¹H-NMR (CDCl₃) δ 3.60(3H,s), 3.62-3.65(3H,m), 5.99(1H,q,J=7Hz), 7.10(1H,s), 7.16(2H,d,J=7Hz), 7.24(1H,d,J=7Hz), 7.31(1H,s), 7.40-7.50(5H,m), 7.55-7.65(3H,m), 7.88(1H,s), 7.98(1H,s), 8.56(1H,d,J=7Hz)

### Example 27

### (1S)-(Furo[2,3-b]pyridin-2-yl)-N-[1-[5-[4-(1-imidazolyl)phenyl]-1-methylimidazol-2-yl]-2-(2-pyridyl)ethyl]formamide

The title compound was obtained from the compound of Preparation 16 in substantially the same manner as in Example 1.
mp 108-110°C
MS(m/z): 490(M⁺+H,bp)
¹H-NMR (CDCl₃) δ 3.60(3H,s), 3.61-6.35(2H,m), 6.00(1H,q,J=7Hz), 7.09(1H,s), 7.10-7.19(2H,m), 7.23(1H,s), 7.28-7.33(2H,m), 7.40-7.47(5H,m), 7.57(1H,ddd,J=7Hz,J=7Hz,J=2Hz), 7.80(1H,d,J=7Hz), 7.90(1H,s), 8.03(1H,dd,J=7Hz,J=2Hz), 8.45(1H,dd,J=7Hz,J=2Hz), 8.56(1H,d,J=7Hz)

### Example 28

### (1S)-N-[1-[5-[4-(1-Imidazolyl)phenyl]-1-methylimidazol-2-yl]-2-(2-pyridyl)ethyl](5-nitrofuro[2,3-b]pyridin-2-yl)formamide

The title compound was obtained from the compound of Preparation 16 in substantially the same manner as in Example 1.
mp 114-118°C
MS(m/z): 535(M⁺+H,bp)
¹H-NMR (CDCl₃) δ 3.60(3H,s), 3.61-3.64(2H,m), 5.98(1H,q,J=7Hz), 7.06(1H,s), 7.10-7.19(2H,m), 7.24(1H,s), 7.30(1H,s), 7.40-7.47(4H,m), 7.55-7.61(2H,m), 7.90(1H,s), 8.04(1H,d,J=7Hz), 8.56(1H,d,J=3Hz), 8.88(1H,d,J=3Hz), 9.35(1H,d,J=3Hz)

### Example 29

### (1S)-N-[1-[1-Ethyl-5-[4-(1-imidazolyl)phenyl]imidazol-2-yl]-2-(2-pyridyl)ethyl][5-(trifluoromethyl)benzo[b]furan-2-yl]formamide

The title compound was obtained from the compound of Preparation 72 in substantially the same manner as in Example 1.
mp 85-89°C
MS(m/z): 569(M⁺-H,bp)
¹H-NMR (CDCl₃) δ 1.18(3H,t,J=7Hz), 3.55-3.68(2H,m), 3.92-4.04(1H,m), 4.09-4.20(1H,m), 5.99(1H,q,J=7Hz), 7.08(1H,s), 7.12(1H,s), 7.22-7.28(3H,m), 7.30(1H,s), 7.40-7.48(4H,m), 7.53-7.72(4H,m), 7.90(1H,s), 7.97(1H,s), 8.56(1H,d,J=3Hz)

### Example 30

### (1S)-N-[1-[5-[4-(1-Imidazolyl)phenyl]-1-propylimidazol-2-yl]-2-(2-pyridyl)ethyl][5-(trifluoromethyl)benzo[b]furan-2-yl]formamide

The title compound was obtained from the compound of Preparation 74 in substantially the same manner as in Example 1.
mp 92-98°C
MS(m/z): 585(M⁺+H,bp)
¹H-NMR (CDCl₃) δ 0.76(3H,t,J=7Hz), 1.40-1.60(2H,m), 3.63(2H,d,J=7Hz), 3.82-3.92(1H,m), 4.00-4.11(1H,m), 5.97(1H,q,J=7Hz), 7.08(1H,s), 7.13-7.17(2H,m), 7.24(1H,s), 7.33(1H,s), 7.40-7.49(4H,m), 7.50(1H,s), 7.54-7.76(4H,m), 7.90(1H,s), 7.98(1H,s), 8.55-8.59(1H,m)

### Example 31

### (1S)-N-[1-[5-[4-(1-Benzimidazolyl)phenyl]-1-methylimidazol-2-yl]-2-(2-pyridyl)ethyl](indol-2-yl)formamide

The title compound was obtained from the compound of Preparation 79 in substantially the same manner as in Example 1.
mp 233-237°C
MS(m/z): 538(M⁺+H,bp)
¹H-NMR (DMSO-d₆) δ 3.58-3.71(2H,m), 3.65(3H,s), 6.04(1H,q,J=7.5Hz), 7.00(1H,s), 7.12(1H,s), 7.14-7.19(3H,m), 7.29(1H,m), 7.35-7.43(3H,m), 7.50-7.60(6H,m), 7.68(1H,d,J=7.5Hz), 7.81(1H,d,J=7.5Hz), 7.88-7.91(1H,m), 8.15(1H,s), 8.58(1H,d,J=7Hz), 9.45(1H,s)

### Example 32

### (1S)-N-[1-[5-[4-(1-Benzimidazolyl)phenyl]-1-methylimidazol-2-yl]-2-(2-pyridyl)ethyl](benzo[b]furan-2-yl)formamide

The title compound was obtained from the compound of Preparation 79 in substantially the same manner as in Example 1.
mp 152-155°C
MS(m/z): 539(M⁺+H),100(bp)
¹H-NMR (DMSO-d₆) δ 3.64(3H,s), 3.67(2H,d,J=7Hz), 6.03(1H,q,J=7Hz), 7.12(1H,s), 7.12-7.17(1H,m), 7.19(1H,d,J=7.5Hz), 7.31(1H,d,J=7.5Hz), 7.35-7.45(4H,m), 7.50-7.60(7H,m), 7.68(1H,d,J=7.5Hz), 7.74(1H,d,J=7.5Hz), 7.88-7.91(1H,m), 8.15(1H,s), 8.57(1H,d,J=7Hz)

### Example 33

### (1S)-N-[1-[5-[4-(2-Ethylimidazol-1-yl)phenyl]-1-methylimidazol-2-yl]-2-(2-pyridyl)ethyl](indol-2-yl)formamide

The title compound was obtained from the compound of Preparation 84 in substantially the same manner as in Example 1.
MS(ES+): 516(M+H)
¹H-NMR (300MHz,CDCl₃) δ 1.28(3H,t,J=7Hz), 2.69(2H,q,J=7Hz), 3.60(3H,s), 3.62-3.67(2H,m), 6.09(1H,m), 6.99(1H,s), 7.02-7.18(5H,m), 7.19-7.46(6H,m), 7.50(1H,m), 7.64(1H,d,J=8Hz), 8.14(1H,d,J=8Hz), 8.52(1H,m)

### Example 34

### (1S)-(5-Chlorobenzo[b]furan-2-yl)-N-[1-[1-methyl-5-[4-(3-pyridyl)-phenyl]imidazol-2-yl]-2-(2-pyridyl)ethyl]formamide

The title compound was obtained from the compound of Preparation 91 in substantially the same manner as in Example 1.
mp 158-160°C
MS(ES-): 532(M-H)
¹H-NMR (300MHz,CDCl₃) δ 3.53-3.70(5H,m), 5.97(1H,m), 7.04-7.19(3H,m), 7.31-7.50(6H,m), 7.52-7.69(4H,m), 7.75(1H,d,J=8Hz), 7.89(1H,m), 8.54(1H,m), 8.61(1H,m), 8.88(1H,m)

### Example 35

### (1S)-(5-Bromobenzo[b]furan-2-yl)-N-[1-[1-methyl-5-[4-(3-pyridyl)-phenyl]imidazol-2-yl]-2-(2-pyridyl)ethyl]formamide

The title compound was obtained from the compound of Preparation 91 in substantially the same manner as in Example 1.
mp 168-171°C
MS(ES+): 578(M+H)
¹H-NMR (300MHz, CDCl₃) δ 3.56-3.66(5H,m), 5.98(1H,m), 7.09(1H,s), 7.10-7.17(2H,m), 7.34-7.46(5H,m), 7.47-7.60(2H,m), 7.64(2H,d,J=8Hz), 7.75(1H,d,J=8Hz), 7.79(1H,s), 7.89(1H,m), 8.56(1H,m), 8.62(1H,m), 8.87(1H,m)

### Example 36

### (1S)-(5-Chlorobenzo[b]furan-2-yl)-N-[1-[1-methyl-5-[4-(2-pyridyl)-phenyl]imidazol-2-yl]-2-(2-pyridyl)ethyl]formamide

The title compound was obtained from the compound of Preparation 93 in substantially the same manner as in Example 1.
mp 175-180°C
MS(ES+): 534(M+H)
¹H-NMR (300MHz,CDCl₃) δ 3.51-3.70(5H,m), 5.91-6.04(1H,m), 7.06-7.20(3H,m), 7.32-7.50(5H,m), 7.52-7.84(5H,m), 8.04(2H,d,J=8Hz), 8.57(1H,m), 8.70(1H,m)

### Example 37

### (1S)-(5-Chlorobenzo[b]furan-2-yl)-N-[1-[1-methyl-5-[4-(4-pyridyl)-phenyl]imidazol-2-yl]-2-(2-pyridyl)ethyl]formamide

The title compound was obtained from the compound of Preparation 95 in substantially the same manner as in Example 1.
mp 135-140°C
MS(ES+): 534(M+H)
¹H-NMR (300MHz, CDCl₃) δ 3.57-3.66(5H,m), 5.98(1H,m), 7.06-7.18(3H,m), 7.33-7.47(5H,m), 7.49-7.73(5H,m), 7.78(1H,d,J=8Hz), 8.56(1H,m), 8.68(2H,d,J=7Hz)

### Example 38

### (1S)-(5-Chlorobenzo[b]furan-2-yl)-N-[1-[1-methyl-5-[4-(5-thiazolyl)phenyl]imidazol-2-yl]-2-(2-pyridyl)ethyl]formamide

The title compound was obtained from the compound of Preparation 97 in substantially the same manner as in Example 1.
mp 165-167°C
MS(ES+): 540(M+H)
¹H-NMR (300MHz,CDCl₃) δ 3.57(3H,s), 3.62(2H,m), 5.97(1H,m), 7.08(1H,s), 7.10-7.17(2H,m), 7.32-7.40(4H,m), 7.45(1H,d,J=9Hz), 7.56(1H,m), 7.60-7.66(3H,m), 7.74(1H,d,J=9Hz), 8.11(1H,s), 8.56(1H,m), 8.79(1H,s)

### Example 39

### (1S)-N-[1-[1-Methyl-5-(4-nitrophenyl)imidazol-2-yl]-2-(2-pyridyl)-ethyl][5-(trifluoromethyl)benzo[b]furan-2-yl]formamide

The title compound was obtained from the compound of Preparation 19 in substantially the same manner as in Example 1.
mp 192-194°C
MS: 536(ES+)
¹H-NMR (DMSO-d₆) δ 3.51(1H,dd,J=15Hz,J=7.5Hz), 3.61(1H,dd,J=15Hz,J=7.5Hz), 3.72(3H,s), 5.90(1H,dd,J=15Hz,J=7.5Hz), 7.16-7.35(2H,m), 7.53-7.90(6H,m), 8.25(1H,s), 8.28(2H,d,J=9Hz), 8.50(1H,d,J=6Hz), 9.50(1H,d,J=9Hz)

### Example 40

### (1S)-N-[1-[1-Ethyl-5-[4-(1-imidazolyl)phenyl]imidazol-2-yl]-2-(2-pyridyl)ethyl](5-formylbenzo[b]furan-2-yl)formamide

The title compound was obtained from the compound of Preparation 72 in substantially the same manner as in Example 1.
ESI-MS: 531(M+H)
¹H-NMR (300MHz,DMSO-d₆) δ 1.08(3H,t,J=8Hz), 3.52(1H,dd,J=15Hz,J=8Hz), 3.58(1H,dd,J=15Hz,J=7Hz), 4.06(1H,quintet,J=8Hz), 4.10(1H,quintet,J=8Hz), 5.90(1H,q,J=8Hz), 7.04(1H,s), 7.13(1H,s), 7.18(1H,dd,J=8Hz,J=5Hz), 7.33(1H,d,J=8Hz), 7.55(2H,d,J=9Hz), 7.66(1H,t,J=8Hz), 7.73(2H,d,J=9Hz), 7.78-7.88(3H,m), 7.98(1H,d,J=8Hz), 8.32(1H,s), 8.39(1H,s), 8.50(1H,d,J=5Hz), 9.48(1H,d,J=8Hz)

### Example 41

### (1S)-(5-Cyanobenzo[b]furan-2-yl)-N-[1-[1-ethyl-5-[4-(1-imidazolyl)phenyl]imidazol-2-yl]-2-(2-pyridyl)ethyl]formamide

The title compound was obtained from the compound of Preparation 72 in substantially the same manner as in Example 1.
ESI-MS: 526(M-H)
¹H-NMR (300MHz,DMSO-d₆) δ 1.07(3H,t,J=8Hz), 3.50(1H,dd,J=15Hz,J=8Hz), 3.58(1H,dd,J=15Hz,J=7Hz), 3.98-4.28(2H,m), 5.88(1H,q,J=8Hz), 7.04(1H,s), 7.13(1H,s), 7.18(1H,dd,J=8Hz,J=5Hz), 7.32(1H,d,J=8Hz), 7.56(2H,d,J=9Hz), 7.66(1H,t,J=8Hz), 7.70-7.78(3H,m), 7.80(1H,s), 7.89(2H,s), 8.32(1H,s), 8.37(1H,s), 8.50(1H,d,J=5Hz), 9.53(1H,d,J=8Hz)

### Example 42

### (1S)-(5-Formylbenzo[b]furan-2-yl)-N-[1-[5-[4-(1-imidazolyl)-phenyl]-1-propylimidazol-2-yl]-2-(2-pyridyl)ethyl]formamide

The title compound was obtained from the compound of Preparation 74 in substantially the same manner as in Example 1.
ESI-MS: 545(M+H)
¹H-NMR (300MHz,DMSO-d₆) δ 0.64(3H,t,J=8Hz), 1.35-1.52(2H,m), 3.53(1H,dd,J=15Hz,J=8Hz), 3.61(1H,dd,J=15Hz,J=7Hz), 3.99(1H,quintet,J=8Hz), 4.14(1H,quintet,J=8Hz), 5.92(1H,q,J=8Hz), 7.04(1H,s), 7.13(1H,s), 7.18(1H,dd,J=8Hz,J=5Hz), 7.32(1H,d,J=8Hz), 7.57(2H,d,J=9Hz), 7.66(1H,t,J=8Hz), 7.73(2H,d,J=9Hz), 7.78-7.90(3H,m), 8.00(1H,d,J=8Hz), 8.33(1H,s), 8.40(1H,s), 8.50(1H,d,J=5Hz), 9.48(1H,d,J=8Hz)

### Example 43

### (1S)-(5-Cyanobenzo[b]furan-2-yl)-N-[1-[5-[4-(1-imidazolyl)phenyl]-1-propylimidazol-2-yl]-2-(2-pyridyl)ethyl]formamide

The title compound was obtained from the compound of Preparation 74 in substantially the same manner as in Example 1.
ESI-MS: 540(M-H)
¹H-NMR (300MHz,DMSO-d₆) δ 0.63(3H,t,J=8Hz), 1.34-1.52(2H,m), 3.52(1H,dd,J=15Hz,J=8Hz), 3.60(1H,dd,J=15Hz,J=7Hz), 3.99(1H,quintet,J=8Hz), 4.13(1H,quintet,J=8Hz), 5.90(1H,q,J=8Hz), 7.04(1H,s), 7.13(1H,s), 7.18(1H,dd,J=8Hz,J=5Hz), 7.32(1H,d,J=8Hz), 7.53(2H,d,J=9Hz), 7.66(1H,t,J=8Hz), 7.70-7.78(3H,m), 7.82(1H,s), 7.88(2H,s), 8.32(1H,s), 8.37(1H,s), 8.48(1H,d,J=5Hz), 9.52(1H,d,J=8Hz)

### Example 44

### (1S)-(5-Bromobenzo[b]furan-2-yl)-N-[1-[5-[4-(1-imidazolyl)phenyl]-1-propylimidazol-2-yl]-2-(2-pyridyl)ethyl]formamide

The title compound was obtained from the compound of Preparation 74 in substantially the same manner as in Example 1.
mp 93-99°C
MS(m/z): 595,597(M⁺,bp)
¹H-NMR (CDCl₃) δ 0.71(3H,t,J=7Hz), 1.40-1.60(2H,m), 3.60(2H,d,J=7Hz), 3.80-3.90(1H,m), 3.98-4.08(1H,m), 5.95(1H,q,J=7Hz), 7.08(1H,s), 7.10-7.15(2H,m), 7.24(1H,s), 7.31(1H,s), 7.35-7.45(6H,m), 7.50-7.60(2H,m), 7.64(1H,d,J=7.5Hz), 7.80(1H,d,J=3Hz), 7.90(1H,s), 8.56(1H,d,J=3Hz)

### Example 45

### (1S)-(Benzo[b]thiophen-2-yl)-N-[1-[5-[4-(1-imidazolyl)phenyl]-1-methylimidazol-2-yl]-2-(2-pyridyl)ethyl]formamide

The title compound was obtained from the compound of Preparation 16 in substantially the same manner as in Example 1.
mp 190-193°C
ESI-MS: 505(M+1)
¹H-NMR (CDCl₃) δ 3.50-3.70(5H,m), 5.91(1H,q,J=6Hz), 7.06(1H,s) 7.10-7.22(2H,m), 7.24(1H,s), 7.32(1H,s), 7.35-7.50(5H,m), 7.59(1H,dt,J=8Hz,2Hz), 7.75(1H,d,J=8Hz), 7.78-7.87(2H,m), 7.90(1H,s), 8.55(1H,d,J=4Hz)

### Example 46

### (1S)-(5-Bromobenzo[b]furan-2-yl)-N-[1-[5-[4-(1-imidazolyl)-phenyl]-1-methylimidazol-2-yl]-2-(2-pyridyl)ethyl]formamide

The title compound was obtained from the compound of Preparation 16 in substantially the same manner as in Example 1.
mp 197-199°C
ESI-MS: 567(M+1)
¹H-NMR (CDCl₃) δ 3.58,3.62(2H,d,J=6Hz), 5.98(1H,q,J-6Hz), 7.08(1H,s), 7.12-7.18(2H,m), 7.24(1H,s), 7.32(1H,s), 7.37(1H,s), 7.38-7.49(5H,m), 7.51-7.63(2H,m), 7.75(2H,d,J=8Hz), 7.80(1H,s), 7.90(1H,s), 8.56(1H,d,J=4Hz)

### Example 47

### (1S)-N-[1-[5-[4-(1-Imidazolyl)phenyl]-1-methylimidazol-2-yl]-2-(2-pyridyl)ethyl](5-nitrobenzo[b]thiophen-2-yl)formamide

The title compound was obtained from the compound of Preparation 16 in substantially the same manner as in Example 1.
mp 228-231°C
ESI-MS: 550(M+1)
¹H-NMR (CDCl₃) δ 3.55-3.80(5H,m), 5.90(1H,q,J=8Hz), 7.05(1H,s), 7.13-7.22(2H,m), 7.24(1H,s), 7.31(1H,s), 7.40-7.54(5H,m), 7.62(1H,dt,J=8Hz,2Hz), 7.85(1H,s), 7.87-8.00(2H,m), 8.25(1H,dd,J=8Hz,2Hz), 8.50-8.62(2H,m), 8.65(1H,d,J=2Hz)

### Example 48

### (1S)-(5-Chlorobenzo[b]furan-2-yl)-N-[1-[5-[4-(1-imidazolyl)-phenyl]-1-propylimidazol-2-yl]-2-(2-pyridyl)ethyl]formamide

The title compound was obtained from the compound of Preparation 74 in substantially the same manner as in Example 1.
mp 173-175°C
ESI-MS: 551(M+1)
¹H-NMR (CDCl₃) δ 0.73(3H,t,J=6Hz), 1.38-1.60(2H,m), 3.62(2H,d,J=6Hz), 3.77-4.17(2H,m), 5.95(1H,q,J=6Hz), 7.04(1H,s), 7.08-7.17(2H,m), 7.23(1H,s), 7.32(1H,s), 7.35-7.50(8H,m), 7.55(1H,dt,J=8Hz,2Hz), 7.60-7.67(2H,m), 7.95(1H,s), 8.56(1H,d,J=4Hz)

### Example 49

### (1S)-(5-Chlorobenzo[b]furan-2-yl)-N-[1-[5-[4-(1-imidazolyl)-phenyl]-1-methylimidazol-2-yl]-2-(2-pyridyl)ethyl]formamide

The title compound was obtained from the compound of Preparation 16 in substantially the same manner as in Example 1.
mp 63-73°C
ESI-MS: 523(M+1)
¹H-NMR (CDCl₃) δ 3.61(3H,s), 3.62-3.70(2H,m), 6.00(1H,q,J=8Hz), 7.08(1H,s), 7.12-7.20(2H,m), 7.25(1H,s), 7.30(1H,s), 7.35-7.50(6H,m), 7.57(1H,t,J=8Hz), 7.64(1H,s), 7.83(1H,d,J=8Hz), 7.88(1H,s), 8.56(1H,d,J=4Hz)

### Example 50

### (1S)-N-[1-[5-[4-(1-Imidazolyl)phenyl]-1-methylimidazol-2-yl]-2-(2-pyridyl)ethyl](5-nitrobenzo[b]furan-2-yl)formamide

The title compound was obtained from the compound of Preparation 16 in substantially the same manner as in Example 1.
mp 221-223°C
ESI-MS: 534(M+1)
¹H-NMR (CDCl₃) δ 3.60(3H,s), 3.61-3.66(2H,m), 5.99(1H,q,J=8Hz), 7.08(1H,s), 7.10-7.20(2H,m), 7.24(1H,s), 7.32(1H,s), 7.35-7.50(4H,m), 7.57(1H,s), 7.58-7.68(2H,m), 7.85-7.94(2H,m), 8.36(1H,dd,J=8Hz,2Hz), 8.58(1H,d,J=6Hz), 8.62(1H,d,J=3Hz)

### Example 51

### (1S)-N-[1-[5-[4-(1-Imidazolyl)phenyl]-1-methylimidazol-2-yl]-2-(2-pyridyl)ethyl](5-methoxybenzo[b]furan-2-yl)formamide

The title compound was obtained from the compound of Preparation 16 in substantially the same manner as in Example 1.
mp 151-153°C
ESI-MS: 519(M+1)
¹H-NMR (CDCl₃) δ 3.55-3.65(2H,m), 3.60(3H,s), 3.85(3H,s), 5.99(1H,q,J=8Hz), 7.00-7.09(3H,m), 7.11-7.20(2H,m), 7.25(1H,s), 7.32(1H,s), 7.36-7.48(5H,m), 7.53-7.61(1H,m), 7.63(1H,d,J=8Hz), 7.90(1H,s), 8.57(1H,d,J=6Hz)

### Example 52

### (1S)-N-[1-[5-[4-(1-Imidazolyl)phenyl]-1-propylimidazol-2-yl]-2-(4-pyridyl)ethyl](indol-2-yl)formamide

The title compound was obtained from the compound of Preparation 100 in substantially the same manner as in Example 1.
mp 238-240°C(decomposition)
MS(m/z): 516 (M⁺+H,bp)
¹H-NMR (CDCl₃) δ 0.65(3H,t,J=7Hz), 1.26-1.43(2H,m), 3.42-3.50(2H,m), 3.54-3.67(1H,m), 3.78-3.90(1H,m), 5.70(1H,q,J=7Hz), 7.01(1H,d,J=2Hz), 7.09-7.11(3H,m), 7.16(1H,t,J=7Hz), 7.24-7.33(3H,m), 7.38-7.48(5H,m), 7.68(1H,d,J=8Hz), 7.91(1H,s), 8.03(1H,s), 8.47-8.50(2H,m), 9.43(1H,s)

### Example 53

### (1S)-N-[1-[1-Ethyl-5-[4-(1-imidazolyl)phenyl]imidazol-2-yl]-2-(4-pyridyl)ethyl](indol-2-yl)formamide

The title compound was obtained from the compound of Preparation 102 in substantially the same manner as in Example 1.
mp 255-260°C (decomposition)
MS(m/z): 502(M⁺+H,bp)
¹H-NMR (CDCl₃-CD₃OD) δ 1.02(3H,t,J=7Hz), 3.43(2H,q,J=7Hz), 3.78-3.86(1H,m), 4.03-4.13(1H,m), 5.68(1H,t,J=7Hz), 7.09(1H,s), 7.14(1H,t,J=7Hz), 7.17-7.21(3H,m), 7.29(1H,t,J=7Hz), 7.36(1H,s), 7.41-7.51(5H,m), 7.68(1H,d,J=7Hz), 7.94(1H,s), 8.43(2H,d,J=7Hz)

### Example 54

### (1S)-(5-Fluorobenzo[b]furan-2-yl)-N-[1-[5-[4-(1-imidazolyl)-phenyl]-1-methylimidazol-2-yl]-2-(2-pyridyl)ethyl]formamide

The title compound was obtained from the compound of Preparation 16 in substantially the same manner as in Example 1.
mp 180-182°C
MS(m/z): 507(M⁺+H,bp)
¹H-NMR (CDCl₃) δ 3.59(3H,s), 3.61-3.65(3H,m), 5.98(1H,q,J=7Hz), 7.07(1H,s), 7.10-7.19(2H,m), 7.24(1H,s), 7.28-7.31(2H,m), 7.40-7.50(6H,m), 7.56(1H,t,J=7.5Hz), 7.70(1H,d,J=7.5Hz), 7.90(1H,s), 8.55(1H,d,J=7Hz)

### Example 55

### (1S)-N-[1-[1-Ethyl-5-[4-(1-imidazolyl)phenyl]imidazol-2-yl]-2-(2-pyridyl)ethyl](5-fluorobenzo[b]furan-2-yl)formamide

The title compound was obtained from the compound of Preparation 72 in substantially the same manner as in Example 1.
mp 80-84°C
MS(m/z): 521(M⁺+H,bp)
¹H-NMR (CDCl₃) δ 1.16(3H,t,J=7Hz), 3.63(2H,heptet,J=7Hz), 3.91-4.05(1H,m), 4.08-4.20(1H,m), 5.99(1H,q,J=7Hz), 7.06(1H,s), 7.12-7.19(3H,m), 7.22-7.28(3H,m), 7.30(1H,s), 7.40(1H,s), 7.42-7.48(4H,m), 7.52-7.64(2H,m), 7.90(1H,s), 8.55(1H,d,J=7Hz)

### Example 56

### (1S)-(5-Fluorobenzo[b]furan-2-yl)-N-[1-[5-[4-(1-imidazolyl)-phenyl]-1-propylimidazol-2-yl]-2-(2-pyridyl)ethyl]formamide

The title compound was obtained from the compound of Preparation 74 in substantially the same manner as in Example 1.
mp 77-81 °C
MS(m/z): 535(M⁺+H,bp)
¹H-NMR (CDCl₃) δ 0.74(3H,t,J=7Hz), 1.40-1.60(2H,m), 3.62(2H,d,J=7Hz), 3.80-3.91(1H,m), 3.98-4.08(1H,m), 5.96(1H,q,J=7Hz), 7.06(1H,s), 7.12-7.19(3H,m), 7.24(1H,s), 7.29-7.33(2H,m), 7.35-7.45(6H,m), 7.54-7.59(1H,m), 7.64(1H,d,J=7.5Hz), 7.90(1H,s), 8.56(1H,d,J=3Hz)

### Example 57

### (1S)-N-[1-[5-[4-(1-Imidazolyl)phenyl]-1-propylimidazol-2-yl]-2-(2-pyridyl)ethyl](5-nitrobenzo[b]furan-2-yl)formamide

The title compound was obtained from the compound of Preparation 74 in substantially the same manner as in Example 1.
MS(ES+): 562(M+H)
¹H-NMR (300MHz,CDCl₃) δ 0.75(3H,t,J=7Hz), 1.36-1.66(2H,m), 3.61(2H,d,J=7Hz), 3.78-3.95(1H,m), 3.96-4.13(1H,m), 5.96(1H,m), 7.04(1H,s), 7.10-7.20(2H,m), 7.23(1H,s), 7.31(1H,s), 7.35-7.51(4H,m), 7.51-7.70(3H,m), 7.84(1H,d,J=8Hz), 7.90(1H,s), 8.35(1H,m), 8.52-8.69(2H,m)

### Example 58

### (1S)-N-[1-[5-[4-(1-Imidazolyl)phenyl]-1-propylimidazol-2-yl]-2-(2-pyridyl)ethyl](5-methoxybenzo[b]furan-2-yl)formamide

The title compound was obtained from the compound of Preparation 74 in substantially the same manner as in Example 1.
MS(ES+): 547(M+H)
¹H-NMR (300MHz,CDCl₃) δ 0.73(3H,t,J=7Hz), 1.38-1.60(2H,m), 3.54-3.71(2H,m), 3.77-3.95(4H,m), 3.97-4.12(1H,m), 5.96(1H,dd,J=8Hz,8Hz), 6.98-7.19(5H,m), 7.23(1H,s), 7.32(1H,s), 7.33-7.66(8H,m), 7.90(1H,s), 8.55(1H,m)

### Example 59

### (1S)-N-[1-[1-Ethyl-5-[4-(1-imidazolyl)phenyl]imidazol-2-yl]-2-(2-pyridyl)ethyl](5-nitrobenzo[b]furan-2-yl)formamide trihydrochloride

The title compound was obtained from the compound of Preparation 72 in substantially the same manner as in Example 1.
mp 190-200°C
MS: 548(ES+)
¹H-NMR (DMSO-d₆) δ 1.12(3H,m), 4.02(3H,s), 4.35(2H,m), 4.37(2H,m), 6.13(1H,m), 7.63-8.45(13H,m), 8.73(1H,s), 8.80(1H,s), 9.88(1H,s)

### Example 60

### (1S)-N-[1-[1-Ethyl-5-[4-(1-imidazolyl)phenyl]imidazol-2-yl]-2-(2-pyridyl)ethyl](5-methoxybenzo[b]furan-2-yl)formamide trihydrochloride

The title compound was obtained from the compound of Preparation 72 in substantially the same manner as in Example 1.
mp 190-195°C
MS: 533(ES+)
¹H-NMR (DMSO-d₆) δ 1.12(3H,m), 3.82(3H,s), 3.93(2H,m), 4.35(2H,m), 6.08(1H,m), 7.06(1H,m), 7.28(1H,s), 7.52-8.18(11H,m), 8.40(1H,s), 8.70(1H,br-s), 9.88(1H,s)

### Example 61

### (1S)-(5-Chlorobenzo[b]furan-2-yl)-N-[1-[1-ethyl-5-[4-(1-imidazolyl)phenyl]imidazol-2-yl]-2-(2-pyridyl)ethyl]formamide trihydrochloride

The title compound was obtained from the compound of Preparation 72 in substantially the same manner as in Example 1.
mp 198-203°C
MS: 537(ES+)
¹H-NMR (DMSO-d₆) δ 1.13(3H,m), 3.93(2H,m), 4.35(2H,m), 6.10(1H,br-s), 7.48-8.18(13H,m), 8.40(1H,s), 8.70(1H,s), 9.86(1H,s)

### Example 62

### (1S)-(5-Methoxybenzo[b]furan-2-yl)-N-[1-[1-methyl-5-(4-nitrophenyl)imidazol-2-yl]-2-(2-pyridyl)ethyl]formamide

The title compound was obtained from the compound of Preparation 19 in substantially the same manner as in Example 1.
mp 210-211°C
MS: 498(ES+)
¹H-NMR (DMSO-d₆) δ 3.50(1H,dd,J=15Hz,J=7.5Hz), 3.60(1H,dd,J=15Hz,J=7.5Hz), 3.70(3H,s), 3.79(3H,s), 5.88(1H,dd,J=15Hz,J=7.5Hz), 7.02-7.34(4H,m), 7.52-7.77(3H,m), 7.75(2H,d,J=9Hz), 8.27(2H,d,J=9Hz), 8.50(1H,d,J=6Hz) 9.27(1H,d,J=9Hz)

### Example 63

### (1S)-(5-Fluorobenzo[b]furan-2-yl)-N-[1-[1-methyl-5-(4-nitrophenyl)imidazol-2-yl]-2-(2-pyridyl)ethyl]formamide

The title compound was obtained from the compound of Preparation 19 in substantially the same manner as in Example 1.
mp 207-208°C
MS: 486(ES+)
¹H-NMR (DMSO-d₆) δ 3.50(1H,dd,J=15Hz,J=7.5Hz), 3.60(1H,dd,J=15Hz,J=7.5Hz), 3.71(3H,s), 5.88(1H,dd,J=15Hz,J=7.5Hz), 7.16-7.35(3H,m), 7.57-7.72(4H,m), 7.75(2H,d,J=9Hz), 8.28(2H,d,J=9Hz), 8.49(1H,d,J=6Hz), 9.38(1H,d,J=9Hz)

### Example 64

### (1S)-(Benzo[b]furan-2-yl)-N-[1-[1-methyl-5-(4-nitrophenyl)-imidazol-2-yl]-2-(2-pyridyl)ethyl]formamide

The title compound was obtained from the compound of Preparation 19 in substantially the same manner as in Example 1.
mp 103-104°C
MS: 466(ES-)
¹H-NMR (DMSO-d₆) δ 3.50(1H,dd,J=15Hz,J=7.5Hz), 3.60(1H,dd,J=15Hz,J=7.5Hz), 3.70(3H,s), 5.88(1H,dd,J=15Hz,J=7.5Hz), 7.16-7.50(4H,m), 7.60-7.69(3H,m), 7.75(2H,d,J=9Hz), 7.77(1H,m), 8.27(2H,d,J=9Hz), 8.50(1H,d,J=6Hz), 9.32(1H,d,J=9Hz)

### Example 65

### (1S)-(Benzothiazol-2-yl)-N-[1-[5-[4-(1-imidazolyl)phenyl]-1-methylimidazol-2-yl]-2-(2-pyridyl)ethyl]formamide

The title compound was obtained from the compound of Preparation 16 in substantially the same manner as in Example 1.
ESI-MS: 506(M+H)
¹H-NMR (300MHz,DMSO-d₆) δ 3.58(2H,d,J=8Hz), 3.64(3H,s), 5.84(1H,q,J=8Hz), 7.07(1H,s), 7.13(1H,s), 7.21(1H,dd,J=8Hz,J=5Hz), 7.30(1H,d,J=8Hz), 7.54-7.69(5H,m), 7.74(2H,d,J=8Hz), 7.81(1H,s), 8.17(1H,d,J=8Hz), 8.22(1H,d,J=8Hz), 8.32(1H,s), 8.52(1H,d,J=5Hz), 9.56(1H,d,J=8Hz)

### Example 66

### (1S)-(5-Ethylbenzo[b]furan-2-yl)-N-[1-[5-[4-(1-imidazolyl)-phenyl]-1-methylimidazol-2-yl]-2-(2-pyridyl)ethyl]formamide

The title compound was obtained from the compound of Preparation 16 in substantially the same manner as in Example 1.
ESI-MS: 517(M+H)
¹H-NMR (300MHz,DMSO-d₆) δ 1.22(3H,t,J=8Hz), 2.71(2H,q,J=8Hz), 3.46-3.58(2H,m), 3.64(3H,s), 5.87(1H,q,J=8Hz), 7.06(1H,s), 7.12(1H,s), 7.18(1H,dd,J=8Hz,J=5Hz), 7.28-7.36(2H,m), 7.51-7.60(5H,m), 7.65(1H,t,J=8Hz), 7.73(2H,d,J=9Hz), 7.81(1H,s), 8.32(1H,s), 8.49(1H,d,J=5Hz), 9.23(1H,d,J=8Hz)

### Example 67

### (1S)-(5-Bromobenzo[b]furan-2-yl)-N-[1-[1-ethyl-5-[4-(1-imidazolyl)phenyl]imidazol-2-yl]-2-(2-pyridyl)ethyl]formamide

The title compound was obtained from the compound of Preparation 72 in substantially the same manner as in Example 1.
ESI-MS: 581(M+)
¹H-NMR (300MHz,DMSO-d₆) δ 1.07(3H,t,J=8Hz), 3.49(1H,dd,J=15Hz,J=8Hz), 3.57(1H,dd,J=15Hz,7Hz), 3.98-4.26(2H,m), 5.88(1H,q,J=8Hz), 7.04(1H,s), 7.13(1H,s), 7.18(1H,dd,J=8Hz,J=5Hz), 7.32(1H,d,J=8Hz), 7.56(2H,d,J=9Hz), 7.58-7.70(4H,m), 7.74(2H,d,J=8Hz), 7.81(1H,s), 8.02(1H,s), 8.33(1H,s), 8.50(1H,d,J=5Hz), 9.43(1H,d,J=8Hz)

### Example 68

### (1S)-(7-Chlorobenzo[b]furan-2-yl)-N-[1-[5-[4-(1-imidazolyl)-phenyl]-1-methylimidazol-2-yl]-2-(2-pyridyl)ethyl]formamide

The title compound was obtained from the compound of Preparation 16 in substantially the same manner as in Example 1.
mp 182-184°C
ESI-MS: 523(M+)
¹H-NMR (300MHz,DMSO-d₆) δ 3.48-3.62(2H,m), 3.66(3H,s), 5.90(1H,q,J=8Hz), 7.08(1H,s), 7.13(1H,s), 7.19(1H,dd,J=8Hz,J=5Hz), 7.31-7.38(2H,m), 7.54-7.61(3H,m), 7.67(1H,t,J=8Hz), 7.72-7.78(4H,m), 7.81(1H,s), 8.32(1H,s), 8.51(1H,d,J=5Hz), 9.44(1H,d,J=8Hz)

### Example 69

### (1S)-(4-Chlorobenzo[b]furan-2-yl)-N-[1-[5-[4-(1-imidazolyl)-phenyl]-1-methylimidazol-2-yl]-2-(2-pyridyl)ethyl]formamide

The title compound was obtained from the compound of Preparation 16 in substantially the same manner as in Example 1.
MS: 523(M+)
¹H-NMR (300MHz,DMSO-d₆) δ 3.44-3.60(2H,m), 3.64(3H,s), 5.88(1H,q,J=8Hz), 7.08(1H,s), 7.13(1H,s), 7.18(1H,dd,J=8Hz,J=5Hz), 7.34(1H,d,J=8Hz), 7.44(1H,d,J=8Hz), 7.48(1H,t,J=8Hz), 7.57(2H,d,J=8Hz), 7.63-7.78(5H,m), 7.82(1H,s), 8.32(1H,s), 8.50(1H,d,J=5Hz), 9.40(1H,d,J=8Hz)

### Example 70

### (1S)-(5-Chlorobenzo[b]furan-2-yl)-N-[1-[1-methyl-5-[4-(2-methylimidazol-1-yl)phenyl]imidazol-2-yl]-2-(2-pyridyl)ethyl]formamide

The title compound was obtained from the compound of Preparation 26 in substantially the same manner as in Example 1.
mp 159-161°C
MS(m/z): 537(M⁺+1,bp)
¹H-NMR (CDCl₃) δ 2.40(3H,s), 3.60(3H,s), 3.63(2H,d,J=7Hz), 5.99(1H,q,J=7Hz), 7.03(2H,d,J=7Hz), 7.10(1H,s), 7.15(2H,d,J=7Hz), 7.33-7.48(7H,m), 7.58(1H,ddd,J=7Hz,J=7Hz,J=2Hz), 7.64(1H,J=2Hz), 7.74(1H,d,J=7Hz), 8.55(1H,d,J=5Hz)

### Example 71

### (1S)-N-[1-[5-(4-Bromophenyl)-1-methylimidazol-2-yl]-2-(2-pyridyl)-ethyl](5-chlorobenzo[b]furan-2-yl)formamide

The title compound was obtained from the compound of Preparation 103 in substantially the same manner as in Example 1.
mp 185-188°C
ESI-MS(M+1): 536
¹H-NMR (CDCl₃) δ 3.53(3H,s), 3.60(2H,d,J=6Hz), 5.95(1H,q,J=6Hz), 7.03(1H,s), 7.10-7.20(4H,m), 7.33-7.60(7H,m), 7.63(1H,d,J=2Hz), 7.77(1H,d,J=8Hz), 8.50-8.57(1H,m)

## Claims

1. A compound of the formula (I): 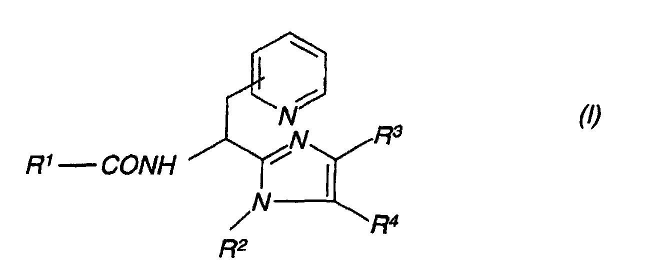 wherein R¹ is (a) indolyl, (b) benzothienyt having suitable substituent(s) selected from the group consisting of nitro and halogen, (c) benzothiazolyl having halogen, (d) furopyridyl which may have nitro or (e) benzofuranyl which may have suitable substituent(s) selected from the group consisting or halogen, (C₁-C₆) alkyl, (C₁-C₆) alkoxy, nitre, cyano, acyl and trihalo (C₁-C₆) alkyl,
R² is (C₁-C₆) alkyl,
R³ is hydrogen or (C₁-C₆) alkyl, and
R⁴ is thionyl or a group of the formula: 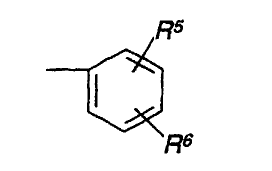 wherein R⁵ is hydrogen or halogen, and
R⁶ is (a) imidazolyl which may have (C₁-C₆) alkyl, (b) benzimidazolyl, (c) pyridyl, (d) pyrrolyl, (e) morpholinyl, (f) thionyl, (g) furyl, (h) phenyl, (j) thiazolyl, (k) halogen or (l) nitro, provided that (i) R³ is (C₁-C₆) alkyl or R⁶ is benzimidazolyl or imidazolyl having (C₁-C₆) alkyl when R¹ is indolyl, and (ii) R⁶ is benzimidazolyl or imidozolyl having (C₁-C₆) alkyl when R¹ is benzofuranyl,
and a pharmaceutically acceptable salt thereof.

2. A compound of the formula (II): 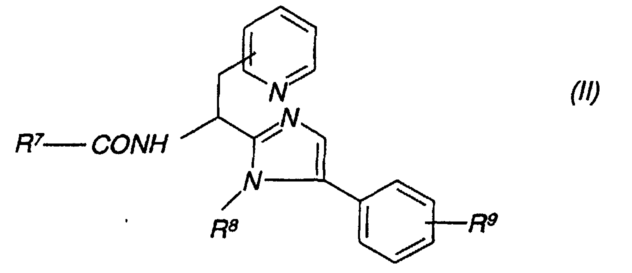 wherein R⁷ is indolyl, benzofuranyl, benzothienyl or benzothiazolyl,
R⁸ is (C₁-C₆) alkyl, and
R⁹ is imidazolyl or nitro,
provided that (i) R⁸ is not methyl when R⁷ is indolyl, and (ii) R⁹ is nitro when R⁷ is benzofuranyl,
and a pharmaceutically acceptable salt thereof.

3. A process for preparing a compound of the formula 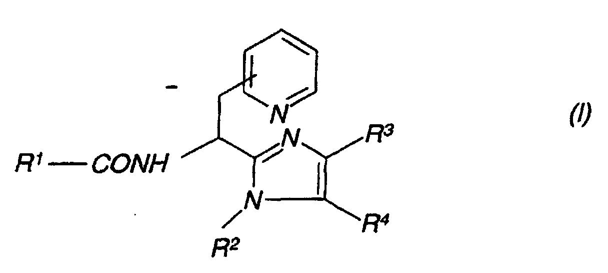 wherein R¹ is (a) indolyl, (b) benzothienyl having suitable substituent(s) selected from the group consisting of nitro and halogen, (c) benzothiazolyl having halogen, (d) furopyridyl which may have nitro or (e) benzofuranyl which may have suitable substituent(s) selected from the group consisting of halogen, (C₁-C₆) alkyl, (C₁-C₆) alkoxy, nitro, cyano, acyl and trihalo (C₁-C₆) alkyl,
R² is (C₁-C₆) alkyl,
R³ is hydrogen or (C₁-C₆) alkyl, and
R⁴ is thienyl or a group of the formula: 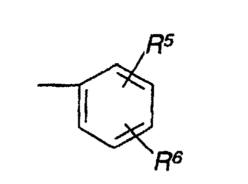 wherein R⁵ is hydrogen or halogen, and
R⁶ is (a) imidazolyl which may have (C₁-C₆) alkyl, (b) benzimidazolyl, (c) pyridyl, (d) pyrrolyl, (e) morpholinyl, (f) thienyl, (g) furyl, (h) phenyl, (j) thiazolyl, (k) halogen or (l) nitro, provided that (i) R³ is (C₁-C₆) alkyl or R⁶ is benzimidazolyl or imidazolyl having (C₁-C₆) alkyl when R¹ is indolyl, and (ii) R⁶ is benzimidazolyl or imidazolyl having (C₁-C₆) alkyl when R¹ is benzofuranyl,
or a salt thereof, which comprises reacting a compound of the formula 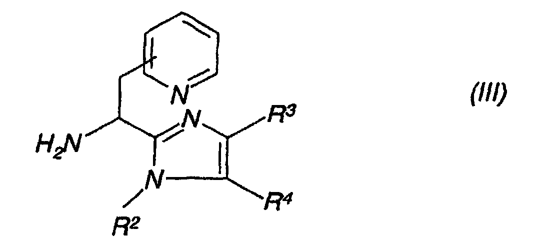 wherein R², R³ and R⁴ are each as defined above, or its reactive derivative at the amino group, such reactive derivative including Schiff's base type imino or its tautomeric enamine type isomer formed by the reaction of the compound (III) with a carbonyl compound such as an aldehyde or a ketone; a silyl derivative formed by the reaction of the compound (III) with a silyl compound such as N,O-bls(trimethylsilyl)acetamide or N-trimethylsilylacetamide; or a derivative formed by the reaction of the compound (III) with phosphorus trichloride or phosgene, or a salt thereof,
with a compound of the formula
R¹-COOH (IV)
wherein R¹ is as defined above, or its reactive derivative at the carboxy group such as an acid halide, an acid azide or an activated ester, or a salt thereof.

4. A process for preparing a compound of the formula 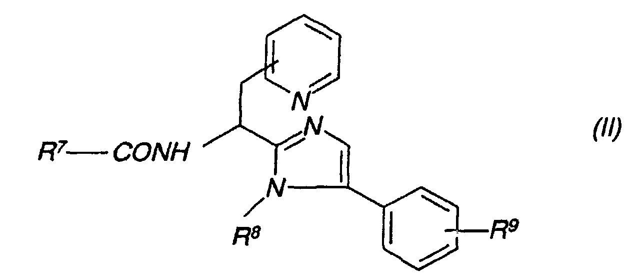 wherein R⁷ is indolyl, benzofuranyl, benzothienyl or benzothlazolyl,
R⁸ is (C₁-C₆) alkyl, and
R⁹ is imidazolyl or nitro,
provided that (i) R⁸ is not methyl when R⁷ is indolyl, and (ii) R⁹ is nitro when R⁷ is benzofuranyl,
or a salt thereof, which comprises reacting a compound of the formula 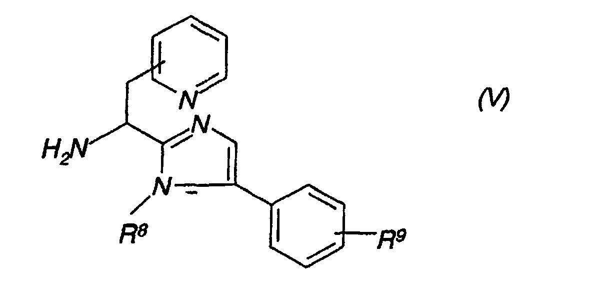 wherein R⁸ and R⁹ are each as defined above, or its reactive derivative at the amino group, such reactive derivative including Schiff's base type imino or its tautomeric enamine type isomer formed by the reaction of the compound (V) with a carbonyl compound such as an aldehyde or a ketone; a silyl derivative formed by the reaction of the compound (V) with a silyl compound such as N,O-bis(trimethylsilyl)acetamide or N-trimethylsilylacetamide; or a derivative formed by the reaction of the compound (V) with phosphorus trichloride or phosgene,
or a salt thereof,
with a compound of the formula
R⁷-COOH (VI)
wherein R⁷ is as defined above, or its reactive derivative at the carboxy group group such as an acid halide, an acid azide or an activated ester,
or a salt thereof.

5. A pharmaceutical composition comprising the compound of Claim 1 or 2 or a pharmaceutically acceptable salt thereof in admixture with a pharmaceutically acceptable carrier.

6. Method for the manufacture of a medicament for prophylactic or therapeutic treatment of NO-mediated diseases **characterized In that** it comprises a compound of any of claims 1 or 2 or pharmaceutically acceptable salts thereof.

## Patentansprüche

1. Verbindung der Formel (I): 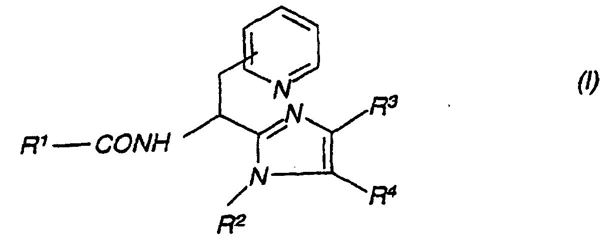 worin
R¹ (a) Indolyl, (b) Benzothienyl mit einem oder mehreren geeigneten Substituenten, die aus der Gruppe ausgewählt sind, die aus Nitro und Halogen besteht, (c) Benzothiazolyl mit einem Halogen, (d) Furopyridyl, welches Nitro enthalten kann, oder (e) Benzofuranyl ist, welches einen oder mehrere geeignete Substituenten haben kann, die aus der Gruppe ausgewählt sind, die aus Halogen, (C₁-C₆) Alkyl, (C₁-C₆) Alkoxy, Nitro, Cyano, Acyl und Trihalogen(C₁-C₆) Alkyl besteht,
R² (C₁-C₆) Alkyl ist,
R³ Wasserstoff oder (C₁-C₆) Alkyl ist, und
R⁴ Thienyl oder eine Gruppe der Formel: 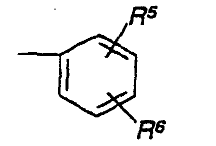 ist, worin
R⁵ Wasserstoff oder Halogen ist, und
R⁶ (a) Imidazolyl, welches ein (C₁-C₆) Alkyl haben kann, (b) Benzimidazolyl, (c) Pyridyl, (d) Pyrrolyl, (e) Morpholinyl, (f) Thienyl, (g) Furyl, (h) Phenyl, (j) Thiazolyl, (k) Halogen oder (1) Nitro ist, vorausgesetzt, dass (i) R³ (C₁-C₆) Alkyl ist oder R⁶ Benzimidazolyl oder Imidazolyl mit einem (C₁-C₆) Alkyl ist, wenn R¹ Indolyl ist, und (ii) R⁶ Benzimidazolyl oder Imidazolyl mit einem (C₁-C₆) Alkyl ist, wenn R¹ Benzofuranyl ist,
und ein pharmazeutisch akzeptables Salz davon.

2. Eine Verbindung der Formel (II): 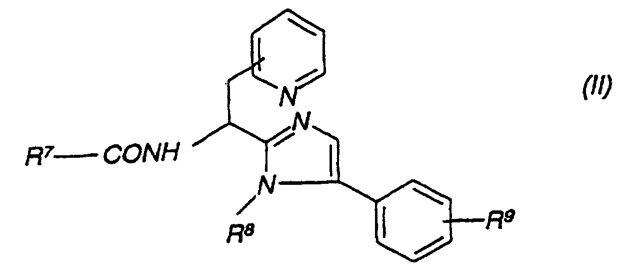 worin
R⁷ Indolyl, Benzofuranyl, Benzothienyl oder Benzothiazolyl ist,
R⁸ (C₁-C₆) Alkyl ist, und
R⁹ imidazolyl oder Nitro ist, vorausgesetzt das (i) R⁸ kein Methyl ist, wann R⁷ Indolyl ist, und (ii) R⁹ Nitro darstellt, wenn R⁷ Benzofuranyl ist,
und ein pharmazeutisch akzeptables Salz davon.

3. Verfahren zur Herstellung einer Verbindung der Formel 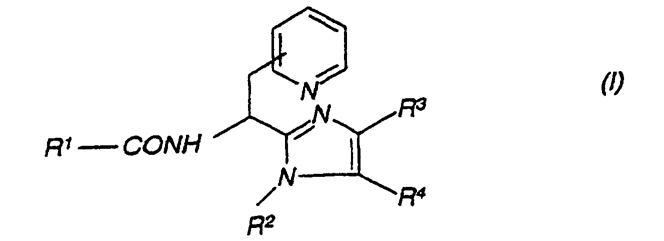 worin
R¹ (a) Indolyl, (b) Benzothienyl mit einem oder mehreren geeigneten Substituenten, die aus der Gruppe ausgewählt sind, die aus Nitro und Halogen besteht, (c) Benzothiazolyl mit Halogen, (d) Furopyridyl, welches Nitro enthalten kann, oder (e) Benzofuranyl ist, das einen oder mehrere geeignete Substituenten haben kann, die aus der Gruppe ausgewählt sind, die aus Halogen, (C₁-C₆) Alkyl, (C₁-C₆) Alkoxy, Nitro, Cyano, Acyl und Trihalogen(C₁-C₆) Alkyl besteht,
R² (C₁-C₆) Alkyl ist,
R³ Wasserstoff oder ein (C₁-C₆) Alkyl ist, und
R⁴ ein Thienyl oder eine Gruppe der Formel: 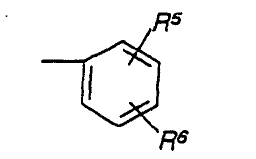 ist, worin
R⁵ Wasserstoffoder Halogen ist, und
R⁶ (a) Imidazolyl, das ein (C₁-C₆) Alkyl haben kann, (b) Benzimidazolyl, (c) Pyridyl, (d) Pyrrolyl, (e) Morpholinyl, (f) Thienyl, (g) Furyl, (h) Phenyl, (j) Thiazolyl, (k) Halogen oder (1) Nitro ist, vorausgesetzt, dass (i) R³ ein (C₁-C₆) Alkyl ist oder R⁶ Benzimidazolyl oder Imidazolyl mit (C₁-C₆) Alkyl ist, wenn R¹ Indolyl ist, und (ii) R⁶ Benzimidazolyl oder Imidazolyl mit (C₁-C₆) Alkyl ist, wenn R¹ Benzofuranyl ist,
oder ein Salz davon, wobei das Verfahren die Reaktion einer Verbindung der Formel 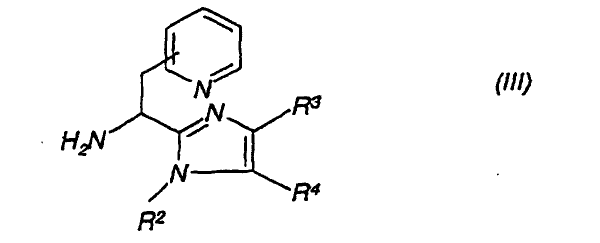 worin
R², R³ und R⁴ so wie oben definiert sind, oder ihr reaktives Derivat an der Aminogruppe, wobei das reaktive Derivat ein Imino des Schiffschen Basentyps oder dessen tautomeres Isomer des Enamintyps umfasst, gebildet durch die Reaktion der Verbindung (III) mit einer Carbonylverbindung, wie ein Aldehyd oder ein Keton; ein Silylderivat, gebildet durch die Reaktion der Verbindung (III) mit einer Silylverbindung, wie N,O-Bis(trimethylsilyl)acetamid oder N-Trimethylsilylacetamid; oder ein Derivat, gebildet durch die Reaktion der Verbindung (III) mit Phosphortrichlorid oder Phosgen,
oder ein Salz davon,
mit einer Verbindung der Formel
R¹ - COOH (IV),
worin R¹ so wie oben definiert ist, oder ihr reaktives Derivat an der Carboxygruppe, wie ein Säurehalogenid, ein Säureazid oder einen aktivierten Ester, oder ein Salz davon umfasst.

4. Verfahren zur Herstellung einer Verbindung der Formel 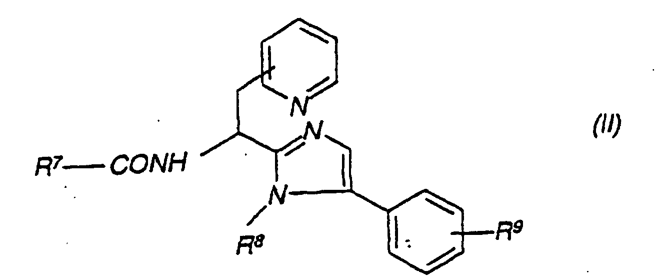 worin
R⁷ Indolyl, Benzofuranyl, Benzothienyl oder Benzothiazolyl ist,
R⁸ (C₁-C₆) Alkyl ist, und
R⁹ imidazolyl oder Nitro darstellt,
vorausgesetzt, dass (i) R⁸ kein Methyl ist, wenn R⁷ Indolyl ist, und (ii) R⁹ Nitro darstellt, wenn R⁷ Benzofuranyl ist,
oder ein Salz davon, wobei das Verfahren die Reaktion einer Verbindung der Formel 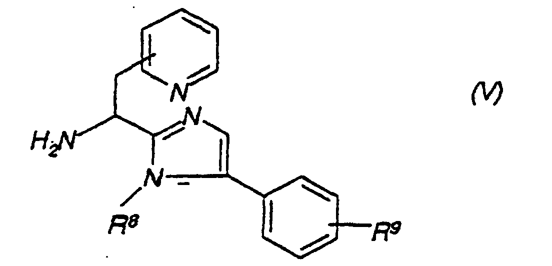 worin R⁸ und R⁹ so wie oben definiert sind, oder ihr reaktives Derivat an der Aminogruppe, wobei das reaktive Derivat ein Imino des Schiffschen Basentyps oder dessen tautomeres Isomer des Enamintyps umfasst, gebildet durch die Reaktion der Verbindung (V) mit einer Carbonylverbindung, wie ein Aldehyd oder ein Keton; ein Silylderivat, gebildet durch die Reaktion der Verbindung (V) mit einer Silylverbindung, wie N,O-Bis(trimethylsilyl)acetamid oder N-Trimethylsilylacetamid; oder ein Derivat, gebildet durch die Reaktion der Verbindung (V) mit Phosphortrichlorid oder Phosgen,
oder ein Salz davon,
mit einer Verbindung der Formel
R⁷ - COOH (VI),
umfasst, worin R⁷ so wie oben definiert ist, oder ihr reaktives Derivat an der Carboxygruppe, wie ein Säurehalogenid, ein Säureazid oder einen aktivierten Ester,
oder ein Salz davon umfasst.

5. Pharmazeutische Zusammenfassung, umfassend die Verbindung nach Anspruch 1 oder 2 oder ein pharmazeutisch akzeptables Salz davon in Beimischung mit einem pharmazeutisch akzeptablen Trägerstoff.

6. Verfahren für die Herstellung eines Medikamentes für die prophylaktische oder therapeutische Behandlung von durch NO verursachten Krankheiten, **dadurch gekennzeichnet, dass** es eine Verbindung nach Anspruch 1 oder 2 oder ein pharmazeutisch akzeptables Salz davon umfasst.

## Revendications

1. Composé de formule (I) : 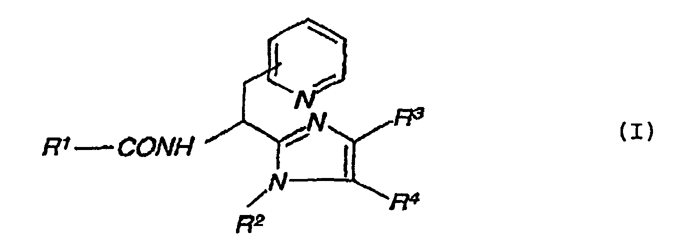 dans laquelle
R¹ est (a) un indolyle, (b) un benzothiényle ayant un ou des substituants appropriés choisis dans le groupe constitué par un nitro et un halogène, (c) un benzothiazolyle ayant un halogène, (d) un furopyridyle qui peut avoir un nitro ou (e) un benzofuranyle qui peut avoir un ou des substituants appropriés choisis dans le groupe constitué par un halogène, un alkyle en C₁-C₆, un alcoxy en C₁-C₆, un nitro, un cyano, un acyle et un trihaloalkyle en C₁-C₆,
R² est un alkyle en C₁-C₆,
R³ est un hydrogène ou un alkyle en C₁-C₆, et
R⁴ est un thiényle ou un groupe de formule : 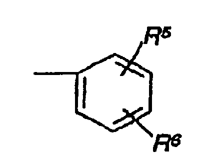
dans laquelle
R⁵ est un hydrogène ou un halogène, et
R⁶ est (a) un imidazolyle qui peut avoir un alkyle en C₁-C₆, (b) un benzimidazolyle, (c) un pyridyle, (d) un pyrrolyle, (e) un morpholinyle, (f) un thiényle, (g) un furyle, (h) un phényle, (j) un thiazolyle, (k) un halogène ou (1) un nitro,
à condition que (i) R³ soit un alkyle en C₁-C₆ ou R⁶ soit un benzimidazolyle ou un imidazolyle ayant un alkyle en C₁-C₆, lorsque R¹ est un indolyle, et (ii) R⁶ soit un benzimidazolyle ou un imidazolyle ayant un alkyle en C₁-C₆, lorsque R¹ est un benzofuranyle,
et un sel pharmaceutiquement acceptable de celui-ci.

2. Composé de formule (II): 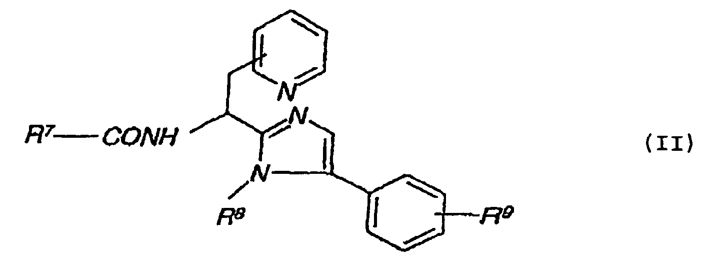 dans laquelle
R⁷ est un indolyle, un benzofuranyle, un benzothiényle ou un benzothiazolyle,
R⁸ est un alkyle en C₁-C₆, et
R⁹ est un imidazolyle ou un nitro,
à condition que (i) R⁶ ne soit pas un méthyle lorsque R⁷ est un indoyle, et (ii) R⁹ soit un nitro lorsque R⁷ est un benzofuranyle,
et un sel pharmaceutiquement acceptable de celui-ci.

3. Procédé pour préparer un composé de formule : 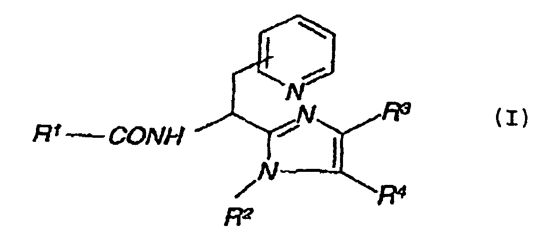 dans laquelle
R¹ est (a) un indolyle, (b) un benzothiényle ayant un ou des substituants appropriés choisis dans le groupe constitué par un nitro et un halogène, (c) un benzothiazolyle ayant un halogène, (d) un furopyridyle qui peut avoir un nitro ou (e) un benzofuranyle qui peut avoir un ou des substituants appropriés choisis dans le groupe constitué par un halogène, un alkyle en C₁-C₆, un alcoxy en C₁-C₆, un nitro, un cyano, un acyle et un trihaloalkyle en C₁-C₆,
R² est un alkyle en C₁-C₆,
R³ est un hydrogène ou un alkyle en C₁-C₆, et
R⁴ est un thiényle ou un groupe de formule : 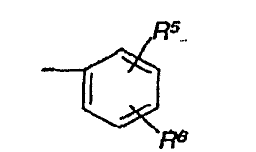
dans laquelle
R⁵ est un hydrogène ou un halogène, et
R⁶ est (a) un imidazolyle qui peut avoir un alkyle en C₁-C₆, (b) un benzimidazolyle, (c) un pyridyle, (d) un pyrrolyle, (e) un morpholinyle, (f) un thiényle, (g) un furyle, (h) un phényle, (j) un thiazolyle, (k) un halogène ou (1) un nitro,
à condition que (i) R³ soit un alkyle en C₁-C₆ ou R⁶ soit un benzimidazolyle ou un imidazolyle ayant un alkyle en C₁-C₆, lorsque R¹ est un indolyle, et (ii) R⁶ soit un benzimidazolyle ou un imidazolyle ayant un alkyle en C₁-C₆, lorsque R¹ est un benzofuranyle,
ou un sel de celui-ci,
qui comprend le fait de faire réagir un composé de formule 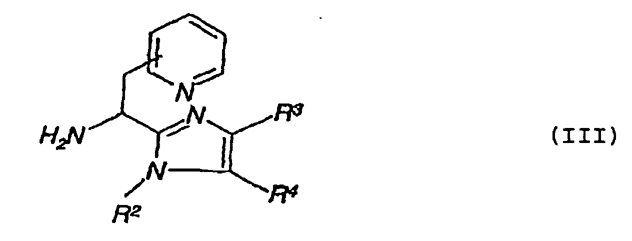 dans laquelle
R², R³ et R⁴ sont chacun tels que définis ci-dessus, ou son dérivé réactif au groupe amino, un tel dérivé réactif incluant un imino de type base de Schiff ou son isomère tautomérique de type énamine formé par la réaction du composé (III) avec un composé carbonylé tel que un aldéhyde ou une cétone; un dérivé silylé formé par la réaction du composé (III) avec un composé silylé tel que le N,O-bis(triméthylsilyl)acétamide ou le N-triméthylsilylacétamide; ou un dérivé formé par la réaction du composé (III) avec du trichlorure de phosphore ou du phosgène,
ou un sel de celui-ci,
avec un composé de formule
R¹-COOH (IV)
dans laquelle
R¹ est tel que défini ci-dessus,
ou son dérivé réactif au groupe carboxy tel que un halogénure d'acide, un azoture d'acide ou un ester activé,
ou un sel de celui-ci.

4. Procédé pour préparé un composé de formule : 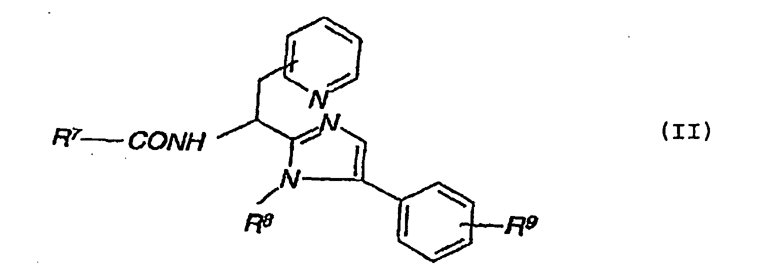 dans laquelle
R⁷ est un indolyle, un benzofuranyle, un benzothiényle ou un benzothiazolyle,
R⁸ est un alkyle en C₁-C₆, et
R⁹ est un imidazolyle ou un nitro,
à condition que (i) R⁶ ne soit pas un méthyle lorsque R⁷ est un indoyle, et (ii) R⁹ soit un nitro lorsque R⁷ est un benzofuranyle,
ou un sel de celui-ci,
qui comprend le fait de faire réagir un composé de formule 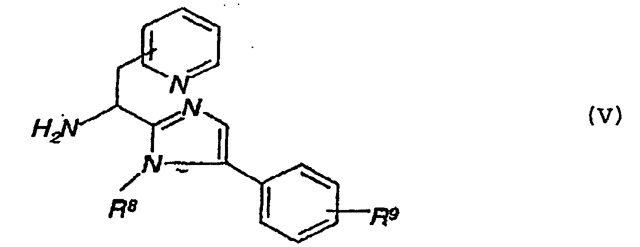 dans laquelle
R⁸ et R⁹ sont chacun tels que définis ci-dessus, ou son dérivé réactif au groupe amino, un tel dérivé réactif incluant un imino de type base de Schiff ou son isomère tautomérique de type énamine formé par la réaction du composé (V) avec un composé carbonylé tel que un aldéhyde ou une cétone; un dérivé silylé formé par la réaction du composé (V) avec un composé silylé tel que le N,O-bis(triméthylsilyl)acétamide ou le N-triméthylsilylacétamide; ou un dérivé formé par la réaction du composé (V) avec du trichlorure de phosphore ou du phosgène,
ou un sel de celui-ci,
avec un composé de formule
R⁷-COOH (VI)
dans laquelle R⁷ est tel que défini ci-dessus,
ou son dérivé réactif au groupe carboxy tel que un halogénure d'acide, un azoture d'acide ou un ester activé,
ou un sel de celui-ci.

5. Composition pharmaceutique comprenant le composé de la revendication 1 ou 2 ou un sel pharmaceutiquement acceptable de celui-ci en mélange avec un support pharmaceutiquement acceptable.

6. Procédé pour la fabrication d'un médicament pour un traitement prophylactique ou thérapeutique d'une maladie liée à NO **caractérisé en ce qu'**il comprend un composé de l'une quelconque des revendications 1 ou 2 ou un sel pharmaceutiquement acceptable de celui-ci.
